# EUROPEAN PATENT SPECIFICATION

(11) **EP 4 136 261 B1**
(45) Date of publication and mention of the grant of the patent: **02.04.2025**
(21) Application number: 21717480.4
(22) Date of filing: 15.04.2021
(51) Int. Cl.: C12Q 1/6883, G16B 5/10

(54) **PROGNOSTIC PATHWAYS FOR VIRAL INFECTIONS**
PROGNOSTISCHE PFADE FÜR VIRUSINFEKTIONEN
VOIES PROGNOSTIQUES DES INFECTIONS VIRALES

(30) Priority: 16.04.2020 EP 20169917; 01.10.2020 EP 20199615
(43) Date of publication of application: 22.02.2023
(73) Proprietor: InnoSIGN B.V., 5656 AE Eindhoven (NL)
(72) Inventor: VAN DE STOLPE, Anja, 5656 AE Eindhoven (NL); BOUWMAN, Wilbert, Hendrik, 5656 AE Eindhoven (NL)
(74) Representative: Algemeen Octrooi- en Merkenbureau B.V.
(86) International application number: PCT/EP2021/059821
(87) International publication number: WO 2021/209567

(56) References cited:
- WO-A1-2019/068562
- US-A1- 2019 085 378
- BOUWMAN WILBERT ET AL: "Quantitative measurement of activity of JAK-STAT signaling pathways in blood samples and immune cells to predict innate and adaptive cellular immune response to viral infection and accelerate vaccine development.", 15 May 2020 (2020-05-15), pages 1 - 28, XP055786433, Retrieved from the Internet <URL:https://www.biorxiv.org/content/10.1101/2020.05.13.092759v1.full.pdf> [retrieved on 20210316], DOI: 10.1101/2020.05.13.092759
- BOUWMAN WILBERT ET AL: "Quantitative measurement of activity of JAK-STAT signaling pathways in blood samples and immune cells to predict innate and adaptive cellular immune response to viral infection and accelerate vaccine development - suppl. information", 15 May 2020 (2020-05-15), pages 1 - 12, XP055786440, Retrieved from the Internet <URL:https://www.biorxiv.org/content/10.1101/2020.05.13.092759v1.supplementary-material> [retrieved on 20210316], DOI: 10.1101/2020.05.13.092759
- ZAAS AIMEE K ET AL: "Gene Expression Signatures Diagnose Influenza and Other Symptomatic Respiratory Viral Infections in Humans", CELL HOST & MICROBE, ELSEVIER, NL, vol. 6, no. 3, 17 September 2009 (2009-09-17), pages 207 - 217, XP002670360, ISSN: 1931-3128, [retrieved on 20090806], DOI: 10.1016/J.CHOM.2009.07.006

## Description

### FIELD OF THE INVENTION

The present invention relates to a method for determining whether an infection is a viral infection or a bacterial infection. The invention further relates to a method for determining the cellular immune response in a blood sample from a subject with a viral infection, and determining the severity of the infection. The invention also relates to products for performing the method of the invention and use of these products in a diagnostic method.

### BACKGROUND OF THE INVENTION

An appropriately functioning immune system is crucial for maintaining health and limiting the damage of disease. An appropriate immune response protects against disease, is relevant for the course of a disease and may be required for optimal effect of therapeutics. The mechanistic principle behind its functioning is that the immune system generates an immune response to non-self antigens, like an infectious agent or an abnormal protein peptide presented within the HLA complex on a cancer cell. Recognition of such antigens is at the core of a functioning immune system.

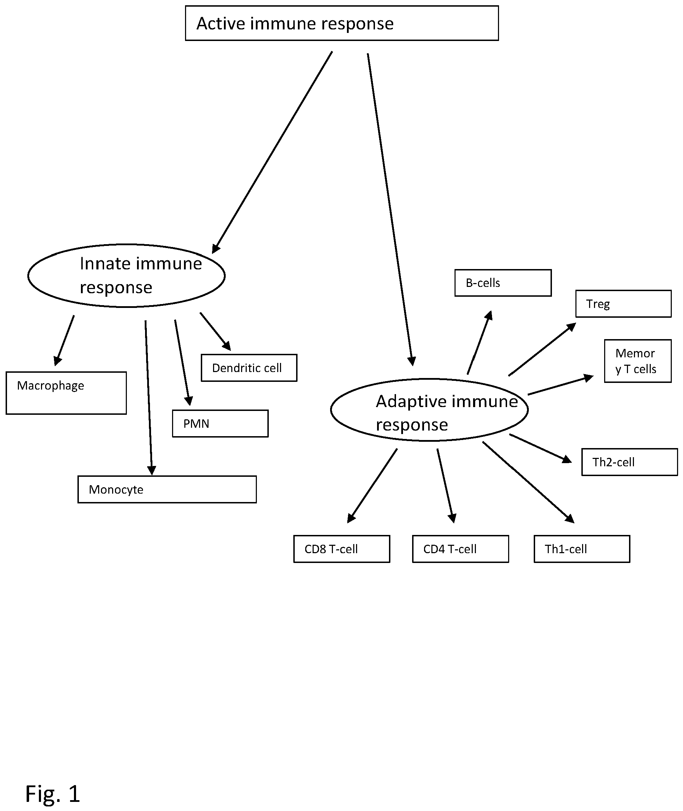

The immune system is made up by a large number of immune cell types that work together in a coordinated manner to produce the right immune response to for example an invading pathogen or an internal disease like cancer (Fig. 1). A distinction is made between the innate immune system which controls the early inflammatory response, and the adaptive immune response that controls long term immune responses and generates immunity. Another distinction is between the humoral response, meaning the antibodies that are generated against the pathogen, and the cellular immune response, executed by natural killer cells and cytotoxic T cells. For bacterial infections, where the pathogen can be recognized outside a cell, the humoral immune response is thought to be very important, while for a viral immune response or cancer, the cellular immune response directed against abnormal or virus-infected cells is also of prime importance.

Viral infections can create worldwide disasters, as exemplified by the COVID-19 pandemic. In this case the worldwide problem is the sudden rapid deterioration in a subset of COVID-19 patients with a pneumonia, requiring ICU admission and frequently artificial invasive ventilation. Other respiratory viruses like other coronaviruses, such as SARS and MERS, but also other viruses like the Respiratory Syncytial Virus (RSV) can cause similar clinical behavior in a subset of patients, resulting in the necessity for ICU admission and invasive ventilation. But also many other types of viruses that do or do not specifically target the respiratory system, can cause epidemics, associated with increased mortality and serious complications, e.g. influenza, Ebola, measles, yellow fever, rotavirus etc.

The course of the disease, risk at complications and clinical outcome of viral infections, including viral pneumonia, is strongly determined by the host immune response. Reduced function of the immune response is caused by factors such as comorbidities, e.g. COPD, drugs, e.g. chemotherapy or corticosteroids, old age. In the COVID-19 pandemic, this reduced immune response frequently results in the life threatening COVID-19 pneumonia, frequently necessitating admission to the ICU. In contrast, many healthy people with a properly functioning immune system report minimal symptoms and rapid recovery (1),(2).

In addition to the frequency of human contacts and the infectiousness of the virus, progression of a pandemic is determined by the percentage of individuals who have developed immunity against the virus. Development of immunity is also determined by the type of virus: some viruses induce strong immunity, such as influenza virus, but other viruses like the COVID-19 virus, often do not elicit a strong immunity. Knowledge on the average strength of the immunity against a virus in individuals in a population is necessary to plan measurements to halt an epidemic.

WO 2019/068562 A1 describes a method for determining JAK-STAT1/2 pathway activity based on a defined set of target genes. US 2019/085378 describes a method of selecting an exon of an RNA whose expression level is informative with respect to infection type of a subject.

### SUMMARY OF THE INVENTION

Measurement of the immune response, especially the cellular immune response is a high need in order to develop models to predict the progression of an epidemic, to take the right decisions with regard to restricting spread of an epidemic, to predict prognosis and identify infected patients at high risk for serious complications or progression of the infection in time for relocation to a hospital with appropriate care options, decide on whether the patient benefits from a stay at the ICU or is better off in a quiet environment with close family (triage for admission to the ICU), to choose the optimal treatment, including drug treatment, and to distinguish between a viral and bacterial infection.

Such a test should measure the functional host immune response state, preferably both the innate and adaptive immune response, and for a clinical application should have a rapid turn-over time to enable a timely clinical decision with regard to ICU admission.

Currently there are no tests available to measure the functional status of the innate and adaptive immune response to a virus based on a blood test.

Therefore, in a first aspect, the invention relates to a method for distinguishing between a bacterial and a viral infection in a blood sample obtained from a subject with an infection, based on the determined expression levels of three or more target genes of the JAK-STAT1/2 cellular signaling pathway, the method comprising:
- receiving the determined expression levels of the three or more target genes of the JAK-STAT1/2 cellular signaling pathway;
- determining the JAK-STAT1/2 cellular signaling pathway activity, wherein the determining the JAK-STAT1/2 cellular signaling pathway activity comprises assigning a numeric value to the JAK-STAT1/2 cellular signaling pathway activity level by evaluating a calibrated mathematical pathway model relating expression levels of the target genes to the activity level of the JAK-STAT1/2 cellular signaling pathway;
   wherein the method further comprises determining an activity level of a signaling pathway associated transcription factor (TF) element, the signaling pathway associated TF element controlling transcription of the three or more target genes, the determining being based on evaluating a calibrated mathematical pathway model relating expression levels of the target genes to the activity level of the JAK-STAT1/2 cellular signaling pathway, and
   inferring the activity of the JAK-STAT1/2 cellular signaling pathway in the blood sample based on the determined activity level of the signaling pathway associated TF element,
- comparing the JAK-STAT1/2 cellular signaling pathway activity determined in the blood sample obtained from the subject with the JAK-STAT1/2 cellular signaling pathway activity determined in a reference blood sample,
   wherein the reference blood sample is obtained from a healthy subject or a subject recovered from an infection,
   and wherein the infection in the subject from which the blood sample is obtained is determined to be viral when the JAK-STAT1/2 cellular signaling pathway activity is higher compared to the JAK-STAT1/2 cellular signaling pathway activity in the reference blood sample, or
   wherein the infection in the subject from which the blood sample is obtained is determined to be bacterial when the JAK-STAT1/2 cellular signaling pathway activity is not higher compared to the JAK-STAT1/2 cellular signaling pathway activity in the reference blood sample,
   wherein the calibrated mathematical pathway model is a centroid or a linear model, or a Bayesian network model based on conditional probabilities, and
   wherein the three or more target genes of the JAK-STAT1/2 cellular signaling pathway are selected from the group consisting of:
      BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFD1L, USP18, and ZNRF3. Preferably said step of comparing the cellular signaling pathway activity is based on comparing the numerical value assigned to said cellular signaling pathway activity.

In an embodiment the determining the JAK-STAT1/2 cellular signaling pathway activity is based on evaluating a calibrated mathematical pathway model relating the expression levels of the three or more JAK-STAT1/2 target genes to an activity level of the family of JAK-STAT1/2 transcription factor (TF) elements, the family of JAK-STAT1/2 TF elements controlling transcription of the three or more JAK-STAT1/2 target genes, the activity of the JAK-STAT1/2 cellular signaling pathway being defined by the activity level of the family of JAK-STAT1/2 TF elements, the calibrated mathematical pathway model being a model that is calibrated using a ground truth dataset including samples in which transcription of the three or more JAK-STAT1/2 target genes is induced by the family of JAK-STAT1/2 TF elements and samples in which transcription of the three or more JAK-STAT1/2 target genes is not induced by the family of JAK-STAT1/2 TF elements,
wherein the JAK-STAT1/2 cellular signaling pathway refers to a signaling process that leads to transcriptional activity of the family of JAK-STAT1/2 TF elements, and wherein the family of JAK-STAT1/2 TF elements are protein complexes each containing a homodimer or a heterodimer comprising STAT1 and/or STAT2.

In a second aspect, the invention relates to a method for determining the cellular immune response to a viral infection or a vaccine in a blood sample obtained from a subject with a viral infection or a subject who received a vaccine, based on the determined expression levels of three or more target genes of the JAK-STAT1/2 cellular signaling pathway, the method comprising:
- receiving the determined expression levels of the three or more target genes of the JAK-STAT1/2 cellular signaling pathway;
- determining the JAK-STAT1/2 cellular signaling pathway activity, wherein the determining the JAK-STAT1/2 cellular signaling pathway activity comprises assigning a numeric value to the JAK-STAT1/2 cellular signaling pathway activity level by evaluating a calibrated mathematical pathway model relating expression levels of the target genes to the activity level of the JAK-STAT1/2 cellular signaling pathway;
- comparing the JAK-STAT1/2 cellular signaling pathway activity determined in the blood sample obtained from the subject with a viral infection or a subject who received a vaccine with the JAK-STAT1/2 cellular signaling pathway activity determined in a reference blood sample obtained from a healthy subject,
   wherein the activity of the JAK-STAT1/2 cellular signaling pathway is compared with the cellular signaling pathway activities determined in the reference blood samples in order to determine whether the immune response to the viral infection is weak or strong, wherein the method further comprises:
      - receiving the determined expression levels of the three or more target genes of the JAK-STAT3 cellular signaling pathway;
      - determining the JAK-STAT3 cellular signaling pathway activity, wherein the determining the JAK-STAT3 cellular signaling pathway activity comprises assigning a numeric value to the JAK-STAT3 cellular signaling pathway activity level by evaluating a calibrated mathematical pathway model relating expression levels of the target genes to the activity level of the JAK-STAT3 cellular signaling pathway;
   wherein the method further comprises determining an activity level of a signaling pathway associated transcription factor (TF) element, the signaling pathway associated TF element controlling transcription of the three or more target genes, the determining being based on evaluating a calibrated mathematical pathway model relating expression levels of the target genes to the activity level of the JAK-STAT1/2 cellular signaling pathway, and
   inferring the activity of the JAK-STAT1/2 cellular signaling pathway in the blood sample based on the determined activity level of the signaling pathway associated TF element,
- comparing the JAK-STAT3 cellular signaling pathway activity determined in the blood sample obtained from the subject with a viral infection or a subject who received a vaccine with the JAK-STAT3 cellular signaling pathway activity determined in a reference blood sample obtained from a healthy subject,
   wherein the calibrated mathematical pathway model is a centroid or a linear model, or a Bayesian network model based on conditional probabilities, and
   wherein the three or more target genes of the JAK-STAT1/2 cellular signaling pathway are selected from the group consisting of:
      BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFD1L, USP18, and ZNRF3, and
      wherein the three or more target genes of the JAK-STAT3 cellular signaling pathway are selected from the group consisting of:
         AKT1, BCL2, BCL2L1, BIRC5, CCND1, CD274, CDKNIA, CRP, FGF2, FOS, FSCN1, FSCN2, FSCN3, HIFIA, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST1, VIM and ZEB1. Preferably said step of comparing the cellular signaling pathway activities is based on comparing the numerical values assigned to said cellular signaling pathway activities.

In an embodiment the determining the JAK-STAT1/2 cellular signaling pathway activity is based on evaluating a calibrated mathematical pathway model relating the expression levels of the three or more JAK-STAT1/2 target genes to an activity level of the family of JAK-STAT1/2 transcription factor (TF) elements, the family of JAK-STAT1/2 TF elements controlling transcription of the three or more JAK-STAT1/2 target genes, the activity of the JAK-STAT1/2 cellular signaling pathway being defined by the activity level of the family of JAK-STAT1/2 TF elements, the calibrated mathematical pathway model being a model that is calibrated using a ground truth dataset including samples in which transcription of the three or more JAK-STAT1/2 target genes is induced by the family of JAK-STAT1/2 TF elements and samples in which transcription of the three or more JAK-STAT1/2 target genes is not induced by the family of JAK-STAT1/2 TF elements,
wherein the JAK-STAT1/2 cellular signaling pathway refers to a signaling process that leads to transcriptional activity of the family of JAK-STAT1/2 TF elements, and wherein the family of JAK-STAT1/2 TF elements are protein complexes each containing a homodimer or a heterodimer comprising STAT1 and/or STAT2.

In a preferred embodiment the method further comprises:
- receiving the determined expression levels of the three or more target genes of the JAK-STAT3 cellular signaling pathway;
- determining the JAK-STAT3 cellular signaling pathway activity, wherein the determining the JAK-STAT3 cellular signaling pathway activity comprises assigning a numeric value to the JAK-STAT3 cellular signaling pathway activity level by evaluating a calibrated mathematical pathway model relating expression levels of the target genes to the activity level of the JAK-STAT3 cellular signaling pathway;
- comparing the JAK-STAT3 cellular signaling pathway activity determined in the blood sample obtained from the subject with a viral infection or a subject who received a vaccine with the JAK-STAT3 cellular signaling pathway activity determined in a reference blood sample obtained from a healthy subject.

Preferably the immune response to a viral infection is considered weak when the numeric value assigned to the JAK-STAT1/2 cellular signaling pathway activity in the blood sample obtained from the subject with a viral infection or a subject who received a vaccine is one standard deviation higher than the numerical value assigned to the JAK-STAT1/2 cellular signaling pathway activity in the reference blood sample of the healthy subject and the immune response to a viral infection is considered strong when the numeric value assigned to the JAK-STAT1/2 cellular signaling pathway activity in the blood sample obtained from the subject with a viral infection or a subject who received a vaccine is two, preferably three or more, standard deviations higher than the numerical value assigned to the JAK-STAT1/2 cellular signaling pathway activity in the reference blood sample of the healthy subject.

Alternatively the comparing the JAK-STAT1/2 and optionally the JAK-STAT3 cellular signaling pathway activities determined in the blood sample obtained from the subject with a viral infection or a subject who received a vaccine further comprises comparing with the JAK-STAT1/2 and optionally the JAK-STAT3 cellular signaling pathway activities determined in a reference blood sample obtained from a reference patient with a weak immune response and the JAK-STAT1/2 and optionally the JAK-STAT3 cellular signaling pathway activities determined in a reference blood sample obtained from a reference patient with a strong immune response, and wherein the strength of the immune response in the subject with a viral infection or the subject who received a vaccine is based on the comparison between the JAK-STAT1/2 cellular signaling pathway activities from the subject with a viral infection or the subject who received a vaccine with the JAK-STAT1/2 cellular signaling pathway activities determined in the reference blood samples obtained from the reference patient with a weak immune response and the reference blood samples obtained from the reference patient with a strong immune response.

In an embodiment of the second aspect of the invention the JAK-STAT1/2 cellular signaling pathway activity corresponds to the strength of the immune response, wherein a higher JAK-STAT1/2 cellular signaling pathway activity signifies a stronger immune response.

In an embodiment of the second aspect of the invention the blood sample is from a subject with a viral infection and wherein a higher JAK-STAT3 cellular signaling pathway activity is indicative of a more severe infection.

In an embodiment of the second aspect of the invention the blood sample is from a subject who received a vaccine and wherein a stronger immune response and optionally a higher JAK-STAT3 cellular signaling pathway activity is indicative of a stronger cellular immunity.

It is understood that multiple references may be used in the comparing step, for example multiple blood samples from multiple healthy subjects. In such case the average numeric values for each cellular signaling pathway can be determined among the multiple reference samples and the comparison step can be performed with the determined average value. Doing so provides the further advantage that a standard deviation can be determined which can be used as a threshold for determining whether a pathway activity is higher or lower as described herein.

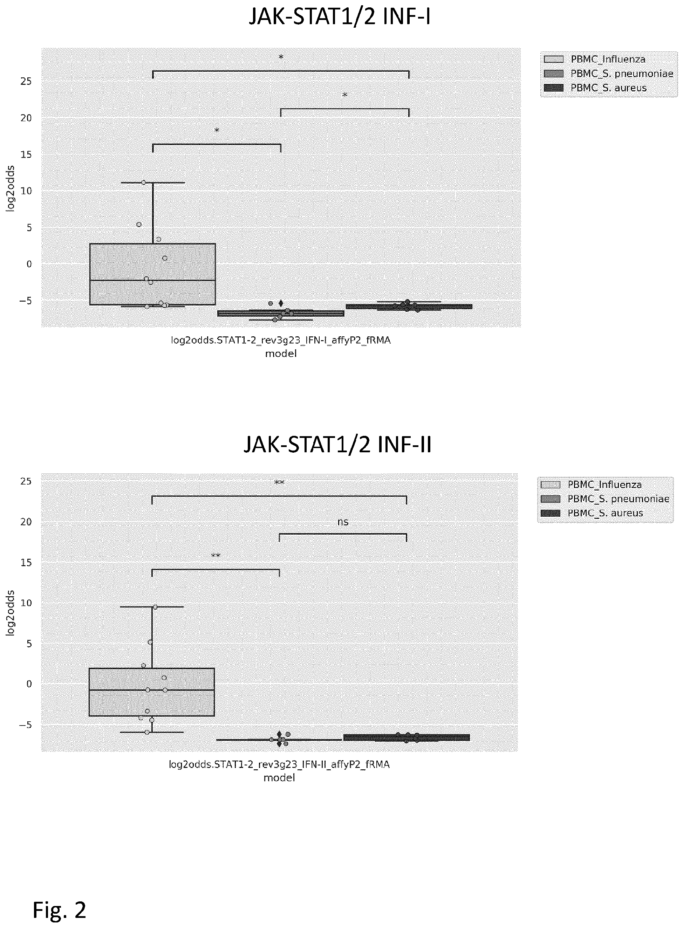

The present invention is based on the finding that by using the claimed methods, based on determining cellular signaling pathway activities, a distinction can be made between a bacterial and a viral infection in a subject. The cellular signaling pathway activities can easily be determined on a blood sample obtained from the subject, and is based on the gene expression levels. The inventors found that JAK-STAT1/2 signaling pathway activity is highly specific for viral infections, and is elevated in subjects with a viral infection or in subjects following successful vaccination against a virus, but not in subjects with a bacterial infection (see figs. 2-4). This is for example contrary to the AR and TGFbeta cellular signaling pathways, which are elevated in subjects with a severe bacterial infections or bacterial sepsis, and may also be elevated in subjects with viral infections. Further, the activity levels can be used to determine the cellular immune response in a subject with a viral infection or a subject who has received a vaccine.

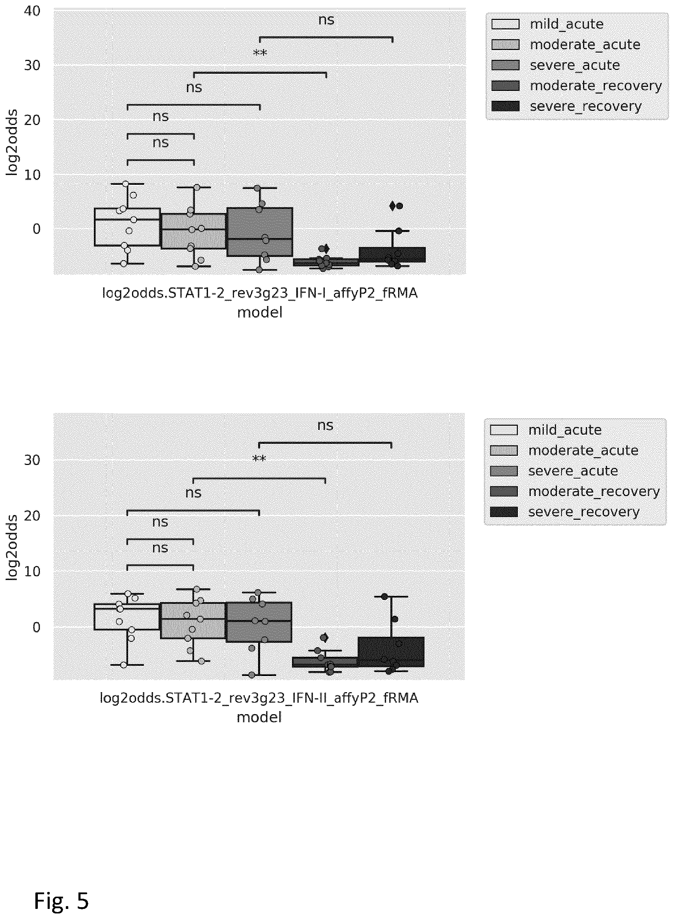

It was found that the activity of the JAK-STAT1/2 signaling pathway correlates with the strength of the immune response in subjects with a viral infection. Additionally the severity of an infection was found to correlate with the JAK-STAT3 signaling pathway activity (see figs. 5-8). Lastly the cellular immunity induced by a vaccine can be determined in a subject who received a vaccine based on the JAK-STAT3, in addition to the JAK-STAT1/2 signaling pathway activities (see fig.s 12-14).

It is noted that an higher JAK-STAT1/2 cellular signaling pathway activity is indicative of a viral infection and a low JAK-STAT1/2 cellular signaling pathway activity is indicative of the absence of a viral infection (meaning the infection is assumed to be bacterial). It cannot be excluded that when a subject with an higher JAK-STAT1/2 cellular signaling pathway activity in the blood sample also has a bacterial infection, however the higher JAK-STAT1/2 cellular signaling pathway activity at least confirms that at least a viral infection is present.

By using a calibrated mathematical model to relate the gene expression levels to a cellular signaling pathway activity, a numerical value can be assigned to the pathway activity. Depending on the model, this value can for example be normalized to result in a value from 0 to 100, where 0 is no pathway activity and 100 is a theoretical maximum pathway activity. Alternatively the value may be normalized such that the average value is 0 and thus decreased pathway activity is represented by a negative value and increased pathway activity is represented by a positive value. It is understood that the values obtained using such model are dependent on the model used, and do not represent absolute values. Therefore, the same model should be used for calibrating, determining reference values and when used in the method of the invention, so that it allows comparison of the obtained numerical values for pathway activity.

Therefore, the numerical value obtained for a pathway activity in a sample may be compared to the numerical value obtained for that pathway activity in a reference sample. By performing such comparison a statement can be made about the pathway activity in the sample (e.g. a blood sample from a subject with an infection) relative to the pathway activity determined in the reference sample (e.g. a blood sample from a healthy subject). Based on the numerical value a statement can be made about the pathway acvtivity in the sample with respect to the pathway activity in the reference sample, e.g. whether the pathway activity in the sample is higher or lower, in correspondence with the numerical value obtained for the pathway activity. E.g. the JAK-STAT1/2 cellular signaling pathway activity can be determined in a blood sample from a healthy person, meaning a person not having an infection. Since it is determined by the inventors that the JAK-STAT1/2 cellular signaling pathway activity in healthy subjects is very low (in a blood sample), this can bet set as a baseline numerical value representing an inactive pathway, i.e. the reference value. By determining the numerical value for the JAK-STAT1/2 cellular signaling pathway activity in the blood sample of a subject with an infection, the numerical value representing that pathway activity can be compared with the reference value, and it can be determined whether the pathway activity is higher or equal (or lower) compared to the reference based on the numerical values. The comparison may be made with multiple reference samples to allow for more accurate results and statistics to be performed.

Alternatively a reference sample can be used to set a baseline pathway activity, allowing further comparison of the pathway activity. For example, the average and standard deviation can be calculated which can be used to calculate values for a healthy individual , and define a threshold for abnormal pathway activity. Alternatively a reference can be used with a known state (e.g. severe infection) and used in the comparison of the pathway activity/activities.

Therefore, in a preferred embodiment the pathway activity is determined to be higher or lower when the obtained numerical value for the pathway activity differs with at least one standard deviation from the numerical value obtained for the pathway activity in the reference sample. When the value is within the range of one standard deviation it is said to be equal or comparable to the pathway activity in the reference sample. It is understood that the threshold may also be set higher, for example 2 times the standard deviation or even 3 times the standard deviation. It is further understood that the threshold may be determined using alternative ways, e.g. statistical methods, to determine a significant deviation from the reference value.

It is understood that the reference value for a certain pathway activity in a sample in principle only needs to be determined once. Therefore, the step of determining a reference pathway activity is not an active step of the methods of the invention, as a predetermined reference pathway activity can be used.

For the purpose of the invention determining the expression levels of the target genes based on the extracted RNA may be a part of the method, meaning the method includes the step of determining the expression levels of the target genes on RNA extracted from the blood sample obtained from the patient using methods known to the skilled person or described herein. The method may further include the step of obtaining a blood sample form the patient in order to extract the RNA. Alternatively the expression levels may have been determined separately and the demining step (of the expression levels of the target genes) is not an active step in the method of the invention. In such case the expression levels are provided as an input value, e.g. a relative expression level in reference to one or more control gene expression levels.

When used herein, "expression level" refers to quantifying the number of mRNA copies transcribed from a gene. Generally this number will not be an absolute value but a relative value, and therefore is preferably normalized for example in reference to the expression of one or more housekeeping genes. Housekeeping genes are genes which are assumed to have constant expression levels independent of cell type and/or functional status of the cell (i.e. from a diseased or healthy subject), and therefore can be used to normalize experimentally determined relative expression levels. Housekeeping genes are generally known to the skilled person, non-limiting examples of housekeeping genes that may be used for normalization are beta-actin, glyceraldehyde-3-phosphate dehydrogenase (GAPDH) and Transcription factor IID TATA binding protein (TBP).

Therefore the phrase "expression level of a target gene" denotes a value that is representative of the amount, e.g. a concentration, of a target gene present in a sample. This value may be based on the amount of a transcription product of the target gene (e.g. mRNA) or a translation product thereof (e.g. protein). Preferably, the expression level is based on the amount of mRNA formed from the target gene. In order to determine the expression level, techniques such as qPCR, multiple qPCR, multiplexed qPCR, ddPCR, RNAseq, RNA expression array or mass spectrometry may be used. For example, a gene expression microarray, e.g. Affymetrix microarray, or RNA sequencing methods, like an Illumina sequencer, can be used.

Sets of cellular signaling pathway target genes whose expression levels are preferably analyzed have been identified, alternatively methods for identifying suitable target genes are described herein. For use to determine pathway activity, for example by a mathematical model, three or more, for example, three, four, five, six, seven, eight, nine, ten, eleven, twelve or more, target genes from each assessed cellular signaling pathway can be analyzed to determine pathway activities.

In an embodiment the signaling pathway measurements are performed using qPCR, multiple qPCR, multiplexed qPCR, ddPCR, RNAseq, RNA expression array or mass spectrometry. For example, a gene expression microarray data, e.g. Affymetrix microarray, or RNA sequencing methods, like an Illumina sequencer, can be used.

The term "subject", as used herein refers to any living being. In some embodiments, the subject is an animal, preferably a mammal. In certain embodiments, the subject is a human being, such as a medical subject. Although the invention is not necessarily limited to a particular group of subjects, it will be apparent that a subject having infection or a subject suspected to have an infection or a subject at risk for developing infection profits the most form the invention described herein. The method is particularly useful for a subject having a viral infection.

The terms "pathway", "signal transduction pathway", "signaling pathway" and "cellular signaling pathway" are used interchangeably herein.

An "activity of a signaling pathway" may refer to the activity of a signaling pathway associated transcription factor (TF) element in the sample, the TF element controlling transcription of target genes, in driving the target genes to expression, i.e., the speed by which the target genes are transcribed, e.g. in terms of high activity (i.e. high speed) or low activity (i.e. low speed), or other dimensions, such as levels, values or the like related to such activity (e.g. speed). Accordingly, for the purposes of the present invention, the term "activity", as used herein, is also meant to refer to an activity level that may be obtained as an intermediate result during "pathway analysis" as described herein.

The term "transcription factor element" (TF element), as used herein, preferably refers to an intermediate or precursor protein or protein complex of the active transcription factor, or an active transcription factor protein or protein complex which controls the specified target gene expression. For example, the protein complex may contain at least the intracellular domain of one of the respective signaling pathway proteins, with one or more co-factors, thereby controlling transcription of target genes. Preferably, the term refers to either a protein or protein complex transcriptional factor triggered by the cleavage of one of the respective signaling pathway proteins resulting in a intracellular domain.

When used herein, the family of JAK-STAT1/2 TF elements are protein complexes each containing a homodimer or a heterodimer comprising STAT1 and/or STAT2. Preferably the homodimer or heterodimer comprises phosphorylated STAT1 and/or STAT2.

When used herein, the family of JAK-STAT3 TF elements are protein complexes each containing a homodimer or a heterodimer comprising STAT3. Preferably the homodimer or heterodimer comprises phosphorylated STAT3.

When used herein, the family of AR TF elements are protein complexes each containing a homodimer or a heterodimer comprising AR-A and/or AR-B. Preferably the AR-A and/or AR-B in the homodimer or heterodimer is/are phosphorylated.

The term "target gene", as used herein, means a gene whose transcription is directly or indirectly controlled by a respective transcription factor element. The "target gene" may be a "direct target gene" and/or an "indirect target gene" (as described herein). Preferably the target gene is a direct target gene.

Pathway analysis enables quantitative measurement of signal transduction pathway activity in blood cells, based on inferring activity of a signal transduction pathway from measurements of mRNA levels of the well-validated direct target genes of the transcription factor associated with the respective signaling pathway (see for example W Verhaegh et al., 2014, supra; W Verhaegh, A van de Stolpe, Oncotarget, 2014, 5(14):5196).

The term "sample", or "blood sample" as used herein, also encompasses the case where e.g. a tissue and/or draining lymph node and/or blood of the subject have been taken from the subject and, e.g., have been put on a microscope slide, and where for performing the claimed method a portion of this sample is extracted, e.g., by means of Laser Capture Microdissection (LCM), or by scraping off the cells of interest from the slide, or by fluorescence-activated cell sorting techniques.

The terms "healthy subject" or "heatlhy reference subject" are used interchangably, and when used herein refer to a subject not having an infection. Preferably the subject does not have a compromised immune system, e.g. because of a genetic disorder or immune modulating drugs. Therefore, a blood sample obtained from a healthy subject refers to a blood sample which was obtained from a subject at a time when the subject did not have an infection, and preferably was not immuno compromised.

Optionally the invention further comprises the steps of:
- receiving the determined expression levels of the three or more target genes of one or more of the AR, TGFbeta and MAPK-AP1 cellular signaling pathways;
- determining the cellular signaling pathway activity of the one or more of the AR, TGFbeta and MAPK-AP1 cellular signaling pathways, wherein the determining the one or more of the AR, TGFbeta and MAPK-AP1 cellular signaling pathway activitiescomprises assigning numeric values to the one or more of the AR, TGFbeta and MAPK-AP1 cellular signaling pathway activity levels by evaluating a calibrated mathematical pathway model relating expression levels of the target genes to the activity level of the one or more of the AR, TGFbeta and MAPK-AP1 cellular signaling pathways;
- comparing the one or more of the AR, TGFbeta and MAPK-AP1 cellular signaling pathway activities determined in the blood sample obtained from the subject with the one or more of the AR, TGFbeta and MAPK-AP1 cellular signaling pathway activities determined in a reference blood sample.

The present invention builds on the findings published in WO2019068623 A1.

Every immune cell type, like CD4+ Th1 and Th2 cells, CD8+ T-cells, T-Reg cells, B-cells, neutrophils, monocytes, macrophages, and dendritic cells has a specific function in the immune response by which it can be in principle characterized and recognized. For each immune cell type there is an inactive (herein also referred to as "resting") state and an activated (herein also referred as a "supportive" state) in which activity is directed towards eradicating an immune target like a cancer antigen or a pathogen. For some types of immune cells (dendritic cells, Treg cells) there is also a "suppressive" state in which the immune cell suppresses other immune cells in their function. Immune cells communicate with each other using cell-cell interaction and through soluble molecules like cytokines and chemokines to coordinate their activity. AR, ER, HH, JAK-STAT1/2 (comprising the JAK-STAT1/2 IFN Type I (which is activated by the Interferon type I cytokines) and JAK-STAT1/2 type II IFN (which is activated by the Interferon type II cytokines)), JAK-STAT3, MAPK-AP-1, NFkB, Notch, PI3K, TGF-β, and Wnt are signal transduction pathways which mediate such communication between cells and determine functional activities in cells as a consequence of the communication. These signaling pathways also play important roles in the functioning of the different immune cell types.

WO2019068623 A1 discloses the finding that analysis of signal transduction pathway activity can be used to characterize the type of immune cells as well as the functional status of the various immune cell types that play a role in the immune response. The resultant immune response as a consequence of communication between immune cells of various types can be towards activity, for example anti-tumor activity, or towards immunosuppression or tolerance against antigens, like is normally the case for self-antigens in the body, but can also be the case in cancer when the immune system does not attack the cancer cells. In the latter case, as an example some membrane proteins interfering with adequate anti-tumor immune cell activity are PD1 on the CD8+ lymphocytes and PD-L1 on the cancer cells. Some (limited) signaling pathway activities in immune cells have been described in relation to their function: PD1 signaling may result in increased FOXO transcription factor activity and reduced PI3K pathway activity (these are inversely related), and increased TGF-β pathway activity; PD-L1 signaling (tumor and immune cells) may activate NFkB and MAPK-AP-1 pathways; effective T cell receptor signaling induces PI3K pathway activity; IL2 activates dendritic cell antigen presentation through activation of the JAK-STAT1/2 pathway. How these pathway activities relate to each other in determining the functional state of the various immune cells has not been known.

WO2019068623 A1 provides a method which enables interpretation of the pathway activities and determination of the functional status of immune cells. The inventors found that the functional status (e.g. resting, supportive, suppressive, naive, memory) of individual immune cell types can be assessed by measuring activity of one or more signal pathways that control immune cell function in the different immune cell types in an immune cell type-specific manner. The inventors therefore inferred the activity of different signal pathways (AR, ER, HH, JAK-STAT1/2, JAK-STAT3, MAPK-AP-1, NFkB, Notch, PI3K, TGF-β, and Wnt pathways) in different immune cell types having a known functional status and developed a computational model for interpretation per immune cell type the measured pathway activities to be able to predict the functional status of the immune cell types having an unknown functional status based on pathway activity. The JAK-STAT1/2 pathway is used to indicate one or both of its variants (JAK-STAT1/2 Interferon type I (IFN type I) and JAK-STAT1/2 Interferon type II (IFN type II)) unless one of the variants is specifically mentioned.

The activity of one or more signaling pathways can thus be used as a biomarker that characterizes the functional state of an immune cell, e.g. a dendritic cell, which will be useful for therapy choice in patients with cancer or another diseases in which the immune response needs to be activated, but also in patients with for example rheumatoid arthritis, or other diseases in which the immune response needs to be dampened.

The method of the invention is based on a single cellular signaling pathway activity or on a "combination of activities of cellular signaling pathways". This means that the method of the invention is influenced by the activities of one or more cellular signaling pathways. The activities of the one or more cellular signaling pathways can be inferred and/or combined by a mathematical model as described herein. In a preferred embodiment, the method of the invention is based on a combination of signaling pathway activities comprising activities of more than 2 cellular signaling pathways. Such combination of signaling pathway activities may include the activities of 3 or 4, or even more than 4 such as 5, 6, 7 or 8, or even more, different signaling pathways.

In an aspect of the disclsoure, the determined activity levels of the JAK-STAT1/2 and optionally the JAK-STAT3 cellular signaling pathway is further used to:
- monitor a patient with an infection, or
- determine the strength of the cellular immunity induced by a viral infection or vaccination in an individual, or
- predict the response to an immune modulatory therapy or drug, or
- monitor the response to a drug or therapy, or
- predict the toxicity of an immunomodulatory therapy or drug, or
- estimate the strength of the cellular immunity that will result in a community during an viral infection epidemic/pandemic, or
- determine the strength of the immunity induced by viral infection or vaccination in an individual with a specific immune compromising condition, such as a specific comorbidity, therapy, lifestyle, or
- diagnose patients with an viral infection during an epidemic or pandemic, or
- develop an drug or therapy to treat the infectious disease, or
- predict the immune activating or immune suppressive state caused by the viral infection.

As described below in the experimental results, the activity of the JAK-STAT1/2 and optionally the JAK-STAT3 cellular signaling pathways are informative on several aspects of an infection, and therefore can be used in the described methods for monitoring, diagnosing, determining and predicting. A particularly useful applications may be to monitor a patient with an infection. Based on the JAK-STAT3 cellular signaling pathway activity the severity of an infection can be determined.

In a preferred embodiment of the first and the second aspect of the invention, the method further comprises the step of determining the expression levels of the three or more, e.g. three, four, five, six, seven eight, nine, ten, eleven, twelve, thirteen or more, target genes of the JAK-STAT1/2 cellular signaling pathway and optionally the three or more, e.g. three, four, five, six, seven eight, nine, ten, eleven, twelve, thirteen or more, target genes of the JAK-STAT3 cellular signaling pathway and/or further comprises the step of providing or obtaining the blood sample from the subject.

The steps of determining the gene expression levels and the providing the blood sample are not essential for the method and may be performed separately. This may e.g. be beneficial in that allows the collection of blood samples and determining the expression levels at separate locations, upon which the data (expression levels) are collected centrally for processing and pathway determination according to the claimed method. It may however be beneficial to include the step of determining the expression level of the target genes of the cellular signaling pathways, and optionally the step of providing blood, to ensure uniform processing of the samples.

Methods to collect and store blood, and optionally to separate the blood sample in separate fractions or specific cell types, are generally known in the field. Upon providing the blood sample it can be processed immediately or it can be stored for later processing, e.g. at appropriate storage conditions at , 4 °C, -20 °C or -80 °C depending on the intended storage time. When processing the mRNA is extracted from the sample using known methods and preferably normalized, e.g. based on the total RNA concentration. Subsequently the extracted mRNA is used to determine the expression levels of the target genes by known methods, such as qPCR, multiple qPCR, ddPCR, RNAseq and RNA expression array.

In a preferred embodiment of the first and the second aspect of the invention, the blood sample is whole blood sample, a peripheral blood mononuclear cell sample, or isolated blood cells such as dendritic cells, CD4+ T cells, CD8+ T cells, CD16- monocytes, CD16+ monocytes, Neutrophils, NK cells and B cells.

The strength of the present method resides in the fact that it can be performed on whole blood samples, meaning less processing steps, it may be beneficial to isolate a subsample or specific cell types from the blood sample to address more specific questions. It is therefore envisioned that the method can be performed on any type of nucleated blood cell. In a preferred embodiment of the first and the second aspect of the invention, the three or more, e.g. three, four, five, six, seven eight, nine, ten, eleven, twelve, thirteen or more, target genes of the JAK-STAT1/2 cellular signaling pathway are selected from the group consisting of:
BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFD1L, USP18, ZNRF3, GBP1, TAP1, ISG15, APOL1, IFI6, IFIRM1, CXCL9, APOL2, IFIT2 and LY6E preferably, from the group consisting of: IRF1, IRF7, IRF8, IRF9, OAS1, PDCD1, ST13, STAT1 and USP1, or from the group consisting of GBP1, IRF9, STAT1, TAP1, ISG15, APOL1, IRF1, IRF7, IFI6, IFIRM1, USP18, CXCL9, OAS1, APOL2, IFIT2 and LY6E and/or
wherein the three or more, e.g. three, four, five, six, seven eight, nine, ten, eleven, twelve, thirteen or more, target genes of the JAK-STAT3 cellular signaling pathway are selected from the group consisting of: AKT1, BCL2, BCL2L1, BIRC5, CCND1, CD274, CDKNIA, CRP, FGF2, FOS, FSCN1, FSCN2, FSCN3, HIFIA, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST1, VIM and ZEB1.

Optionally the method further comprises determining the expression levels of three or more, e.g. three, four, five, six, seven eight, nine, ten, eleven, twelve, thirteen or more, target genes of the TGFbeta cellular signaling pathway are selected from the group consisting of:
ANGPTL4, CDC42EP3, CDKN1A, CTGF, GADD45A, GADD45B, HMGA2, ID1, IL11, JUNB, PDGFB, PTHLH, SERPINE1, SGK1, SKIL, SMAD4, SMAD5, SMAD6, SMAD7, SNAI2, VEGFA.

Optionally the method further comprises determining the expression levels of three or more, e.g. three, four, five, six, seven eight, nine, ten, eleven, twelve, thirteen or more, target genes of the AR cellular signaling pathway are selected from the group consisting of:
KLK2, PMEPA1, TMPRSS2, NKX3_1, ABCC4, KLK3, FKBP5, ELL2, UGT2B15, DHCR24, PPAP2A, NDRG1, LRIG1, CREB3L4, LCP1, GUCY1A3, AR and EAF2.

Optionally the method further comprises determining the expression levels of three or more, e.g. three, four, five, six, seven eight, nine, ten, eleven, twelve, thirteen or more, target genes of the MAPK-AP1 cellular signaling pathway are selected from the group consisting of:
BCL2L11, CCND1, DDIT3, DNMT1, EGFR, ENPP2, EZR, FASLG, FIGF, GLRX, IL2, IVL, LOR, MMP1, MMP3, MMP9, SERPINE1, PLAU, PLAUR, PTGS2, SNCG, TIMP1, TP53, and VIM.

According to the first and the second aspect of the invention, the activity of the JAK-STAT1/2 cellular signaling pathway and optionally the JAK-STAT3 cellular signaling pathway in the blood sample is inferred by a method comprising: receiving expression levels of three or more target genes of the JAK-STAT1/2 cellular signaling pathway and optionally the JAK-STAT3 cellular signaling pathway,
determining an activity level of a signaling pathway associated transcription factor (TF) element, the signaling pathway associated TF element controlling transcription of the three or more target genes, the determining being based on evaluating a calibrated mathematical pathway model relating expression levels of the target genes to the activity level of the JAK-STAT1/2 cellular signaling pathway and optionally the JAK-STAT3 cellular signaling pathway, and
inferring the activity of the JAK-STAT1/2 cellular signaling pathway and optionally the JAK-STAT3 cellular signaling pathway in the blood sample based on the determined activity level of the signaling pathway associated TF element,
wherein the calibrated mathematical pathway model is preferably a centroid or a linear model, or a Bayesian network model based on conditional probabilities.

Preferably the determining of the activity or activities of the signaling pathway(s), the combination of multiple pathway activities and applications thereof is performed as described for example in the following documents: published international patent applications WO2013011479 (titled "ASSESSMENT OF CELLULAR SIGNALING PATHWAY ACTIVITY USING PROBABILISTIC MODELING OF TARGET GENE EXPRESSION"), WO2014102668 (titled "ASSESSMENT OF CELLULAR SIGNALING PATHWAY ACTIVITY USING LINEAR COMBINATION(S) OF TARGET GENE EXPRESSIONS"), WO2015101635 (titled "ASSESSMENT OF THE PI3K CELLULAR SIGNALING PATHWAY ACTIVITY USING MATHEMATICAL MODELLING OF TARGET GENE EXPRESSION"), WO2016062891 (titled "ASSESSMENT OF TGF-β CELLULAR SIGNALING PATHWAY ACTIVITY USING MATHEMATICAL MODELLING OF TARGET GENE EXPRESSION"), WO2017029215 (titled "ASSESSMENT OF NFKB CELLULAR SIGNALING PATHWAY ACTIVITY USING MATHEMATICAL MODELLING OF TARGET GENE EXPRESSION"), WO2014174003 (titled "MEDICAL PROGNOSIS AND PREDICTION OF TREATMENT RESPONSE USING MULTIPLE CELLULAR SIGNALLING PATHWAY ACTIVITIES"), WO2016062892 (titled "MEDICAL PROGNOSIS AND PREDICTION OF TREATMENT RESPONSE USING MULTIPLE CELLULAR SIGNALING PATHWAY ACTIVITIES"), WO2016062893 (titled "MEDICAL PROGNOSIS AND PREDICTION OF TREATMENT RESPONSE USING MULTIPLE CELLULAR SIGNALING PATHWAY ACTIVITIES"), WO2018096076 (titled "Method to distinguish tumor suppressive FOXO activity from oxidative stress"), and in the patent applications WO2018096076 (titled "Method to distinguish tumor suppressive FOXO activity from oxidative stress"), WO2019068585 (titled "Assessment of Notch cellular signaling pathway activity using mathematical modelling of target gene expression"), WO2019120658 (titled "Assessment of MAPK-MAPK-AP1 cellular signaling pathway activity using mathematical modelling of target gene expression"), WO2019068543 (titled "Assessment of JAK-JAK-STAT3 cellular signaling pathway activity using mathematical modelling of target gene expression"), WO2019068562 (titled "Assessment of JAK-STAT1/2 cellular signaling pathway activity using mathematical modelling of target gene expression"), and WO2019068623 (titled "Determining functional status of immune cells types and immune response").

The models have been biologically validated for ER, AR, PI3K-FOXO, HH, Notch, TGF-β, Wnt, NFkB, JAK-STAT1/2, JAK-JAK-STAT3 and MAPK-MAPK-AP1 pathways on several cell types.

Unique sets of cellular signaling pathway target genes whose expression levels are preferably analyzed have been identified. For use in the mathematical models, three or more, for example, three, four, five, six, seven, eight, nine, ten, eleven, twelve or more, target genes from each assessed cellular signaling pathway can be analyzed to determine pathway activities.

Common to the pathway analysis methods for determining the activities of the different signaling pathways as disclosed herein is a concept, which is preferably applied herein for the purposes of the present invention, wherein the activity of a signaling pathway in a cell such as a cell present in a blood sample is determinable by receiving expression levels of one or more, preferably three or more, target genes of the signaling pathway, determining an activity level of a signaling pathway associated transcription factor (TF) element in the sample, the TF element controlling transcription of the three or more target genes, the determining being based on evaluating a calibrated mathematical pathway model relating expression levels of the one or more, preferably three or more target genes to the activity level of the signaling pathway, and optionally inferring the activity of the signaling pathway in the cell present in a blood sample based on the determined activity level of the signaling pathway associated TF element. As described herein, the activity level can be directly used as an input to determine the immune response in a subject and/or determine whether an infection is viral and/or determine the severity of a viral infection and/or determine the cellular immmunity conferred by a vaccine, which is also contemplated by the present invention.

The term "activity level" of a TF element, as used herein, denotes the level of activity of the TF element regarding transcription of its target genes.

The calibrated mathematical pathway model may be a probabilistic model, preferably a Bayesian network model, based on conditional probabilities relating the activity level of the signaling pathway associated TF element and the expression levels of the three or more target genes, or the calibrated mathematical pathway model may be based on one or more linear combination(s) of the expression levels of the three or more target genes. For the purposes of the present invention, the calibrated mathematical pathway model is a centroid or a linear model, or a Bayesian network model based on conditional probabilities.

In particular, the determination of the expression level and optionally the inferring of the activity of a signaling pathway in the subject may be performed, for example, by inter alia (i) evaluating a portion of a calibrated probabilistic pathway model, preferably a Bayesian network, representing the cellular signaling pathways for a set of inputs including the expression levels of the three or more target genes of the cellular signaling pathway measured in a sample of the subject, (ii) estimating an activity level in the subject of a signaling pathway associated transcription factor (TF) element, the signaling pathway associated TF element controlling transcription of the three or more target genes of the cellular signaling pathway, the estimating being based on conditional probabilities relating the activity level of the signaling pathway associated TF element and the expression levels of the three or more target genes of the cellular signaling pathway measured in the sample of the subject, and optionally (iii) inferring the activity of the cellular signaling pathway based on the estimated activity level of the signaling pathway associated TF element in the sample of the subject. This is described in detail in the published international patent application WO 2013/011479 A2 ("Assessment of cellular signaling pathway activity using probabilistic modeling of target gene expression").

In an exemplary alternative, the determination of the expression level and optionally the inferring of the activity of a cellular signaling pathway in the subject may be performed by inter alia (i) determining an activity level of a signaling pathway associated transcription factor (TF) element in the sample of the subject, the signaling pathway associated TF element controlling transcription of the three or more target genes of the cellular signaling pathway, the determining being based on evaluating a calibrated mathematical pathway model relating expression levels of the three or more target genes of the cellular signaling pathway to the activity level of the signaling pathway associated TF element, the mathematical pathway model being based on one or more linear combination(s) of expression levels of the three or more target genes, and optionally (ii) inferring the activity of the cellular signaling pathway in the subject based on the determined activity level of the signaling pathway associated TF element in the sample of the subject. This is described in detail in the published international patent application WO 2014/102668 A2 ("Assessment of cellular signaling pathway activity using linear combination(s) of target gene expressions").

Further details regarding the inferring of cellular signaling pathway activity using mathematical modeling of target gene expression can be found in W Verhaegh et al., "Selection of personalized patient therapy through the use of knowledge-based computational models that identify tumor-driving signal transduction pathways", Cancer Research, Vol. 74, No. 11, 2014, pages 2936 to 2945.

To facilitate rapid identification of references, the above-mentioned references have been assigned to each signaling pathway of interest here and exemplarily corresponding target genes suitable for determination of the signaling pathway's activity have been indicated. In this respect, particular reference is also made to the sequence listings for the target genes provided with the above-mentioned references.
AR: KLK2, PMEPA1, TMPRSS2, NKX3 1, ABCC4, KLK3, FKBP5, ELL2, UGT2B15, DHCR24, PPAP2A, NDRG1, LRIG1, CREB3L4, LCP1, GUCY1A3, AR and EAF2 (WO 2013/011479, WO 2014/102668); KLK2, PMEPA1, TMPRSS2, NKX3 1, ABCC4, KLK3, FKBP5, ELL2, UGT2B15, DHCR24, PPAP2A, NDRG1, LRIG1, CREB3L4, LCP1, GUCY1A3, AR, and EAF2 (WO 2014/174003);
JAK-STAT1/2: BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFD1L, USP18, and ZNRF3, preferably, from the group consisting of: IRF1, IRF7, IRF8, IRF9, OAS1, PDCD1, ST13, STAT1 and USP18 (WO 2019/068562A1);
JAK-STAT3: AKT1, BCL2, BCL2L1, BIRC5, CCND1, CD274, CDKN1A, CRP, FGF2, FOS, FSCN1, FSCN2, FSCN3, HIF1A, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST1, VIM and ZEB1 (WO 2019/068543 A1);
MAPK-AP-1: BCL2L11, CCND1, DDIT3, DNMT1, EGFR, ENPP2, EZR, FASLG, FIGF, GLRX, IL2, IVL, LOR, MMP1, MMP3, MMP9, SERPINE1, PLAU, PLAUR, PTGS2, SNCG, TIMP1, TP53 and VIM (WO 2019/120658 A1);
NFkB: BCL2L1, BIRC3, CCL2, CCL3, CCL4, CCL5, CCL20, CCL22, CX3CL1, CXCL1, CXCL2, CXCL3, ICAM1, IL1B, IL6, IL8, IRF1, MMP9, NFKB2, NFKBIA, NFKB IE, PTGS2, SELE, STAT5A, TNF, TNFAIP2, TNIP1, TRAF1 and VCAM1 (WO 2017/029215);
TGF-β: ANGPTL4, CDC42EP3, CDKNIA, CDKN2B, CTGF, GADD45A, GADD45B, HMGA2, ID1, IL11, SERPINE1, INPP5D, JUNB, MMP2, MMP9, NKX2-5, OVOL1, PDGFB, PTHLH, SGK1, SKIL, SMAD4, SMAD5, SMAD6, SMAD7, SNAI1, SNAI2, TIMP1 and VEGFA (WO 2016/062891, WO 2016/062893);

### Determination of interferon (IFN) type I or type II domination in the JAK-STAT1/2 signaling pathway.

For the STAT1/2 pathway assay, the pathway model used for Affymetrix datasets comprises 23 target genes and is calibrated using the GSE38351 dataset. The JAK-STAT1/2 uses for calibration monocytes stimulated with or wothout Interferon. For the RNAseq data discussed below an RNA sequencing specific model was constructed using 12 target genes. This model is calibrated on a THP1 cell line stimulated with or without (control) IFNalpha.

### COVID-19

A subset of COVID-19 patients [13] develop serious pneumonia, ARDS, and pulmonary emboli, with high mortality rate [12].

The severity of acute COVID-19 disease is determined by the inflammatory response, that is governed by the innate immune response. Prognosis is dependent on the extent of the inflammatory response and the ability of the adaptive immune response to mount a sufficient T-cell and B-cell response (with antibody production) to the invasive virus. A shift in immune response balance towards decreased adaptive immune and increased innate inflammatory response confers a bad prognosis.

### Monitoring of severity of COVID-19

Measurement of the status of the innate and adaptive immune response during the disease will enable improved monitoring of the severity state of the patient, which is essential for timely initiation of treatments, such as artificial ventilation, but also start and stop of antiinflammatory drugs, or other drugs that influence the activity of innate and adaptive immune response.

Current severity monitoring is based on routine blood diagnostics, such as CRP levels as a measure for inflammation, and white blood cell counts as a sole measure for the activity of the immune response. The functional state of blood cells, that is, how active are the white blood cells with respect to innate immune response and adaptive immune response, is not part of the current diagnostic options.

### Determination of prognosis in COVID-19

Prognosis of the clinical outcome of a patient with COVID-19 depends on the balance between the innate and adaptive immune response. A strong adaptive immune response is expected to confer a good prognosis. As described above, measurement of the functional state of the innate and adaptive immune response is currently not possible.

### Choice of, and efficacy monitoring of, an immunomodulatory treatment in COVID-19

Many drugs have been tried in acute COVID-19 disease, aiming at reduction of the viral load or mitigation of the inflammatory response. So far, dexamethasone treatment has proven to have a beneficial effect, however this broad immunosuppressive treatment also interferes with mounting an adequate adaptive immune response, and may therefore negatively impact longer term prognosis.

There is a high need for other more selective immunomodulatory treatments that enable reduction of the inflammatory response, while stimulating, or at least not interfering with, the adaptive immune response.

The COVID-19 patient population is characterized by a large clinical variety, e.g. in genetic background, in comorbidities, in use of drugs prior to onset of COVID-19. This is an important reason behind the fact that only a subgroup of patients reacts beneficial to all the different treatments that have been tried.

To enable a more rational and more clinically effective therapeutic approach it is necessary to measure the functional state of the immune response and more specifically, the cellular mechanisms that determine the state of the innate and of the adaptive immune response. Those cellular mechanism are signal transduction pathways, like the androgen receptor (AR), estrogen receptor (ER), progesterone receptor (PR), NFkB, PI3K-FOXO, MAPK-AP1, JAK-STAT1/2, JAK-STAT3, TGFbeta, Notch, Hedgehog, Wnt pathways. Especially important are the AR, MAPK-AP1 and JAK-STAT1/2 pathways. More recently developed drugs are directed towards specific inhibition of one of these signaling pathways, so-called targeted drugs, while older drugs are often known to inhibit one or more of these pathways. For example dexamethasone is known to inhibit activity of the NFkB pathway.

Measurement of the activity of these signaling pathways in white blood cells enables the determination of the functional state of the innate and the adaptive immune response.

Since current diagnostics do not allow measurement of the activity of these signaling pathways it has not been possible to choose a targeted drug treatment on a personalized treatment basis in patients with COVID-19. Personalized treatment means choosing the drug that is expected to be effective in the specific patient based on a biomarker measurement.

For the same reason, it is not possible to assess whether a treatment has the wanted effect on the immune response, that is, the effect cannot be monitored in a patient. Also adverse effects of a treatment on the immune response in a patient cannot be measured.

Summarising: there is a high need for tests to measure the cellular mechanisms (signal transduction pathway activities) that determine the functional state of the immune response in a patients with COVID-19, to (1) enable assessment of severity of the disease to take the best clinical action; (2) to predict response to, and enable monitoring of the effect of, an immunomodulatory therapy;

This invention describes how new assays to quantitatively measure activity of the signal transduction pathways that determine the innate and adaptive immune response, simultaneously on a blood sample, can be used to: assess severity of COVID-19, monitor severity, choose targeted drug treatment, and monitor effectiveness of targeted drug treatment.

In view of the data presented for the COVID datasets as well as the Dengue Fever, RSV, etc. it can be concluded that viral infection triggers the adaptive immune response which is illustrated by the increase in JAK-STAT1/2 pathway activity. It is however noted that particularly in more critical infections, this elevation in JAK-STAT1/2 pathway activity is not observed, or observed to a much lesser extent. This is particularly pronounced in the COVID-19 datasets, but was also found to be true for other virusses. Without wishing to be bound be theory, it is hypothesized that this lower JAK-STAT1/2 pathway activity in critical patients is indicative for failure to mount a proper adaptive immune response which may be one of the causes of the severity of the disease. It is expected that in viral infected patients the JAK-STAT1/2 pathway activity as measured in whole blood, will initially increase (during the acute phase) and then decrease to "normal" levels in the recovery phase. Absence of an increase in the JAK-STAT1/2 pathway activity suggests thus a failure to mount a proper adaptive immune respnose and has a strong correlation to a more severe symptoms. Therefore, the JAK-STAT1/2 pathway activity in a blood sample from a patient in the acute phase can predict severeness of the viral infection, based on the perceived mounted adaptive immune response. This is useful as these patients may receive additional care or different treatment in view of the reduced / absent adaptive immune response.

Therefore, in an aspect the invention relates to a method a method for stratifying a subject with a viral infection as critical based on a blood sample obtained from a subject with a confirmed viral infection, based on the determined expression levels of three or more target genes of the JAK-STAT1/2 cellular signaling pathway, the method comprising:
- receiving the determined expression levels of the three or more target genes of the JAK-STAT1/2 cellular signaling pathway;
- determining the JAK-STAT1/2 cellular signaling pathway activity, wherein the determining the JAK-STAT1/2 cellular signaling pathway activity comprises assigning a numeric value to the JAK-STAT1/2 cellular signaling pathway activity level by evaluating a calibrated mathematical pathway model relating the expression levels of the three or more JAK-STAT1/2 target genes to an activity level of the JAK-STAT1/2 cellular signaling pathway; and

wherein the method further comprises determining an activity level of a signaling pathway associated transcription factor (TF) element, the signaling pathway associated TF element controlling transcription of the three or more target genes, the determining being based on evaluating a calibrated mathematical pathway model relating expression levels of the target genes to the activity level of the JAK-STAT1/2 cellular signaling pathway, and
inferring the activity of the JAK-STAT1/2 cellular signaling pathway in the blood sample based on the determined activity level of the signaling pathway associated TF element,
comparing the JAK-STAT1/2 cellular signaling pathway activity determined in the blood sample obtained from the subject with the JAK-STAT1/2 cellular signaling pathway activity determined in a reference blood sample,
wherein the reference blood sample is obtained from a healthy subject,
and wherein the subject with the viral infection is is considered critical if the determined JAK-STAT1/2 pathway activity is equal or lower than the JAK-STAT1/2 pathway activity determined in the reference blood sample of the healthy subject, or wherein the subject with the viral infection is considered not critical if the determined JAK-STAT1/2 pathway activity is higher than the JAK-STAT1/2 pathway activity determined in the reference blood sample of the healthy subject,
wherein said blood sample from the subject with the confirmed viral infection has been obtained during the acute phase of said infection
wherein the calibrated mathematical pathway model is a centroid or a linear model, or a Bayesian network model based on conditional probabilities, and
wherein the three or more target genes of the JAK-STAT1/2 cellular signaling pathway are selected from the group consisting of:
   BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFD1L, USP18, and ZNRF3.

In an embodiment the viral infection is a coronavirus infection, preferably a SARS or a MERS infection, more preferably a COVID-19 infection.

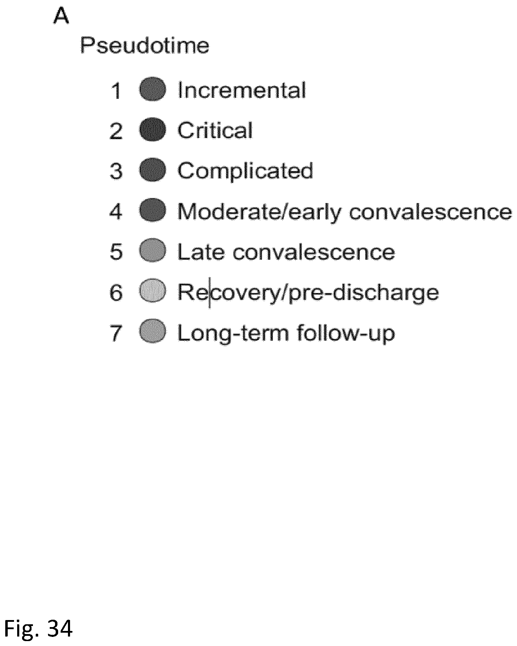

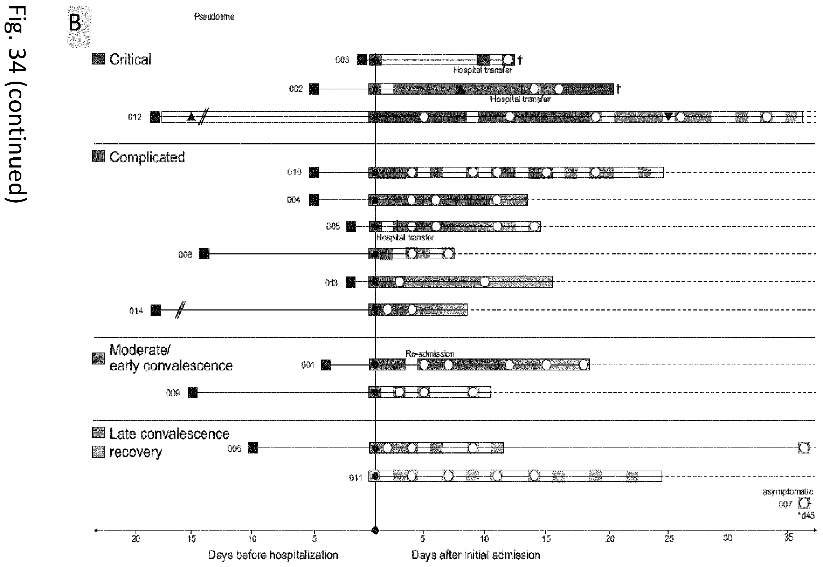

It was further found that in COVID-19 patiets AR signaling pathway activity correlates with severeness of the infection, where a higher AR pathweay activity is found in more severe infection (see e.g. Fig. 34). In fact AR pathway inhibitors are currently being tried as a viable treatment option for COVID-19. Our results suggest that a subset of patients (those with elevated AR pathway activity) would benefit from such treatment. Therefore a patient stratification is desirable. The pathway analysis developed by the inventors allows for the first time a quantiative determination of pathway activity in a blood sample from a subject.

When used herein, the term critical when referring to a subject with a viral infection refers to a subject having a clinically serious disease. For example the term may refer to a disease state where the subject any one of unstable vital signs outside the normal range or not normal vital signs, has questionable or unfavorable indicators for recovery, or where the subject is unconscious.

Therefore, in an aspect the disclosure relates to a method for stratifying a subject with a COVID-19 infection for suitability for treatment with an AR pathway inhibitor based based on a blood sample obtained from the subject with a COVID-19 infection, based on the determined expression levels of three or more target genes of the AR cellular signaling pathway, the method comprising:
receiving the determined expression levels of the three or more target genes of the AR cellular signaling pathway;
   - determining the AR cellular signaling pathway activity based on evaluating a calibrated mathematical pathway model relating the expression levels of the three or more AR target genes to an activity level of the family of AR transcription factor (TF) elements, the family of AR TF elements controlling transcription of the three or more AR target genes, the activity of the AR cellular signaling pathway being defined by the activity level of the family of AR TF elements, the calibrated mathematical pathway model being a model that is calibrated using a ground truth dataset including samples in which transcription of the three or more AR target genes is induced by the family of AR TF elements and samples in which transcription of the three or more AR target genes is not induced by the family of AR TF elements,
wherein the AR cellular signaling pathway refers to a signaling process that leads to transcriptional activity of the family of AR TF elements, and wherein the family of AR TF elements are protein complexes each containing a homodimer or a heterodimer comprising AR-A and/or AR-B,
wherein the determining the AR cellular signaling pathway activity comprises assigning a numeric value to the AR cellular signaling pathway activity level by evaluating the calibrated mathematical pathway model relating expression levels of the target genes to the activity level of the AR cellular signaling pathway;
comparing the AR cellular signaling pathway activity determined in the blood sample obtained from the subject with the AR cellular signaling pathway activity determined in a reference blood sample,
wherein the reference blood sample is obtained from a healthy subject,
and wherein the subject with the COVID-19 infection is not a candidate for treatment with an AR inhibitor if the determine AR pathway activity is equal or lower than the AR pathway activity determined in the reference blood sample of the healthy subject, or wherein the subject with the COVID-19 infection is is a candidate if the determined AR pathway activity is higher than the AR pathway activity determined in the reference blood sample of the healthy subject.

In an aspect of the methods described herein are a computer implemented method.

When used herein the relative terms high (higher), low (lower) or equal (similar) when referring to pathway activity relative to a standard or reference are to be interpreted as follows:
In case the standard or reference is determined based on pathway activities measured in multiple samples the standard or reference can be set as a range of the mean (or average) value plus or minus one standard deviation. If the value measured in the sample is above the mean (or average) plus one standard deviation or below the mean (or average) minus one standard deviation the pathway activity is determined to be high or low respwectively. Alternatively the reference value can be set as a predefined range of values, where a value above or below this range constitutes a high or low pathway activity. The latter may be useful if only a single reference value is available and/or no meaningful mean (or average) can be calculated.

The disclosure further provides an apparatus for distinguishing between a bacterial and a viral infection in a blood sample and/or for determining the cellular immune response to a viral infection or a vaccine in a blood sample, comprising at least one digital processor configured to perform the method of the first and/or the second aspect of the invention and the various embodiments thereof. In a preferred aspect the disclosure relates to an apparatus for distinguishing between a bacterial and a viral infection in a blood sample and/or for determining the cellular immune response to a viral infection or a vaccine in a blood sample, the apparatus comprising a digital processor configured to perform the method according to any one of the preceding claims, comprising an input adapted to receive data indicative of a target gene expression profile for the three or more target genes of the JAK-STAT1/2 cellular signaling pathway, optionally data indicative of a target gene expression profile for the three or more target genes of the JAK-STAT3 cellular signaling pathway and/or the TGFbeta signaling pathway and/or the AR signaling pathway and/or the MAPK-AP1 cellular signaling pathway.

In a fourth aspect of the invention is provided a non-transitory storage medium for distinguishing between a bacterial and a viral infection in a blood sample and/or for determining the cellular immune response to a viral infection or a vaccine in a blood sample, storing instructions that are executable by a digital processing device to perform the method of the first and/or the second aspect of the invention and the various embodiments thereof. In a preferred embodiment the invention relates to a computer program product comprising instructions which, when the program is executed by a computer, cause the computer to carry out a method comprising:
- receiving data indicative of a target gene expression profile for three or more target genes of the JAK-STAT1/2 cellular signaling pathway, optionally further receiving data indicative of the target gene expression levels of three or more target genes of the JAK-STAT3 cellular signaling pathway and/or the TGFbeta signaling pathway and/or the AR signaling pathway and/or the MAPK-AP1 cellular signaling pathway,
- determining the JAK-STAT1/2 cellular signaling pathway activity, and optionally JAK-STAT3 cellular signaling pathway activity and/or the TGFbeta signaling pathway activity and/or the AR signaling pathway activity and/or the MAPK-AP1 cellular signaling pathway activity based on the determined expression levels of said three or more target genes of the JAK-STAT1/2 cellular signaling pathway and optionally the JAK-STAT3 cellular signaling pathway and/or the TGFbeta signaling pathway and/or the AR signaling pathway and/or the MAPK-AP1 cellular signaling pathway.

The non-transitory storage medium may be a computer-readable storage medium, such as a hard drive or other magnetic storage medium, an optical disk or other optical storage medium, a random access memory (RAM), read only memory (ROM), flash memory, or other electronic storage medium, a network server, or so forth. The digital processing device may be a handheld device (e.g., a personal data assistant or smartphone), a notebook computer, a desktop computer, a tablet computer or device, a remote network server, or so forth.

In a fifth aspect of the invention is provided a computer program for distinguishing between a bacterial and a viral infection in a blood sample and/or for determining the cellular immune response to a viral infection or a vaccine in a blood sample, comprising program code means for causing digital processing device to perform the method of the first and/or the second aspect of the invention and the various embodiments thereof, when the computer program is run on a digital processing device. The computer program may be stored/distributed on a suitable medium, such as an optical storage medium or a solid-state medium, supplied together with or as part of other hardware, but may also be distributed in other forms, such as via the Internet or other wired or wireless telecommunication systems.

In a sixth aspect of the invention is provided a kit of parts, comprising components for determining the expression levels of three or more, e.g. three, four, five, six, seven eight, nine, ten, eleven, twelve, thirteen or more, target genes of the JAK-STAT1/2 cellular signaling pathway and optionally three or more, e.g. three, four, five, six, seven eight, nine, ten, eleven, twelve, thirteen or more, target genes of the JAK-STAT3 cellular signaling pathway,
wherein the three or more, e.g. three, four, five, six, seven eight, nine, ten, eleven, twelve, thirteen or more, target genes of the JAK-STAT1/2 cellular signaling pathway are selected from the group consisting of:
BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFD1L, USP18, ZNRF3, GBP1, TAP1, ISG15, APOL1,, IFI6, IFIRM1, CXCL9, APOL2, IFIT2 and LY6E, preferably, from the group consisting of: IRF1, IRF7, IRF8, IRF9, OAS1, PDCD1, ST13, STAT1 and USP1, or from the group consisting of GBP1, IRF9, STAT1, TAP1, ISG15, APOL1, IRF1, IRF7, IFI6, IFIRM1, USP18, CXCL9, OAS1, APOL2, IFIT2 and LY6E and optionally
wherein the three or more, e.g. three, four, five, six, seven eight, nine, ten, eleven, twelve, thirteen or more, target genes of the JAK-STAT3 cellular signaling pathway are selected from the group consisting of: AKT1, BCL2, BCL2L1, BIRC5, CCND1, CD274, CDKNIA, CRP, FGF2, FOS, FSCN1, FSCN2, FSCN3, HIFIA, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST1, VIM and ZEB1, and
wherein the components for determining the expression level are primers and probes, and optionally further comprising the apparatus according to the third aspect of the invention, the non-transitory storage medium of the fourth aspect of the invention or the computer program of the fifth aspect of the invention.

In a seventh aspect of the invention is provided use of the kit of the sixth aspect of the invention in the method for distinguishing between a bacterial and a viral infection in a blood sample obtained from a subject with an infection, based on the determined expression levels of three or more target genes of the JAK-STAT1/2 cellular signaling pathway, the method comprising: determining the expression levels of the three or more target genes of the JAK-STAT1/2 cellular signaling pathway; determining the JAK-STAT1/2 cellular signaling pathway activity, wherein the determining the JAK-STAT1/2 cellular signaling pathway activity comprises assigning a numeric value to the JAK-STAT1/2 cellular signaling pathway activity level by evaluating a calibrated mathematical pathway model relating the expression levels of the three or more JAK-STAT1/2 target genes to an activity level of the JAK-STAT1/2 cellular signaling pathway, wherein the method further comprises determining an activity level of a signaling pathway associated transcription factor (TF) element, the signaling pathway associated TF element controlling transcription of the three or more target genes, the determining being based on evaluating a calibrated mathematical pathway model relating expression levels of the target genes to the activity level of the JAK-STAT1/2 cellular signaling pathway, and inferring the activity of the JAK-STAT1/2 cellular signaling pathway in the blood sample based on the determined activity level of the signaling pathway associated TF element, comparing the JAK-STAT1/2 cellular signaling pathway activity determined in the blood sample obtained from the subject with the JAK-STAT1/2 cellular signaling pathway activity determined in a reference blood sample, wherein the reference blood sample is obtained from a healthy subject or a subject recovered from an infection, and wherein the infection in the subject from which the blood sample is obtained is determined to be viral when the JAK-STAT1/2 cellular signaling pathway activity is higher compared to the JAK-STAT1/2 cellular signaling pathway activity in the reference blood sample, or wherein the infection in the subject from which the blood sample is obtained is determined to be bacterial when the JAK-STAT1/2 cellular signaling pathway activity is not higher compared to the JAK-STAT1/2 cellular signaling pathway activity in the reference blood sample, wherein the calibrated mathematical pathway model is a centroid or a linear model, or a Bayesian network model based on conditional probabilities, and wherein the three or more target genes of the JAK-STAT1/2 cellular signaling pathway are selected from the group consisting of: BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFD1L, USP18, and ZNRF3.

The disclosure further provides for use of the kit according to the sixth aspect of the invention in the *in vitro* or *ex vivo* diagnosis of a viral infection in a subject. Preferably in a subject with an infection.

This application describes several preferred embodiments. Modifications and alterations may occur to others upon reading and understanding the preceding detailed description. It is intended that the application is construed as including all such modifications and alterations insofar as they come within the scope of the appended claims or the equivalents thereof.

Other variations to the disclosed embodiments can be understood and effected by those skilled in the art in practicing the claimed invention, from a study of the drawings, the disclosure, and the appended claims.

It shall be understood that the methods of the first aspect, the computer implemented invention of the second aspect, the apparatus of the third aspect, the non-transitory storage medium of fourth aspect, the computer program of the fifth aspect, the kits of the sixth aspect have similar and/or identical preferred embodiments, in particular, as defined in the dependent claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Calculations like the determination of the mortality risk performed by one or several units or devices can be performed by any other number of units or devices.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

General: In all the figures where signal transduction pathway analysis scores are depicted, these are given as log2odds scores for pathway activity, derived from the probability scores for pathway activity provided by the Bayesian pathway model analysis. Log2odds scores indicate the level of activity of a signaling pathway on a linear scale.

Analyzed public datasets are indicated with their GSE number (in principle at the bottom of each figure), and individual samples with their GSM number (in principle most right column for clustering diagrams).

All validation samples for a signaling pathway model or an immune response/system model are independent samples and have not been used for calibration of the respective model to be validated.
Fig. 1 depicts a diagram showing different cells in the blood involved in the active immune response. The immune respons can be divided in the innate immune response and adaptive immune response, each of which is controlled by different cell types as depicted.
Fig. 2 depicts pathway activity analysis performed on GSE6269. Peripheral blood samples from pediatric patients with acute infections of *Influenza, S. pneumoniae* or S. *aureus* were used for the pathway analysis. Only significant pathways using Mann-Whitney-Wilcoxon test are included in the figure. Both the JAK-STAT1/2 type I and type II interferon are increased in only influenza but not in the bacterial infections.

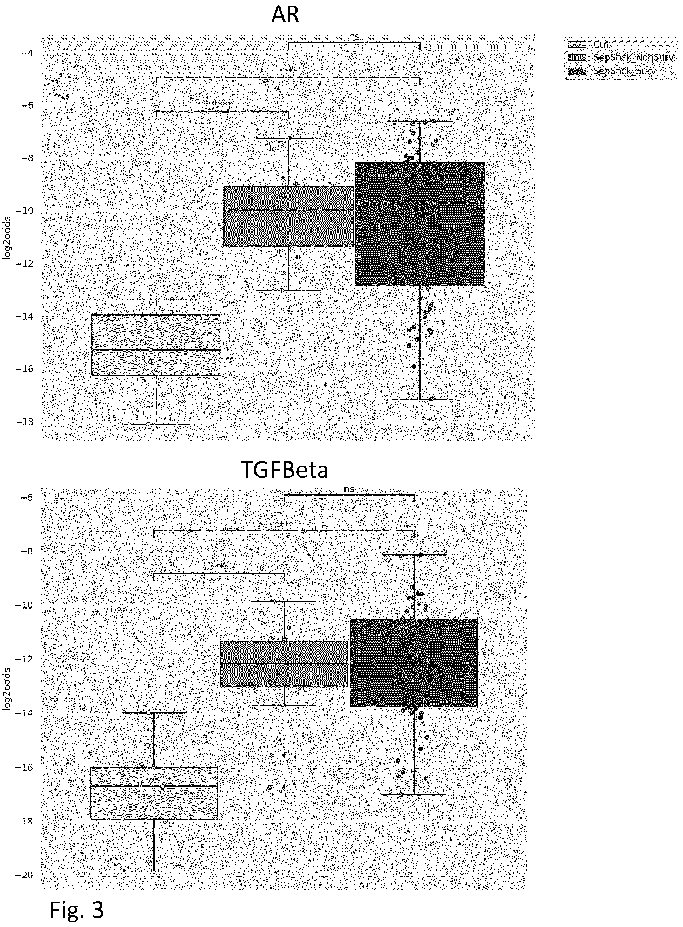

Fig. 3 depicts pathway activity analysis of dataset GSE4607 of children <10 years of age admitted to the pediatric intensive care unit and meeting the criteria for septic shock caused by a bacterial infection. Shown are AR and TGFbeta signaling activity. Significance was calculated using Mann-Whitney-Wilcoxon test and are included in the figure.

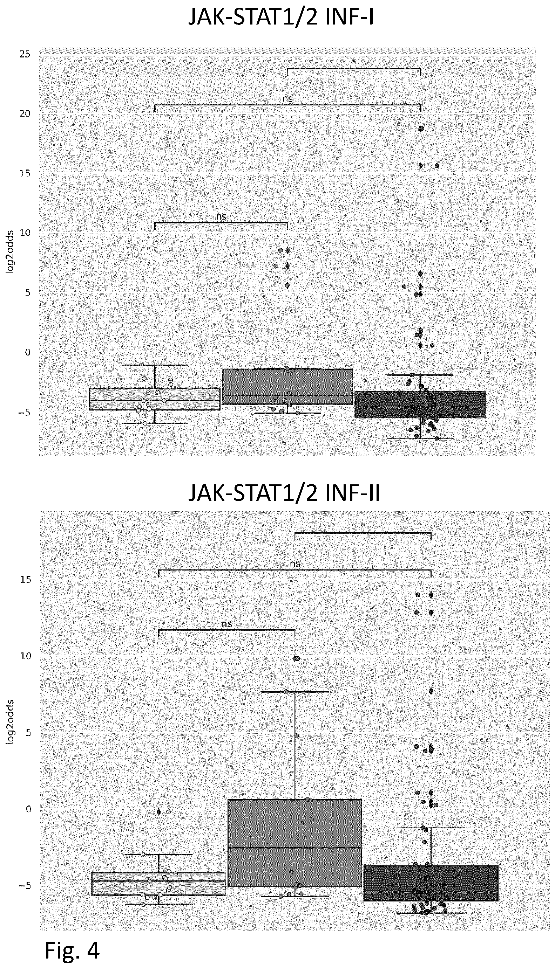

Fig. 4 depicts pathway activity analysis of dataset GSE4607 of children <10 years of age admitted to the pediatric intensive care unit and meeting the criteria for septic shock caused by a bacterial infection. Shown are JAK-STAT1/2 type I and type II interferon signaling activity. Significance was calculated using Mann-Whitney-Wilcoxon test and are included in the figure.
Fig. 5 depicts pathway analysis of dataset GSE69606 including in pediatric Respiratory Syncytial Virus (RSV) (mild, moderate, severe) and recovery (moderate and severe) patients. Analysis was performed on PBMCs. Shown are JAK-STAT1/2 type I and type II interferon signaling activity.

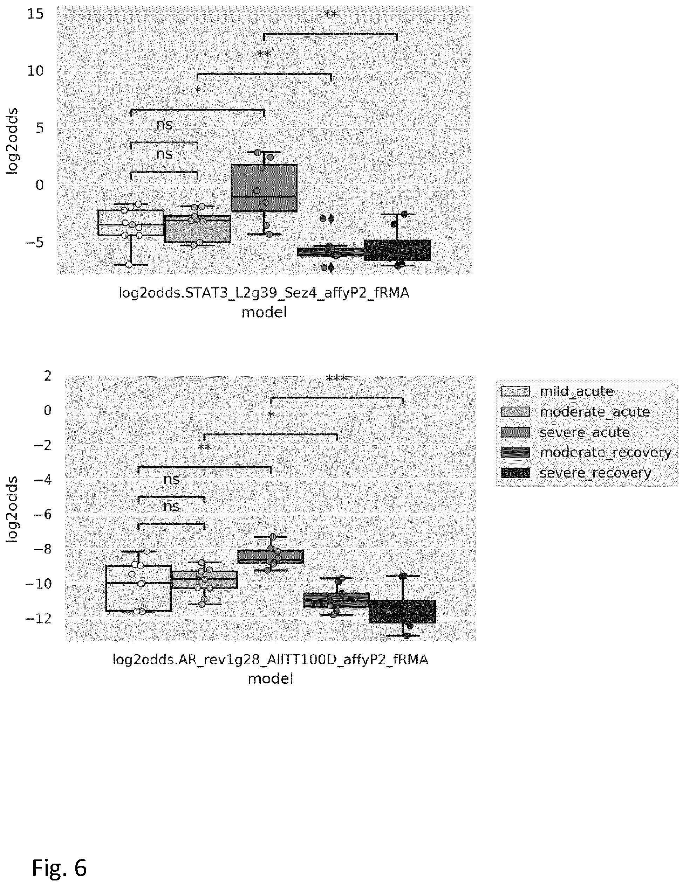

Fig. 6 depicts pathway analysis of dataset GSE69606 including in pediatric Respiratory Syncytial Virus (RSV) (mild, moderate, severe) and recovery (moderate and severe) patients. Analysis was performed on PBMCs. Shown are JAK-STAT3 and AR signaling activity.

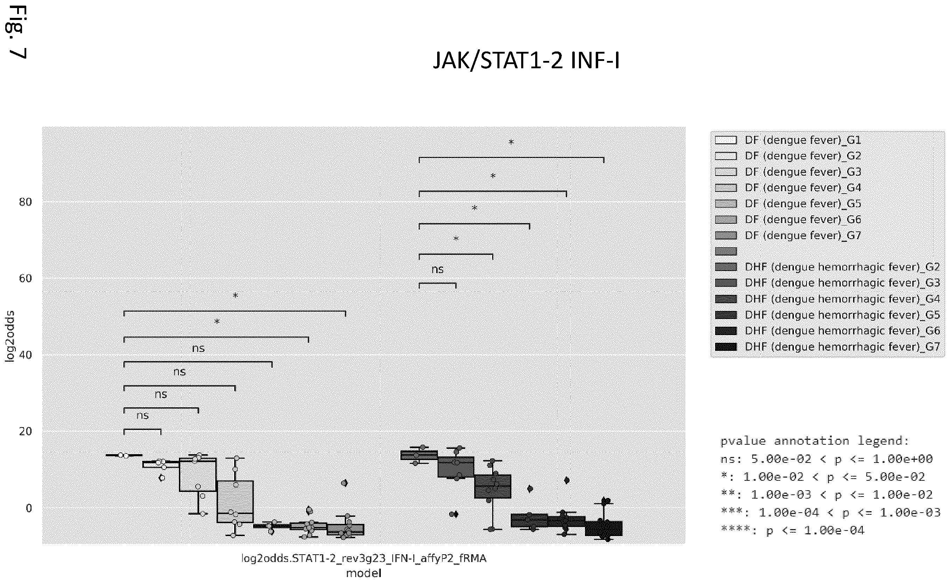

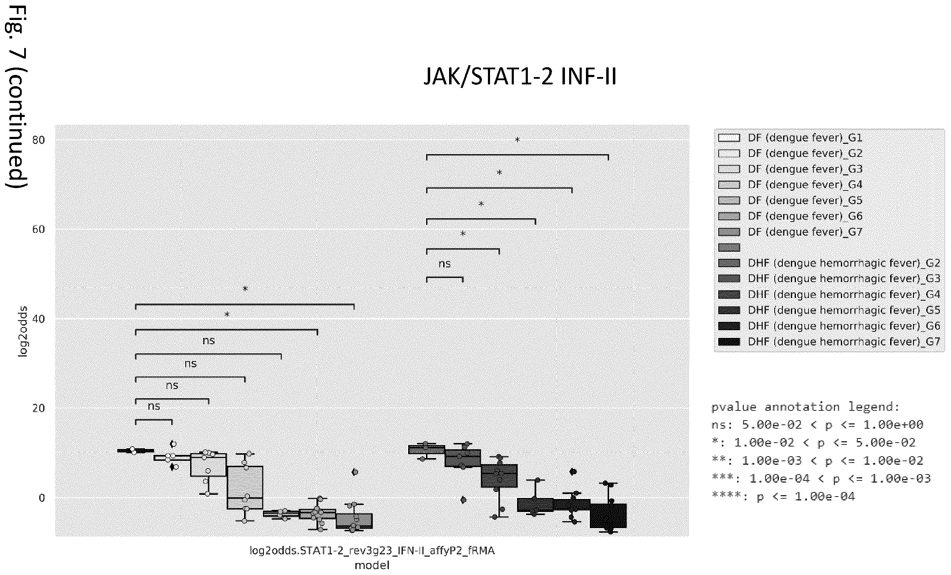

Fig. 7 depicts Pathway analysis of dataset GSE43777, dengue viral infection measured in PBMC samples from DF (dengue fever) and DHF (dengue hemorrhagic fever) with acute infection. JAK-STAT1/2 type I and type II interferon signaling pathway scores including Mann-Whitney-Wilcoxon test scores are shown in the figure. Each stage represents a group of patients after x days from fever onset, Stages (days after onset fever): G1: 0 days, G2: 2 days, G3:4, G4:4 days, G5, 5 days, G6: 6-10 days, G7: >20 days. Legend and significance are shown in figure.

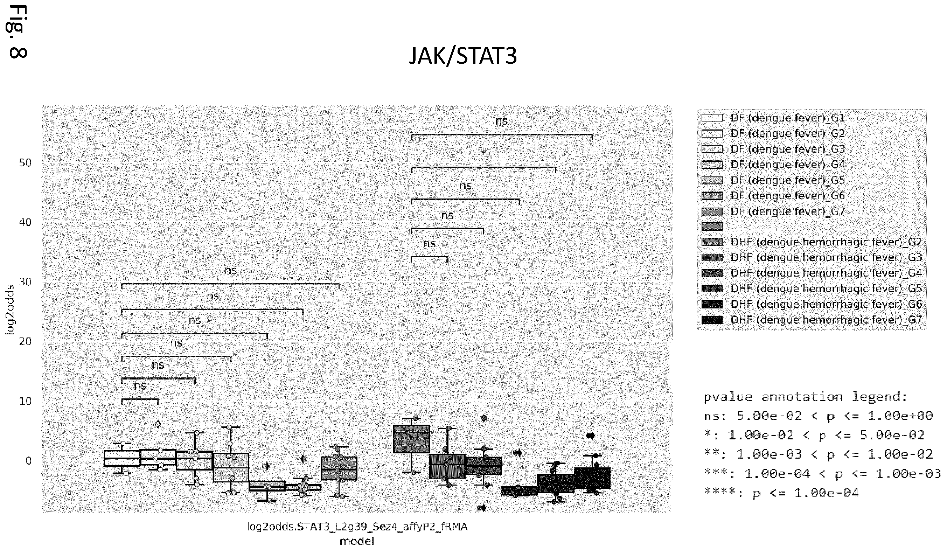

Fig. 8 depicts Pathway analysis of dataset GSE43777, dengue viral infection measured in PBMC samples from DF (dengue fever) and DHF (dengue hemorrhagic fever) with acute infection. JAK-STAT3 signaling pathway scores including Mann-Whitney-Wilcoxon test scores are shown in the figure. Each stage represents a group of patients after x days from fever onset, Stages (days after onset fever): G1: 0 days, G2: 2 days, G3:4, G4:4 days, G5, 5 days, G6: 6-10 days, G7: >20 days. Legend and significance are shown in figure. JAK-STAT3 pathway activity is higher in infections with a more severe clinical course.

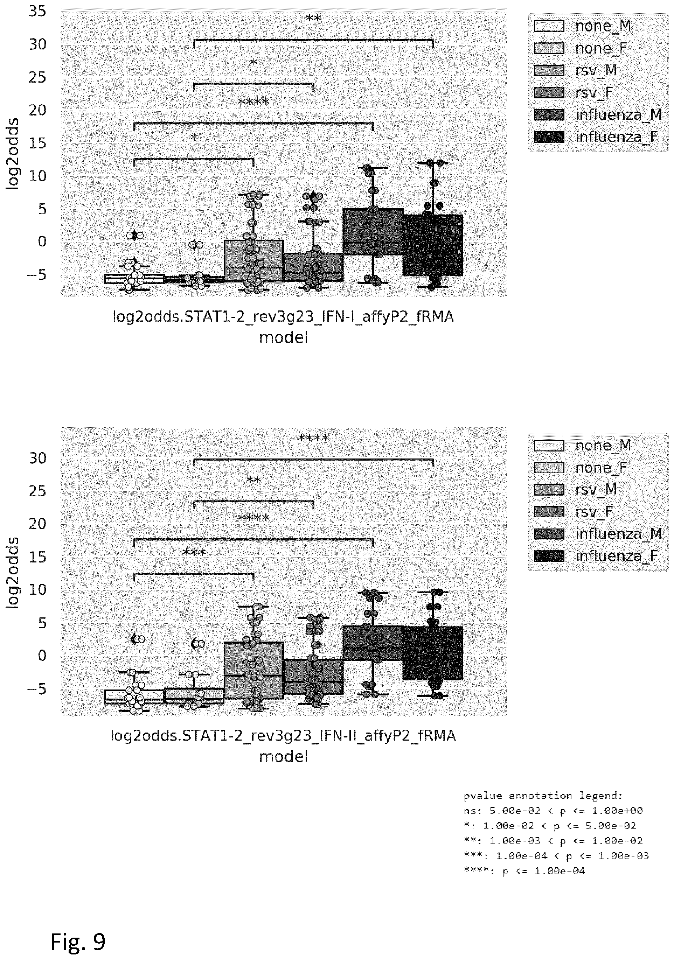

Fig. 9 depicts pathway analysis of dataset GSE34205 including patients hospitalized with acute RSV and influenza virus infection. Blood samples were collected from children within 42-72 hours of hospitalization. The PBMCs were used for the study. The pathways STAT1/2 type I and type II interferon signaling pathway activity are shown and significance determined using Mann-Whitney-Wilcoxon test. Legend and significance are included in the figure. The strength of the generated cellular immunity by the infection is reflected by activity of the JAK-STAT1/2 pathway, and varies with type if viral infection.

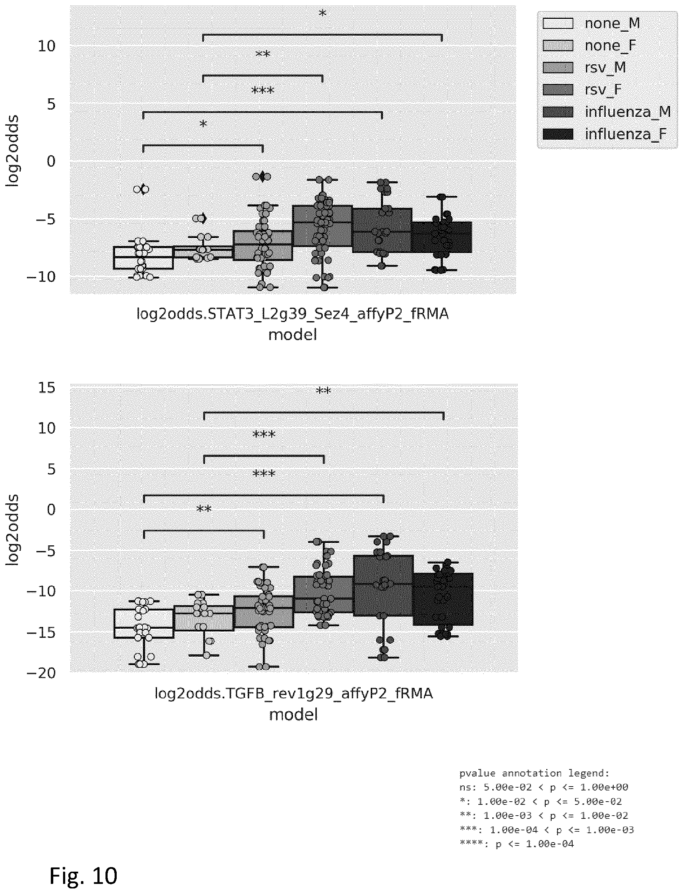

Fig. 10 depicts pathway analysis of dataset GSE34205 including patients hospitalized with acute RSV and influenza virus infection. Blood samples were collected from children within 42-72 hours of hospitalization. The PBMCs were used for the study. The pathways STAT3 and TGFbeta signaling pathway activity are shown and significance determined using Mann-Whitney-Wilcoxon test. Legend and significance are included in the figure.

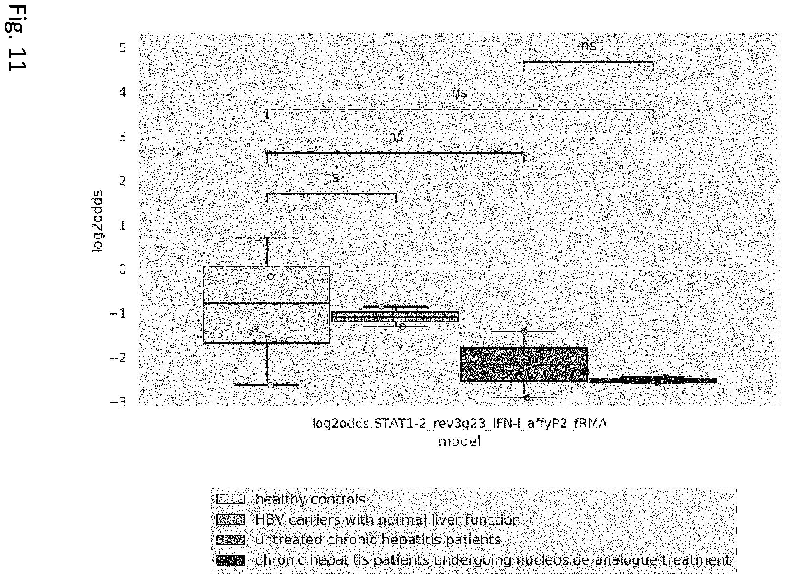

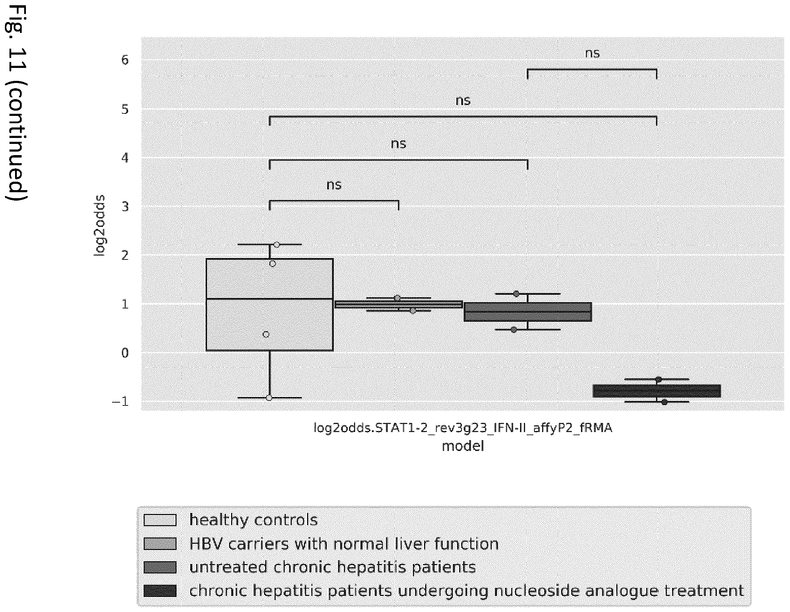

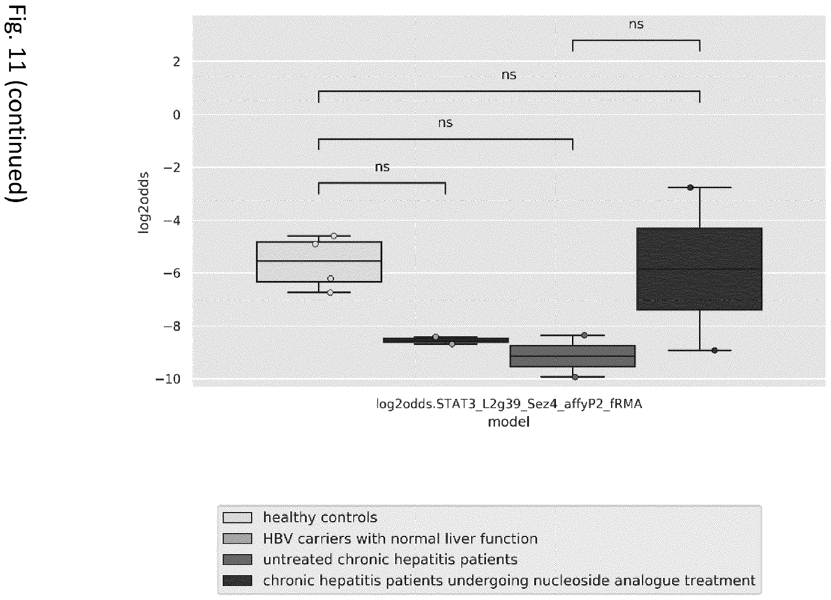

Fig. 11 depicts pathway analysis of dataset GSE119322, dendritic cell function in patients with Hepatitis carriers with normal liver function, untreated chronic hepatitis and chronic hepatitis patients undergoing nucleoside analogue treatment. Pathways JAK-STAT1/2 (type I and type II interferon) and JAK-STAT3 are shown including their significance using Mann-Whitney-Wilcoxon test. JAK-STAT1 pathway activity is lower in chronic hepatitis B patients, with suppressed cellular immunity.

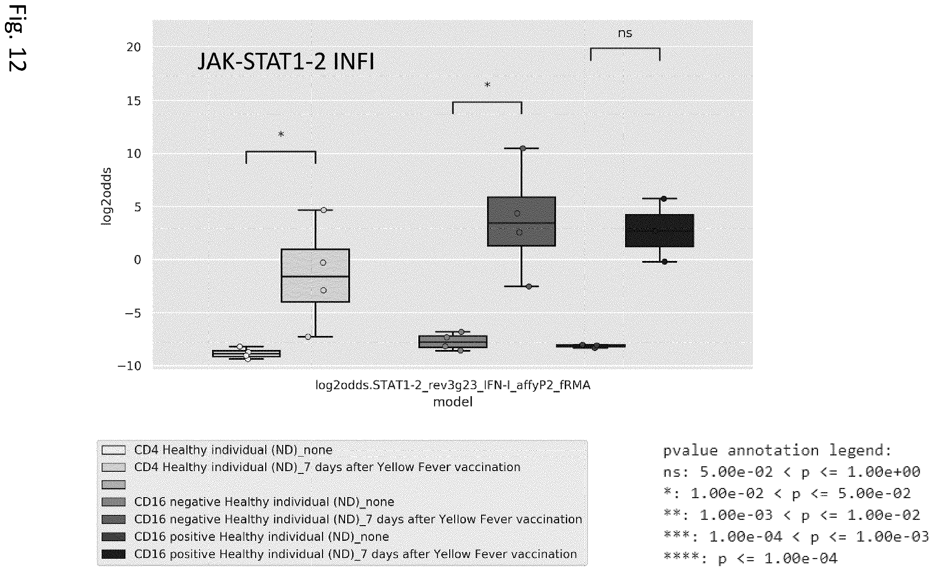

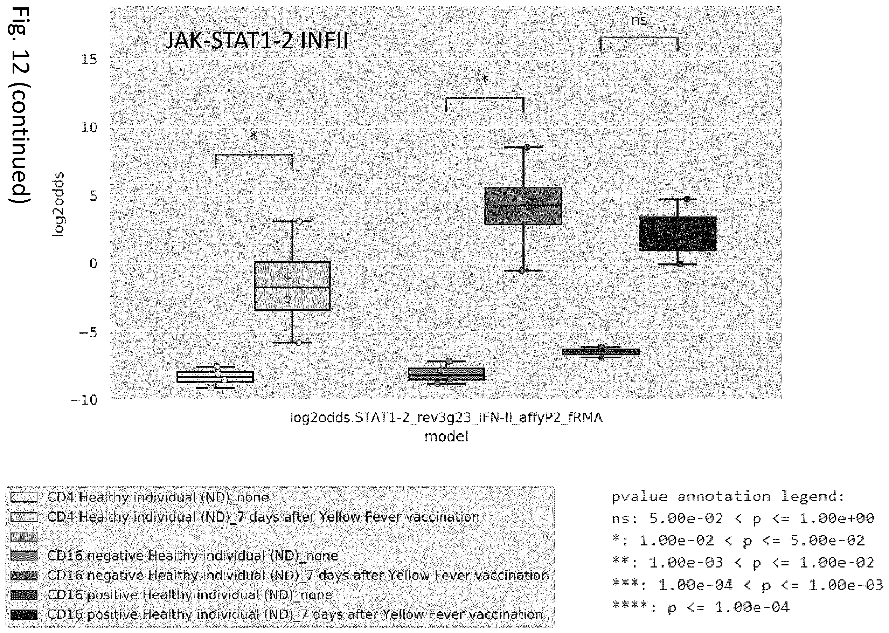

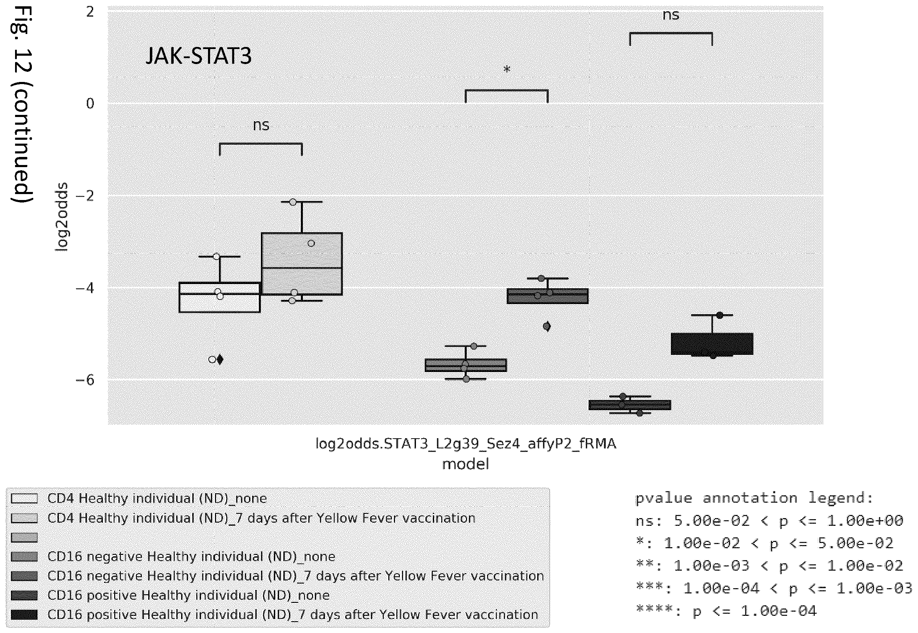

Fig. 12 depicts pathway analysis of dataset GSE51997 comprising healthy and yellow fever (YFV-17D) vaccinated volunteers. CD4 and CD16 negative and positive cells were isolated and used in the study. Depicted are the JAK-STAT1/2 (type I and type II interferon) and the JAK-STAT3 pathway activities. Legend and significance are included in the figure.

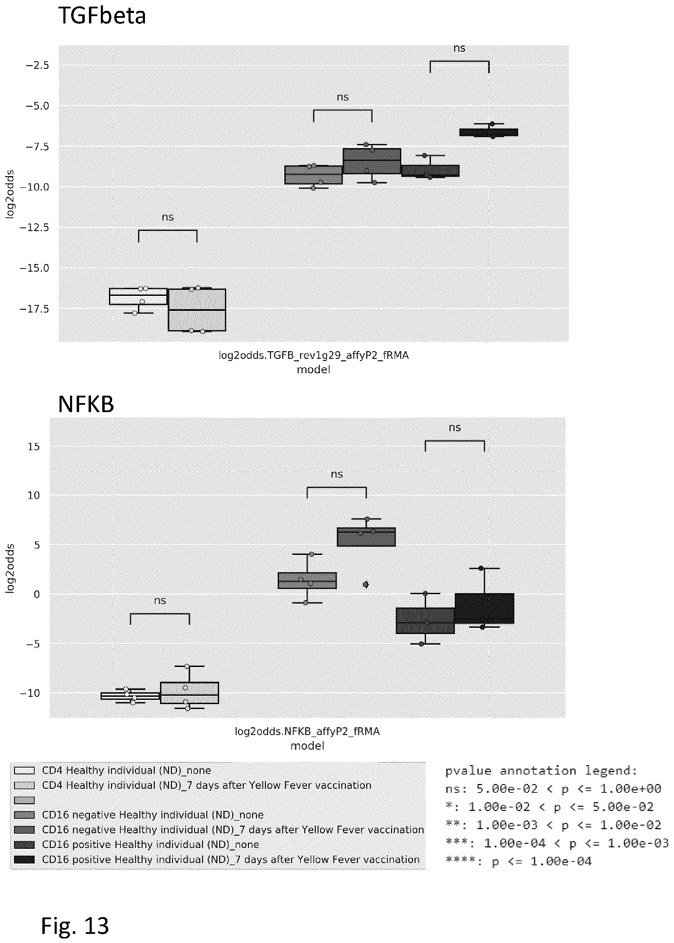

Fig. 13 depicts pathway analysis of dataset GSE51997 comprising healthy and yellow fever (YFV-17D) vaccinated volunteers. CD4 and CD16 negative and positive cells were isolated and used in the study. Depicted are the TGFbeta and the NFkB pathway activities. Legend and significance are included in the figure. JAK-STAT pathway activity increases within days after viral vaccination.

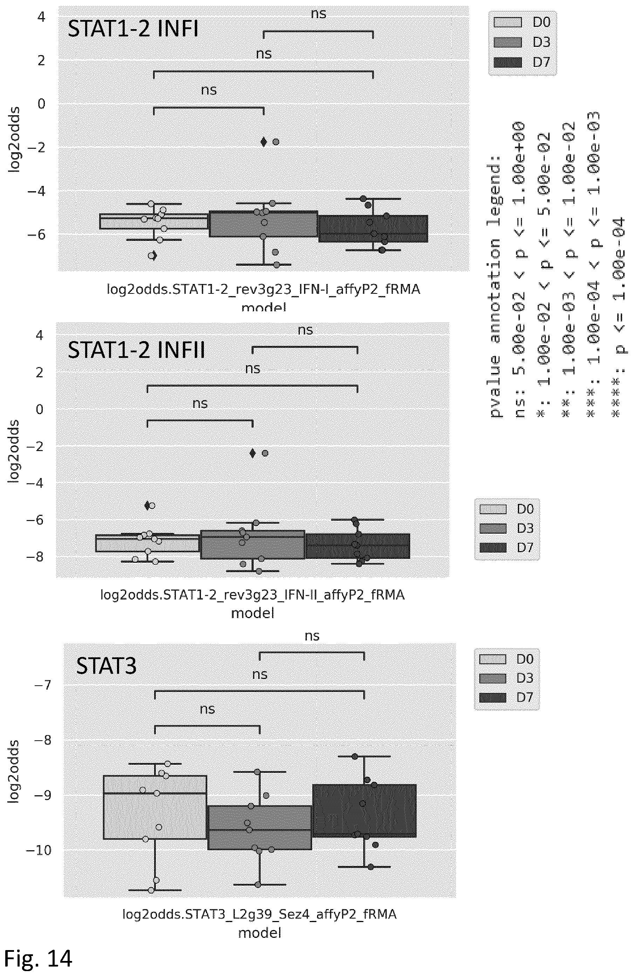

Fig. 14 depicts Pathway analysis of dataset GSE29614. Time Course of Young Adults Vaccinated with Influenza TIV (Trivalent Inactivated Influenza Vaccine) Vaccine during the 2007/08 Flu Season. Blood samples isolated at days 0, 3, 7 days post-vaccination. Depicted are the JAK-STAT1/2 (type I and type II interferon) and the JAK-STAT3 pathway activities. Legend and significance using Mann-Whitney-Wilcoxon test are shown in the figure.

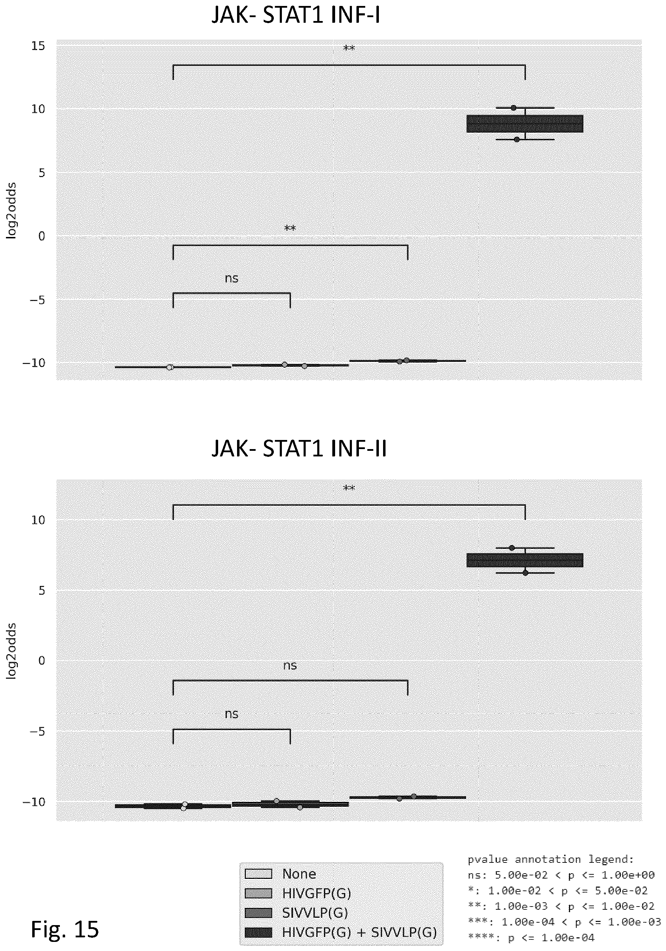

Fig. 15 depicts pathway analysis of dataset GSE22589 containing uninfected human monocyte-derived dendritic cells (MDDCs); MDDCs infected with an envelope-defective GFP-encoding VSV-G-pseudotyped HIV-1 vector (HIVGFP(G)) and with VSV-G pseudotyped virus-like particles derived from SIVmac to deliver Vpx (SIVVLP(G)), alone or in combination. Cells were infected at day 4 of differentiation and harvested 48 hours later. Depicted are the JAK-STAT1/2 (type I and type II interferon) pathway activities. Legend and significance are included in the figure. JAK-STAT1/2 pathway activity is only increased when the combined vectors are introduced into the cells and only this combination was considered as eliciting a successful response.

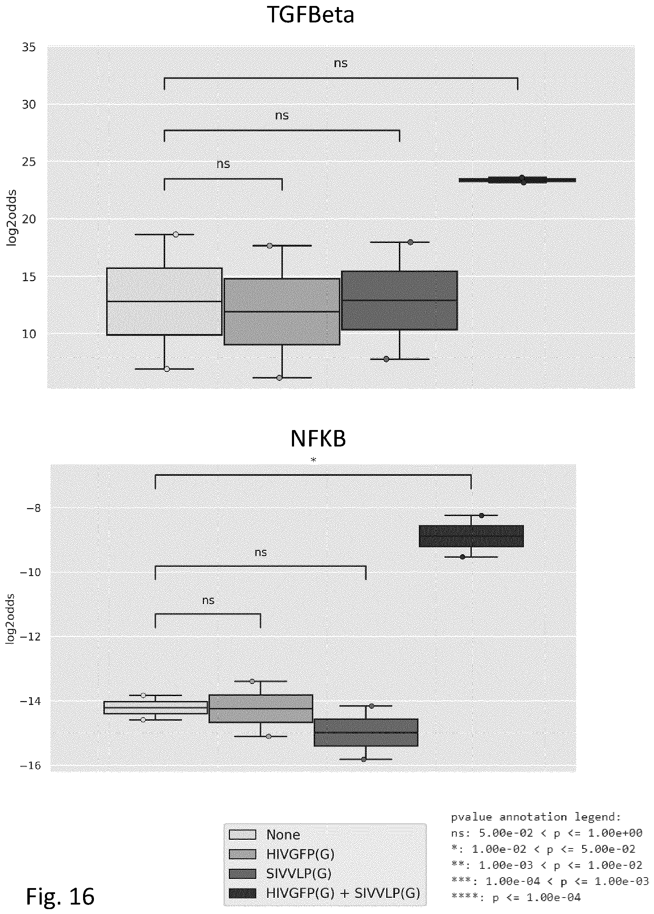

Fig. 16 depicts pathway analysis of dataset GSE22589 containing uninfected human monocyte-derived dendritic cells (MDDCs); MDDCs infected with an envelope-defective GFP-encoding VSV-G-pseudotyped HIV-1 vector (HIVGFP(G)) and with VSV-G pseudotyped virus-like particles derived from SIVmac to deliver Vpx (SIVVLP(G)), alone or in combination. Cells were infected at day 4 of differentiation and harvested 48 hours later. Depicted are the JAK-STAT3 and NFkB pathway activities. Legend and significance are included in the figure.

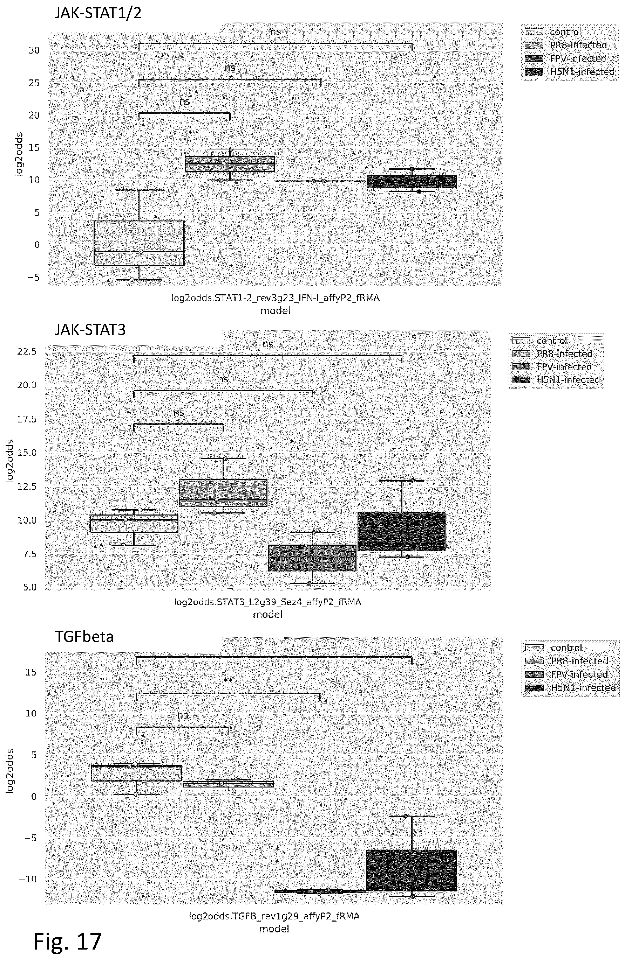

Fig. 17 depicts pathway analysis of dataset GSE35283 containing monocytes infected with low (PR8) and high pathogenic influenza viruses (FPV (H7N7) and H5N1). Depicted are the JAK-STAT1/2 (type I and type II interferon) and the JAK-STAT3 pathway activities. Legend and significance are included in the figure. JAK-STAT1/2 pathway activity distinguishes between low and high pathogenic virus.

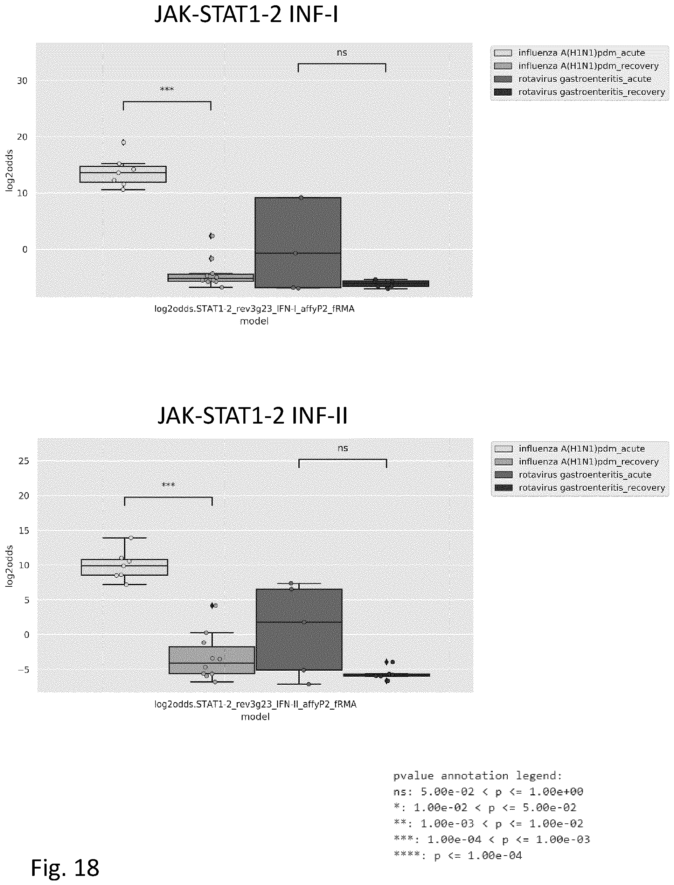

Fig. 18 depicts pathway analysis of dataset GSE50628 of influenza A(H1N1)pdm09 and rotavirus gastroenteritis infected patients. Whole blood samples were collected from patients in the acute phase of the disease and in the recovery phase. Significance was calculated using Mann-Whitney-Wilcoxon test. Depicted are the JAK-STAT1/2 (type I and type II interferon) pathway activities. Legend and significance are shown in the figure.

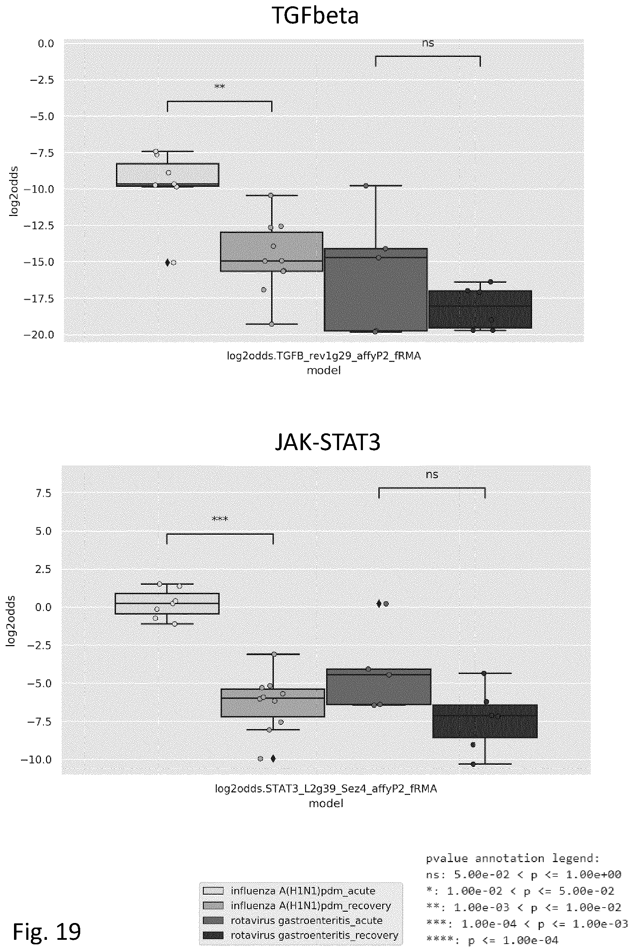

Fig. 19 depicts pathway analysis of dataset GSE50628 of influenza A(H1N1)pdm09 and rotavirus gastroenteritis infected patients. Whole blood samples were collected from patients in the acute phase of the disease and in the recovery phase. Significance was calculated using Mann-Whitney-Wilcoxon test. Depicted are the TGFbeta and the JAK-STAT3 pathway activities. Legend and significance are shown in the figure.

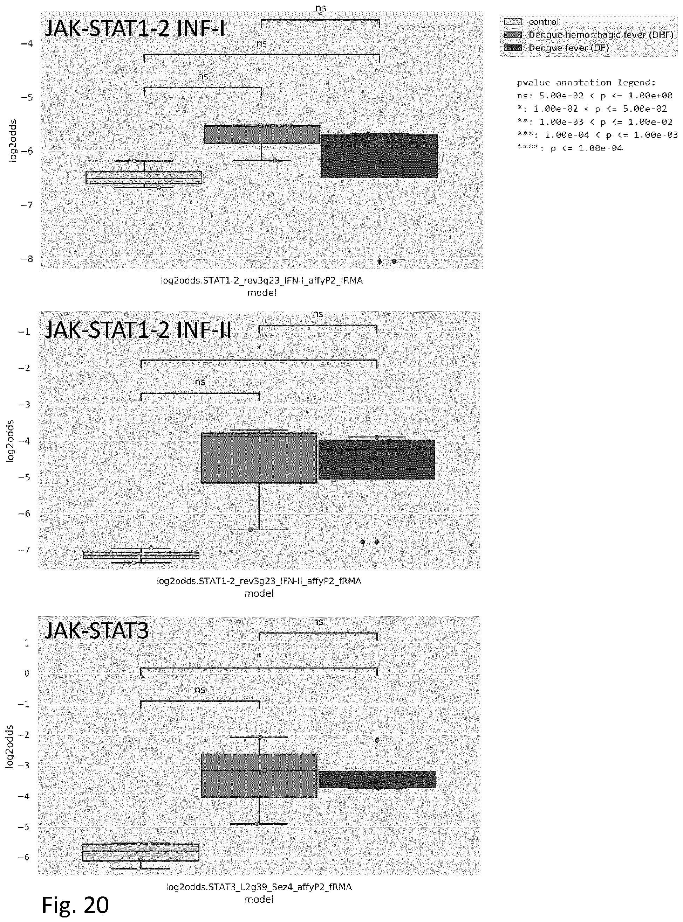

Fig. 20 Pathway analysis of dataset GSE84331, CD8 T Cell Responses in Dengue Virus-Infected Patients from India. The HLA-DR+CD38+ CD8 T cells from the PBMCs of dengue patients from Siriraj Hospital in Bangkok, Thailand and compare with the sorted naive (CCR7+CD45RA+) CD8 T cells from Thai healthy donors. Pathways JAK-STAT1/2 and 3 and Mann-Whitney-Wilcoxon test results are shown.

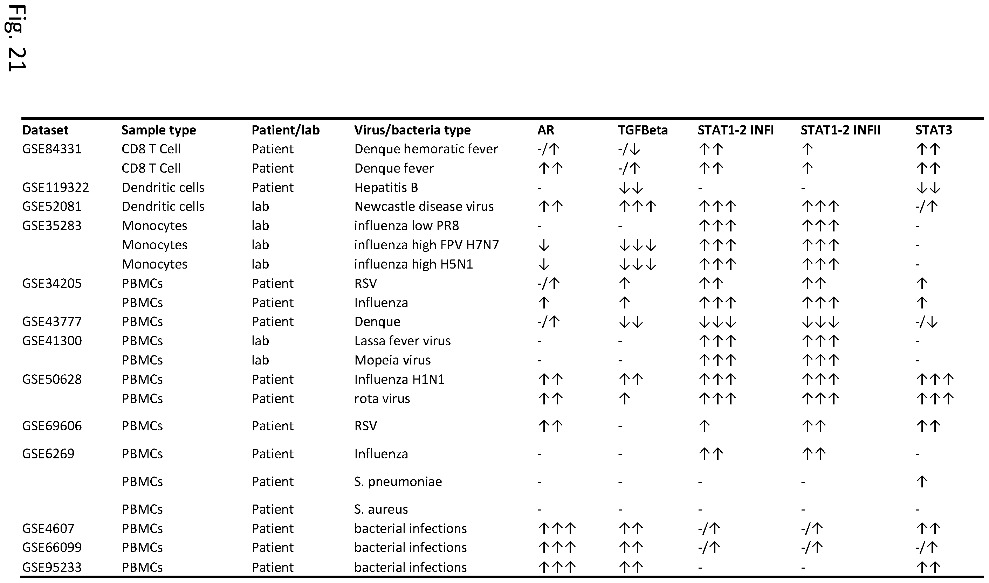

Fig. 21 depicts a summary of the determined pathway activities per dataset and indicating the sample type. Upwards arrows indicate increased pathway activity, downwards arrows indicate reduced pathway activity.

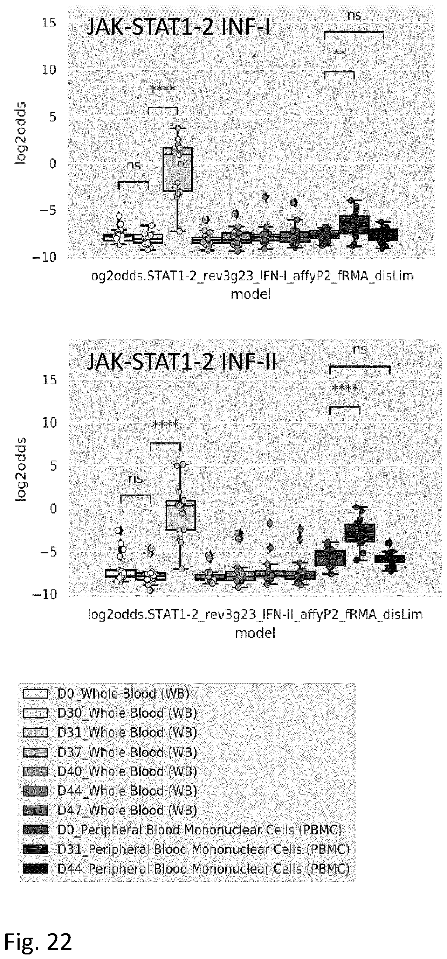

Fig. 22 depicts pathway analysis of tuberculosis vaccine candidate dataset GSE102459. Human subjects were injected with M72/AS01E tuberculosis vaccine candidate at D0 and D30, whole blood samples are collected at D0, D30, D31, D37, D40, D44 and D47 and PBMC samples are collected at D0, D31, D44. Pathway activity scores are shown in log2odds values. Two sided Mann-Whitney-Wilcoxon tests were performed; p-values are indicated in the figures. P-values indicate: *p<0.05; **p<0.01; ***/****p<0.001. Depicted are the JAK-STAT1/2 IFN-I and IFN II pathway activities. Legend is shown in the figure.

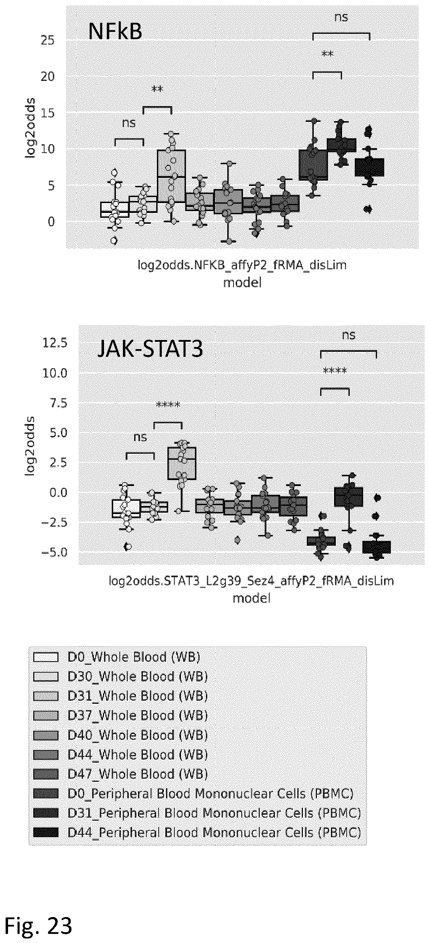

Fig. 23 depicts pathway analysis of tuberculosis vaccine candidate dataset GSE102459. Human subjects were injected with M72/AS01E tuberculosis vaccine candidate at D0 and D30, whole blood samples are collected at D0, D30, D31, D37, D40, D44 and D47 and PBMC samples are collected at D0, D31, D44. Pathway activity scores are shown in log2odds values. Two sided Mann-Whitney-Wilcoxon tests were performed; p-values are indicated in the figures. P-values indicate: *p<0.05; **p<0.01; ***/****p<0.001. Depicted are the NFkB and JAK-STAT3 pathway activities. Legend is shown in the figure.

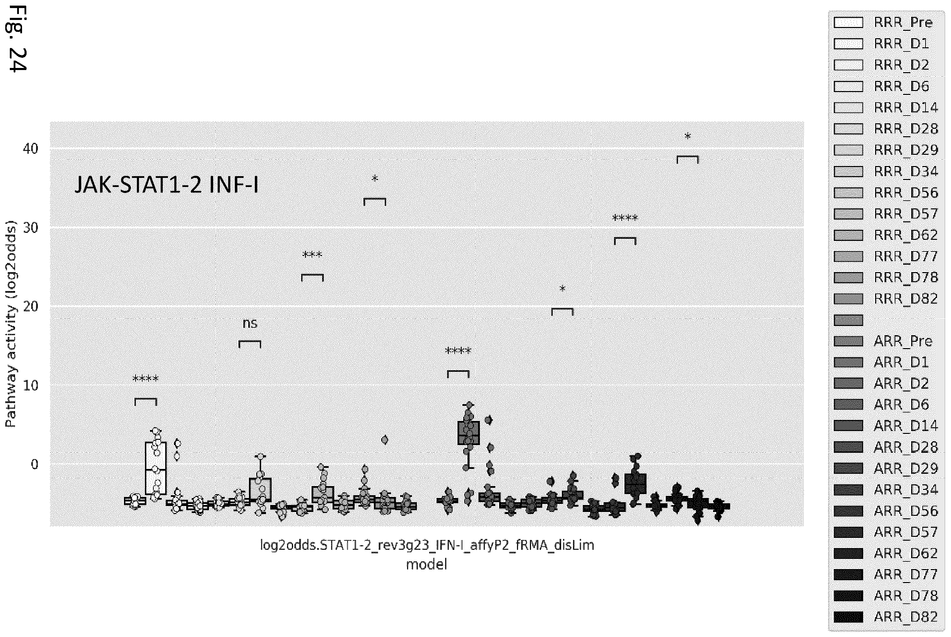

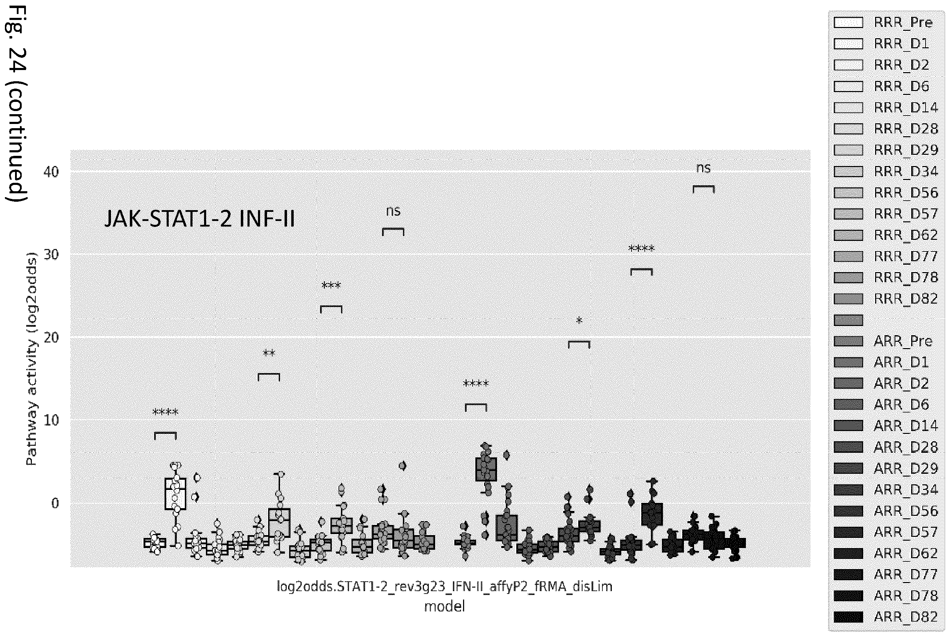

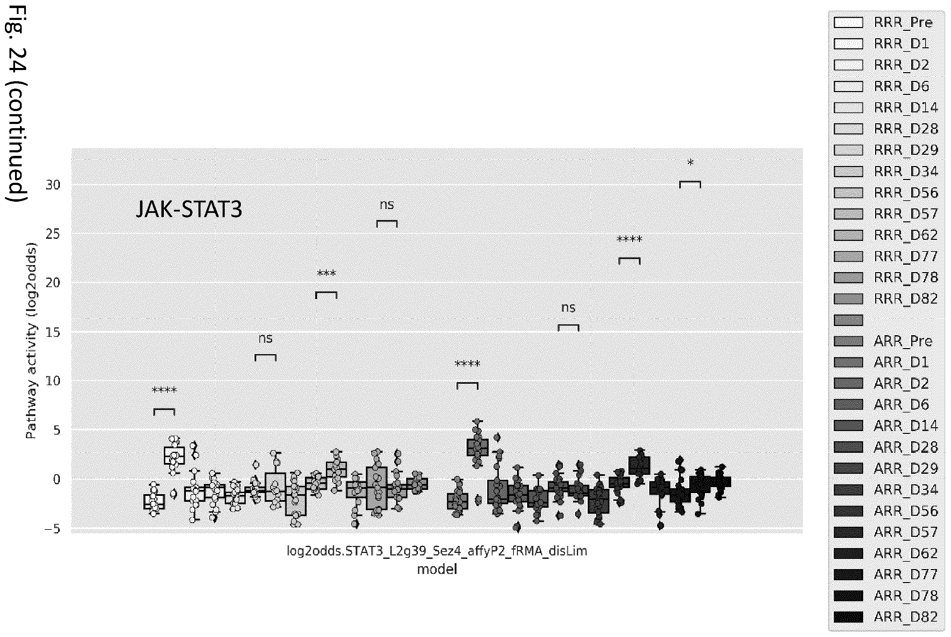

Fig. 24 depicts Pathway analysis of malaria vaccines, dataset GSE89292. Human subjects were vaccinated using ARR of RRR and challenged with Malaria. Participants in both study arms were vaccinated at 28-day intervals, and subjected to controlled malaria infection 21 days following the final immunization. Pathway activity scores are shown in log2odds values. Two sided Mann-Whitney-Wilcoxon tests were performed; p-values are indicated in the figures. P-values indicate: *p<0.05; **p<0.01; ***/****p<0.001. Depicted are the JAK-STAT1/2 IFN-I and IFN II and the JAK-STAT3 pathway activities. Legend is shown in the figure.

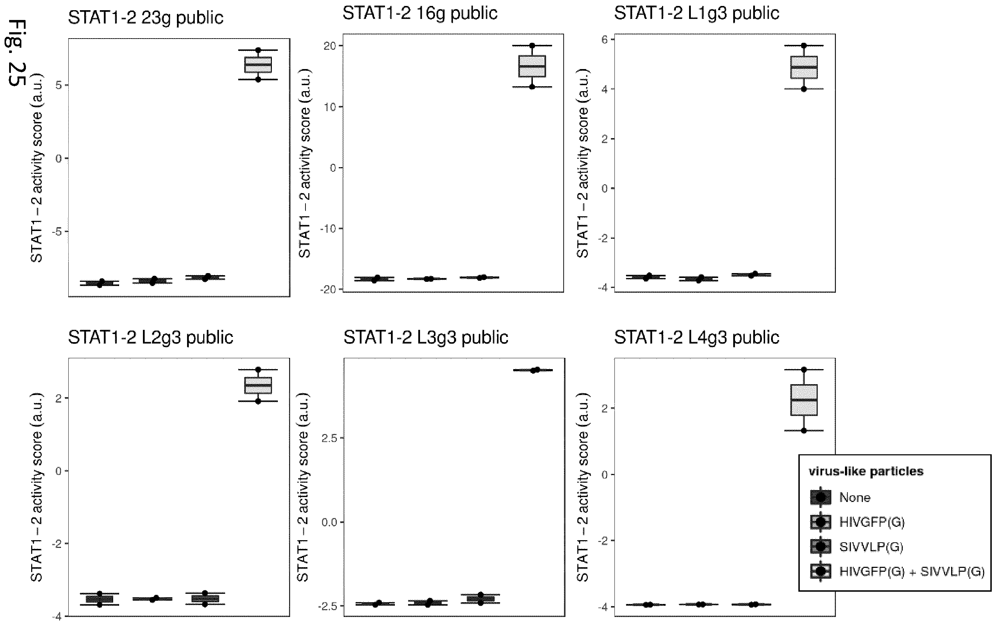

Fig. 25 depicts pathway analysis of dataset GSE22589 containing uninfected human monocyte-derived dendritic cells (MDDCs); MDDCs infected with an envelope-defective GFP-encoding VSV-G-pseudotyped HIV-1 vector (HIVGFP(G)) and with VSV-G pseudotyped virus-like particles derived from SIVmac to deliver Vpx (SIVVLP(G)), alone or in combination. Cells were infected at day 4 of differentiation and harvested 48 hours later. Depicted is the JAK-STAT1/2 pathway activity using the 23 gene (23g), the 16 gene (16g) and 4 randomly selected 3 gene models (l1g3, l2g3, l3g3 and l4g3).

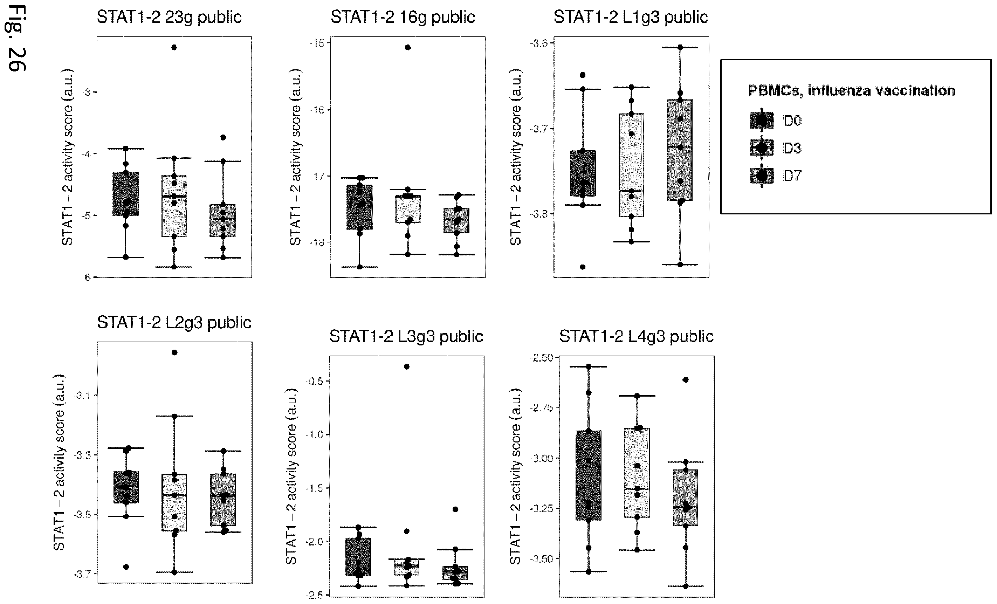

Fig. 26 depicts Pathway analysis of dataset GSE29614. Time Course of Young Adults Vaccinated with Influenza TIV (Trivalent Inactivated Influenza Vaccine) Vaccine during the 2007/08 Flu Season. Blood samples isolated at days 0, 3, 7 days post-vaccination. Depicted is the JAK-STAT1/2 pathway activity using the 23 gene (23g), the 16 gene (16g) and 4 randomly selected 3 gene models (l1g3, l2g3, l3g3 and l4g3).

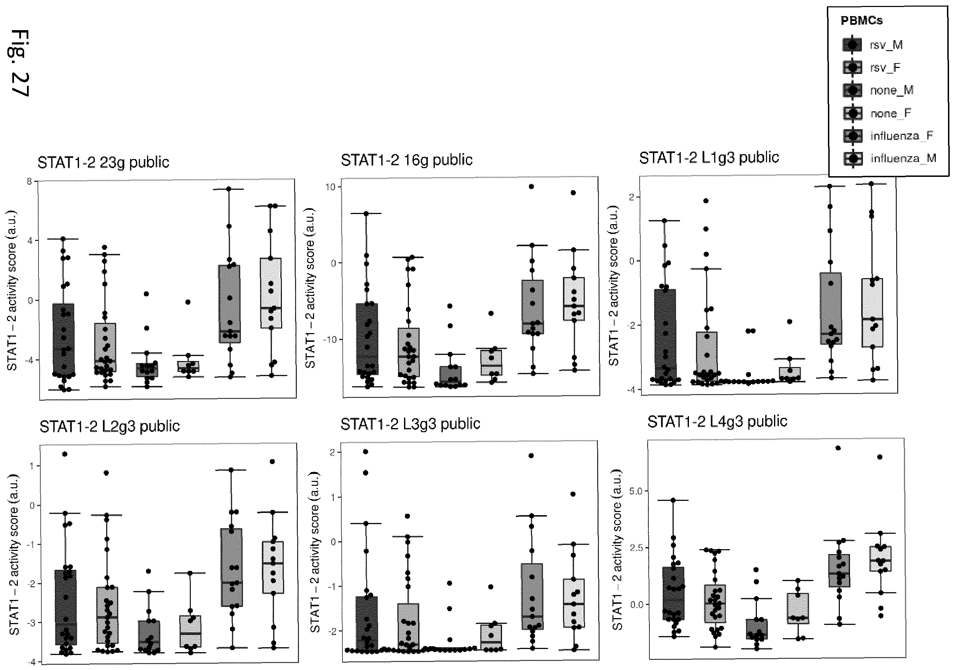

Fig. 27 depicts pathway analysis of dataset GSE34205 including patients hospitalized with acute RSV and influenza virus infection. Blood samples were collected from children within 42-72 hours of hospitalization. The PBMCs were used for the study. Depicted is the JAK-STAT1/2 pathway activity using the 23 gene (23g), the 16 gene (16g) and 4 randomly selected 3 gene models (l1g3, l2g3, l3g3 and l4g3).

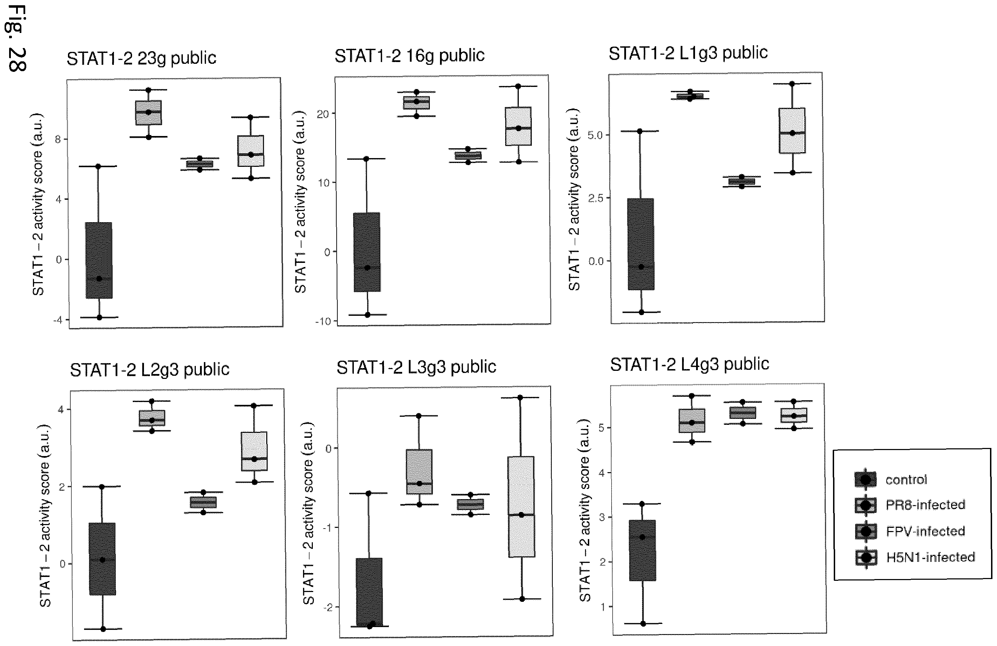

Fig. 28 depicts pathway analysis of dataset GSE35283 containing monocytes infected with low (PR8) and high pathogenic influenza viruses (FPV (H7N7) and H5N1). Depicted is the JAK-STAT1/2 pathway activity using the 23 gene (23g), the 16 gene (16g) and 4 randomly selected 3 gene models (l1g3, l2g3, l3g3 and l4g3).

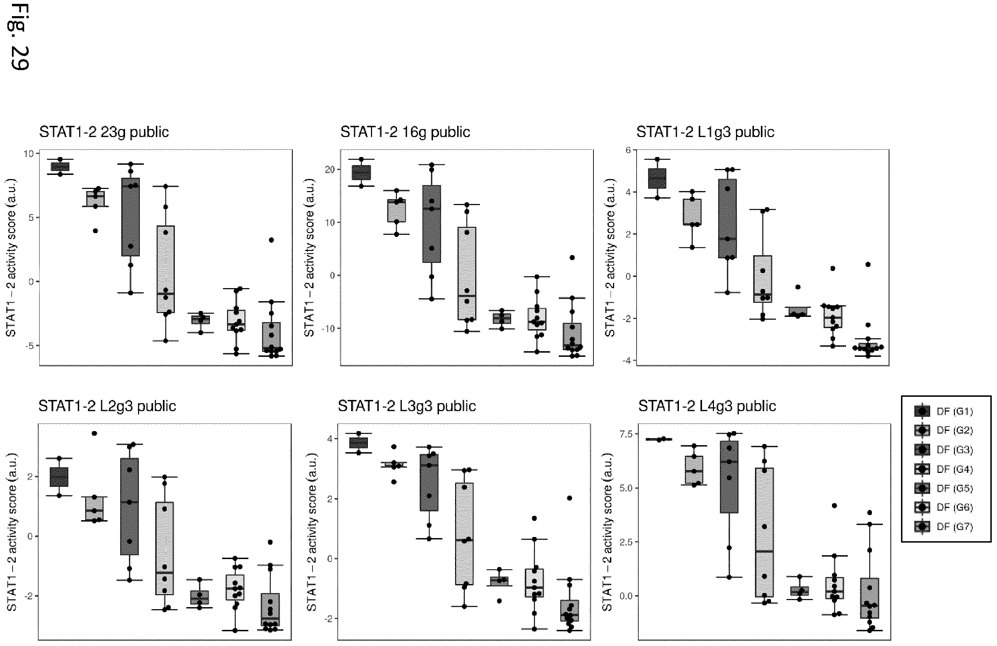

Fig. 29 depicts Pathway analysis of dataset GSE43777, dengue viral infection measured in PBMC samples from DF (dengue fever) and DHF (dengue hemorrhagic fever) with acute infection. Each stage represents a group of patients after x days from fever onset, Stages (days after onset fever): G1: 0 days, G2: 2 days, G3:4, G4:4 days, G5, 5 days, G6: 6-10 days, G7: >20 days. Depicted is the JAK-STAT1/2 pathway activity using the 23 gene (23g), the 16 gene (16g) and 4 randomly selected 3 gene models (l1g3, l2g3, l3g3 and l4g3).

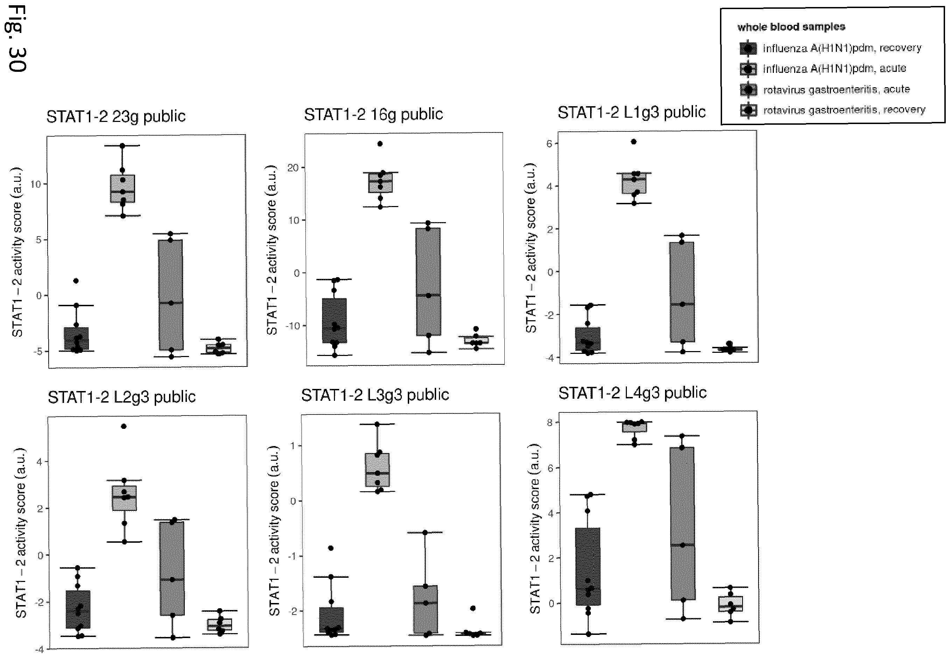

Fig. 30 depicts pathway analysis of dataset GSE50628 of influenza A(H1N1)pdm09 and rotavirus gastroenteritis infected patients. Whole blood samples were collected from patients in the acute phase of the disease and in the recovery phase. Depicted is the JAK-STAT1/2 pathway activity using the 23 gene (23g), the 16 gene (16g) and 4 randomly selected 3 gene models (l1g3, l2g3, l3g3 and l4g3).

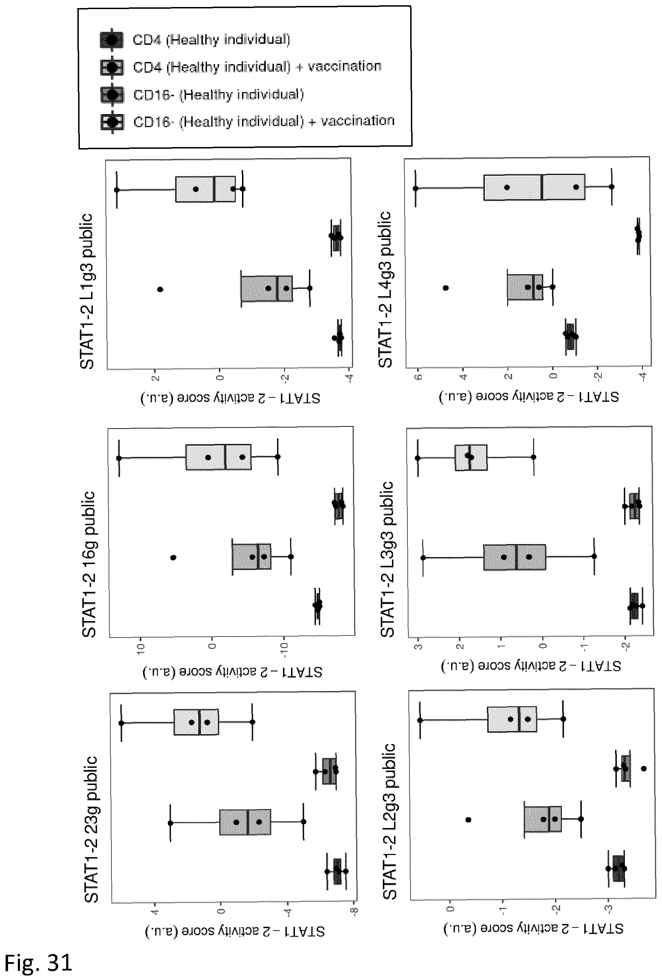

Fig. 31 depicts pathway analysis of dataset GSE51997 comprising healthy and yellow fever (YFV-17D) vaccinated volunteers. CD4 and CD16 negative and positive cells were isolated and used in the study. Depicted is the JAK-STAT1/2 pathway activity using the 23 gene (23g), the 16 gene (16g) and 4 randomly selected 3 gene models (l1g3, l2g3, l3g3 and l4g3).

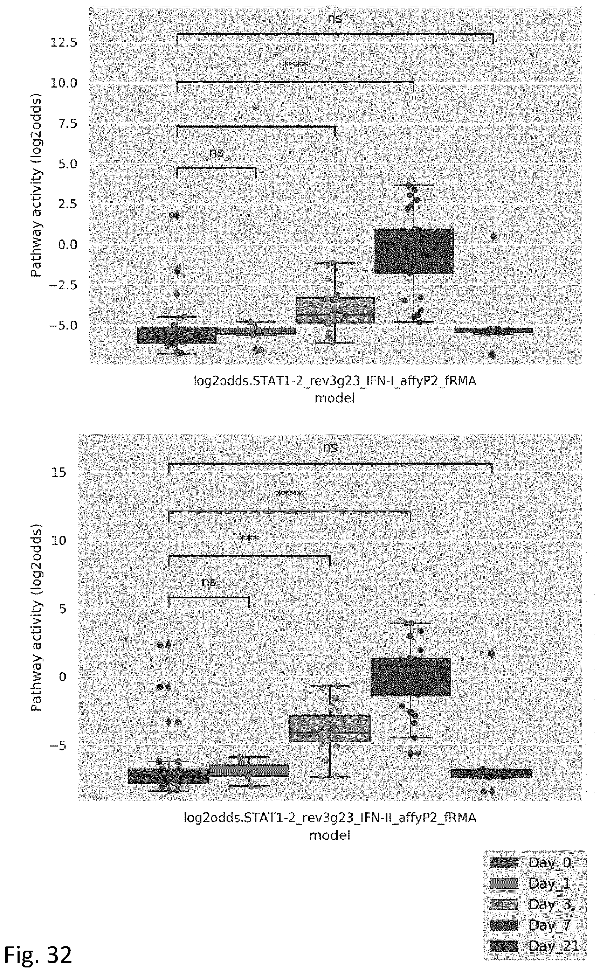

Fig. 32 depicts pathway analysis of dataset GSE13486 comprising healthy yellow fever (YFV-17, live attenuated yellow fever virus strain without adjuvant) vaccinated volunteers. PBMCs were isolated and used in the study. Depicted are the JAK-STAT1/2 (type I and type II interferon) pathway activities. Legend and significance using Mann-Whitney-Wilcoxon test are shown in the figure.

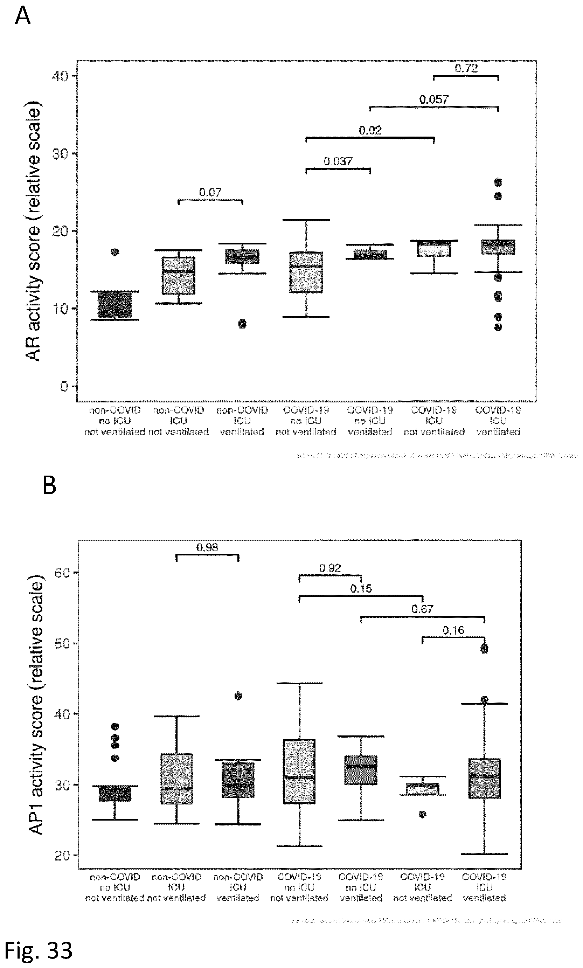

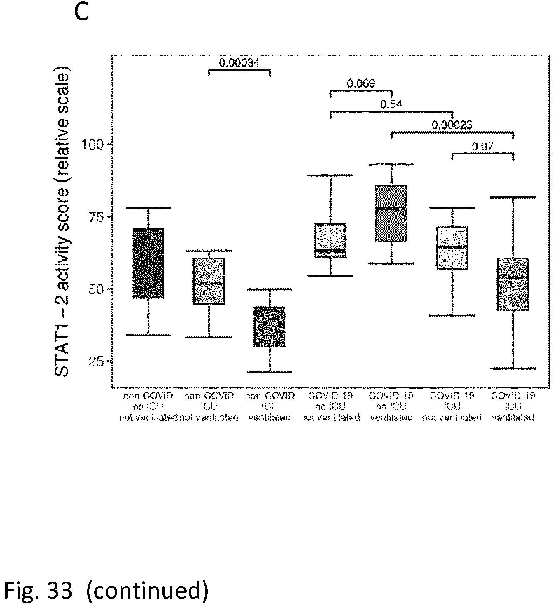

Fig. 33 depicts RNA sequencing pathway analysis of dataset GSE157103 [12] containing COVID-19 and non COVID patients with respiratory issues. ICU and ventilation status is indicated. A) AR pathway activity scores, B) AP1 pathway activity scores and C) STAT1-2 pathway activity scores. Patient samples are shown as duplicate measurements (each sample was measured twice, and shown in the figure).
Fig. 34 depicts pseudotime (disease trajectory) scores (A) and longitudinal patient data categorized based on severity (B). see details [15].

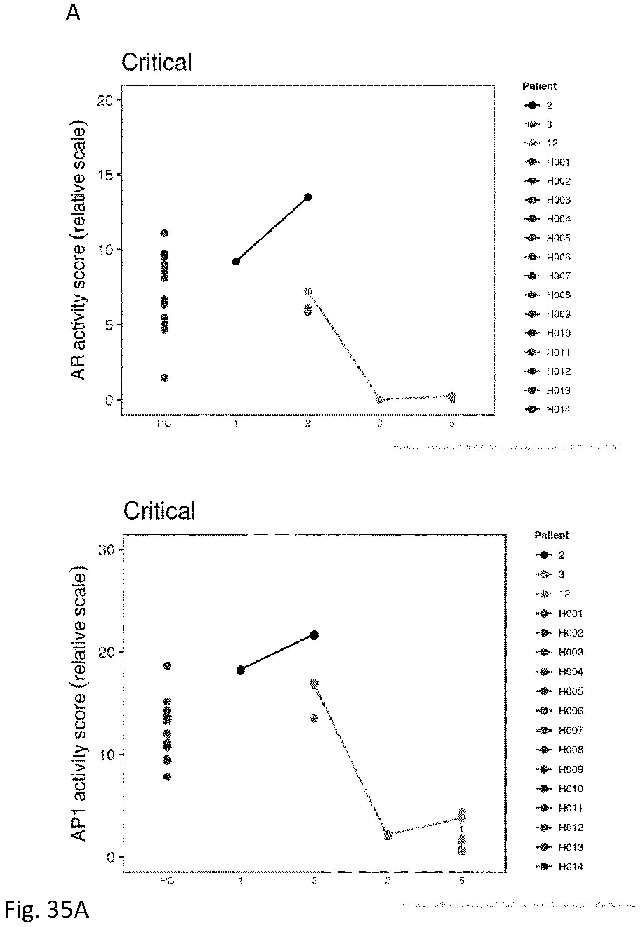

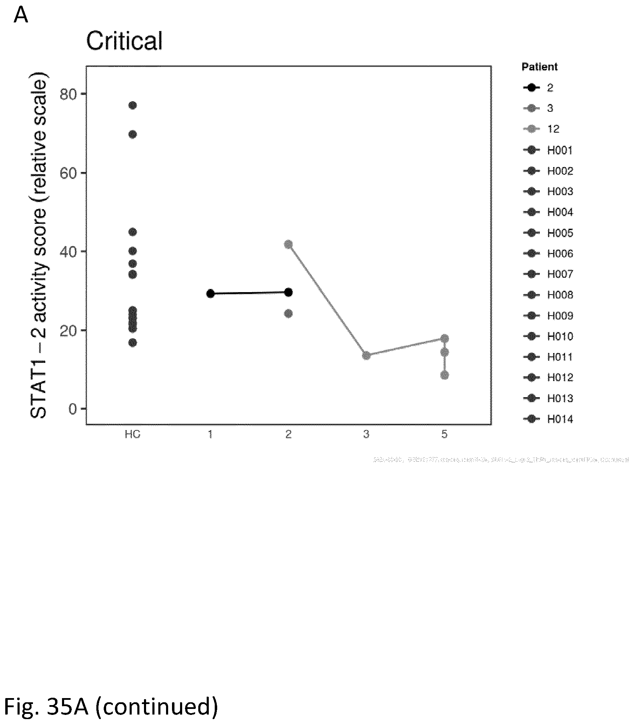

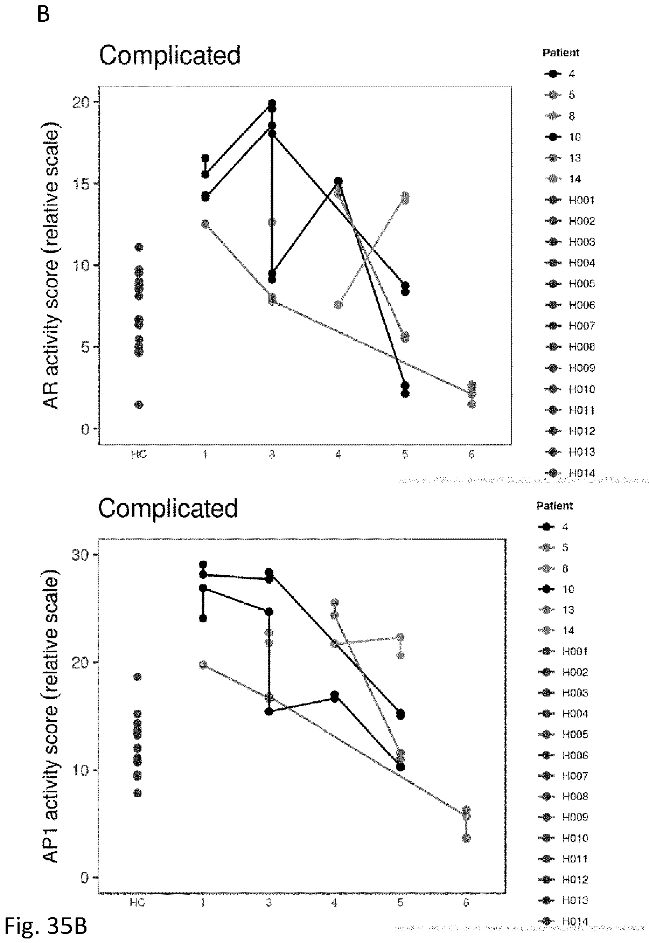

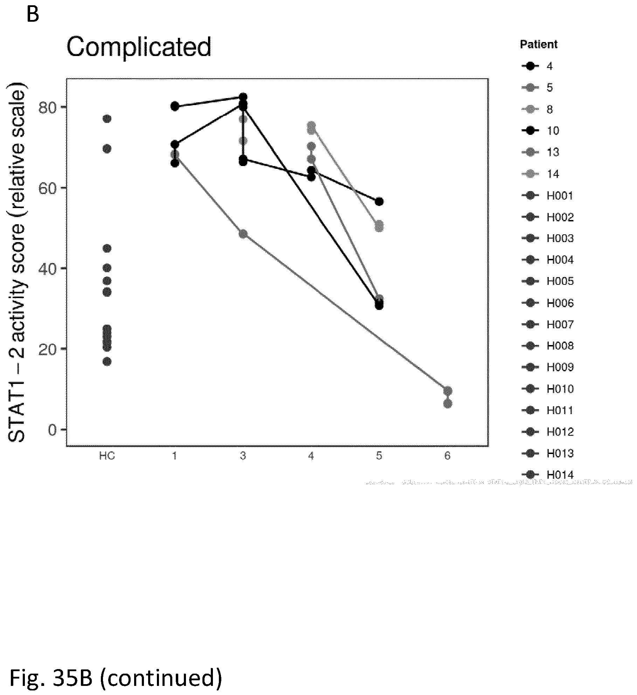

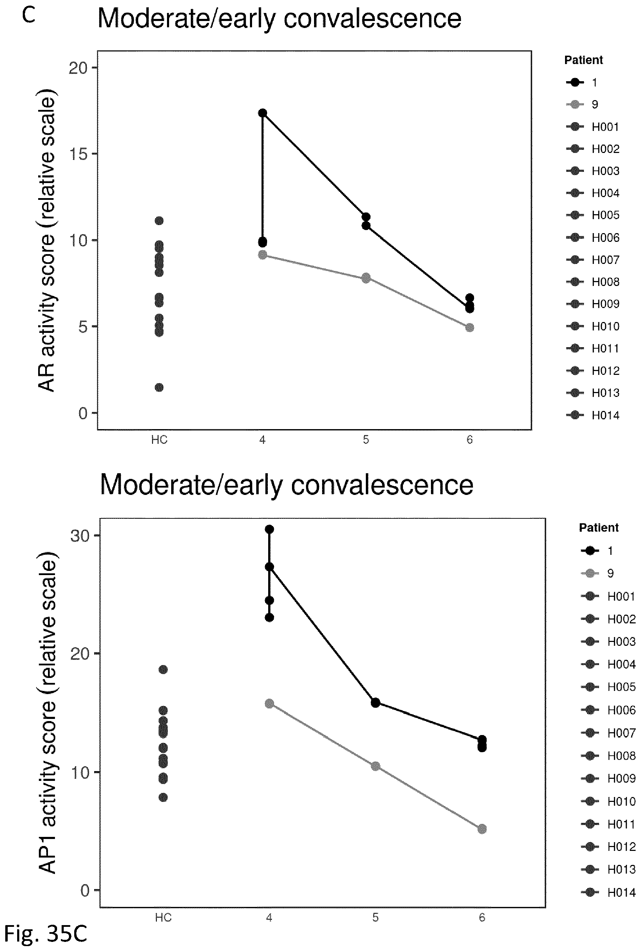

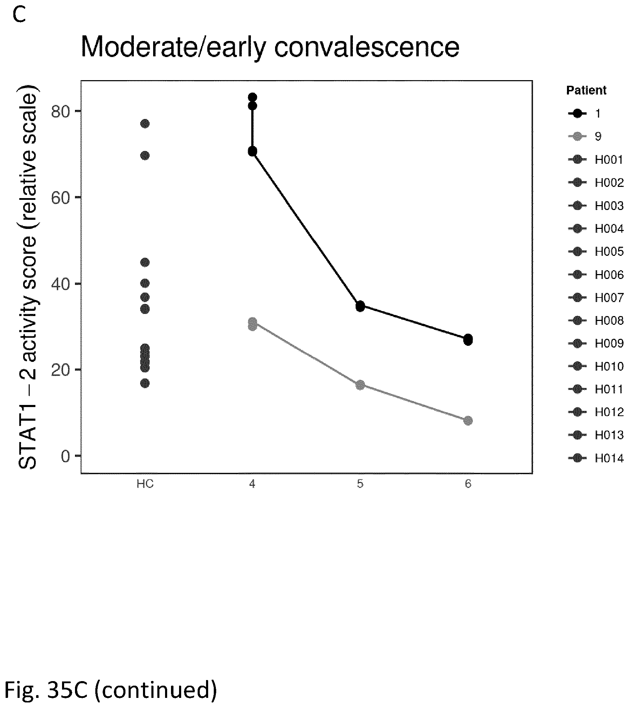

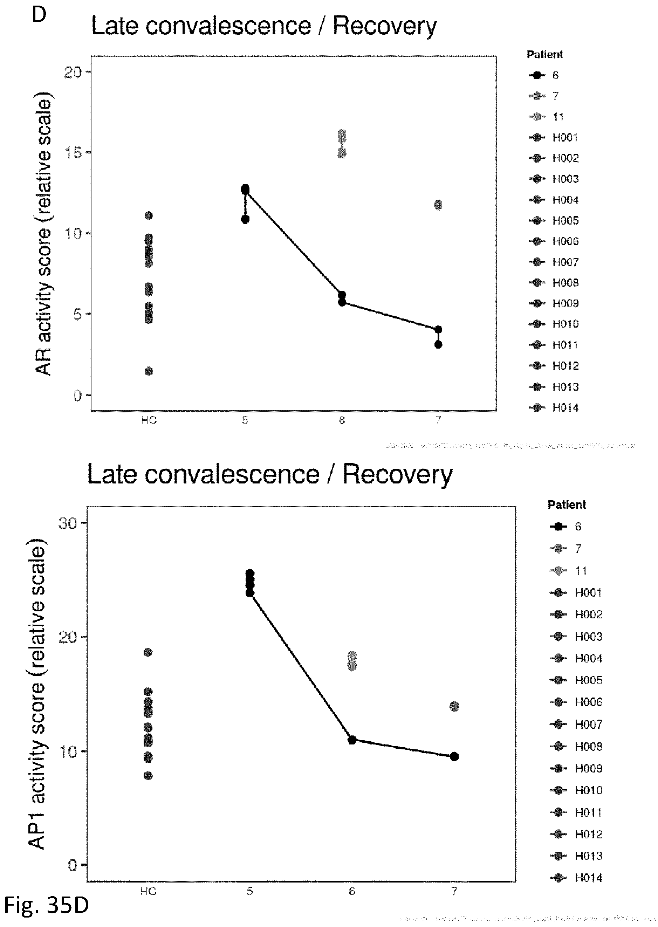

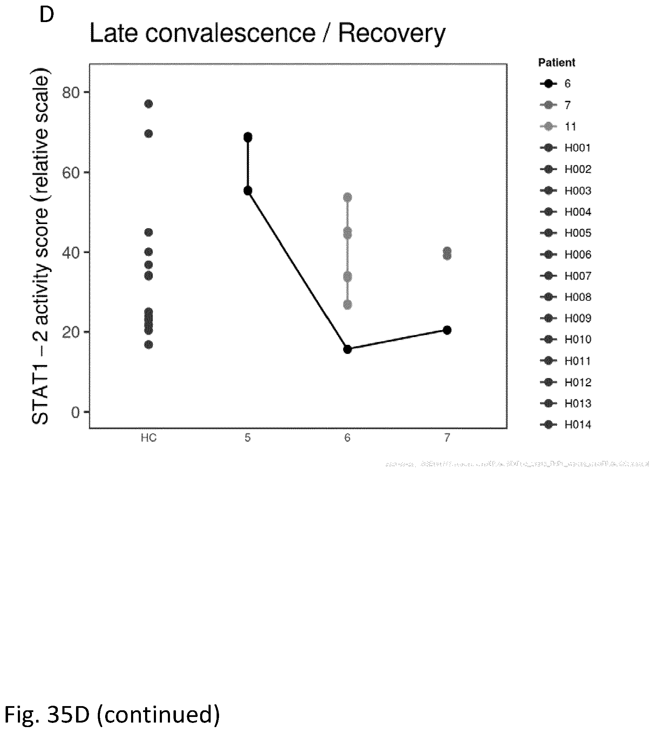

Fig. 35 depicts AR (left), AR (middle) and STAT1-2 (right) RNA sequencing pathway activity scores of patients 1,2,4,10 and HC of dataset GSE161777 [15]. containing COVID patients and Healthy controls (HC). COVID-19 patients are categorized per severity; Critical ill patients (3A), complicated patients (3B), Moderate/early convalescence (3C) and Late convalescence/Recovery (3D).

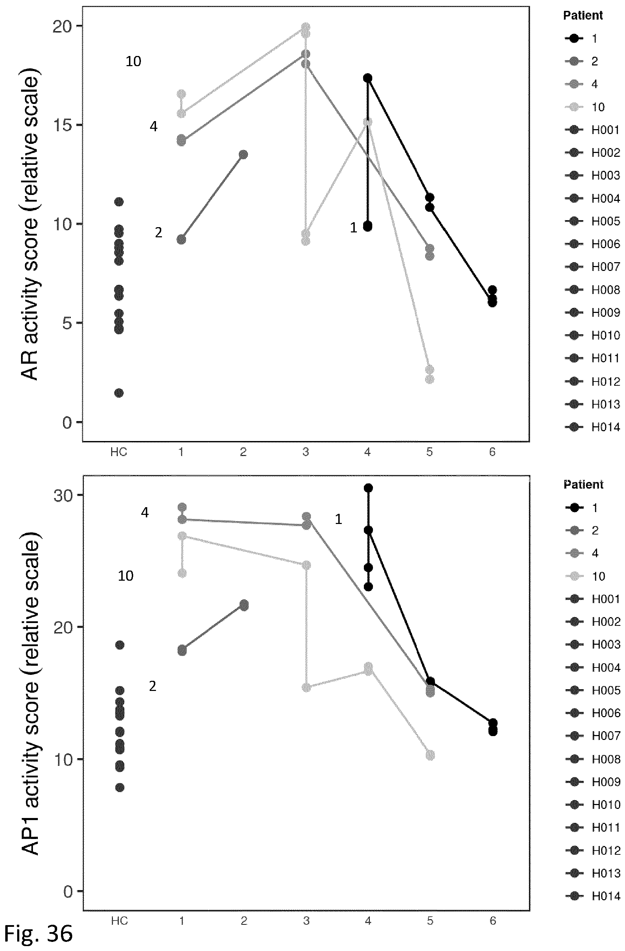

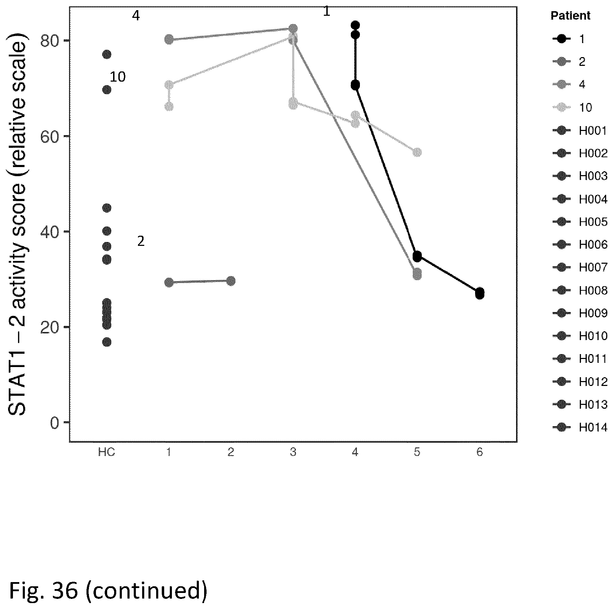

Fig. 36 depicts AR (left), AR (middle) and STAT1-2 (right) RNA sequencing pathway activity scores of patients 1,2,4,10 and HC of dataset GSE161777 [15]. All COVID-19 patients showed disease worsening and improvement, except for patient 2 where disease progression lead to death.

### Examples

### Methods and sample description

Using the Gene Expression Omnibus (GEO) database (https://www.ncbi.nlm.nih.gov/gds/) Affymetrix HG-U133Plus2.0 datasets from clinical and preclinical studies were used. Information about the used datasets, sample type and and preparations can be found in Table 1 below. The literature references related to the original datasets are also included in the table. We used the pathway analysis to determine the signal transduction pathway activities (included pathways; AR, ER, PR, GR, HH, Notch, TGFbeta, WNT, JAK-STAT1/2, JAK-STAT3,NFkB, PI3K, MAPK). We compared the different pathway activities within the groups per dataset. Results per dataset can be found in the figure description.

The pathway analysis method to measure the cellular immune response was used to analyze Affymetrix U133P2.0 expression microarray data from a number of clinical studies of patients infected with a virus, or vaccinated with a specific vaccine, and preclinical studies on vaccine development. In the pathway analysis method, a calibrated mathematical method was used for each pathway to relate the expression levels of target genes for each pathway to the pathway activity. The pathway activity is represented by a numerical value. In all viral infections that we studied to date in which patients recovered, increased activity of the JAK-STAT1/2 pathway was observed, in combination with changes in a number of other immune-modulating signaling pathways (JAK-STAT3, TGFbeta, NFkB and AR pathway) which were informative for the raised cellular immune response, where the increases in JAK-STAT3 pathway activity was especially informative on the clinical course of the infection (severity).

### Distinction between viral and bacterial disease (Figs. 2-4)

Pathway analysis from a number of clinical viral studies shows that we can distinguish on an individual patient basis between viral and bacterial infection (Figs. 2-4). For example the JAK-STAT1/2 pathway activity was specifically increased in viral infections, not in bacterial infections. Therefore, the JAK-STAT1/2 pathway activity (type I and type II interferon) is and indicator of viral infection and not for bacterial infection in which among other AR and TGFbeta is of importance.

Both AR and TGFbeta pathways are increased in sepsis shock survivors and non survivors patients compared to healthy controls. JAK-STAT1-2 INFI and INFII pathway activity is only increased in a selection of patients. The patients with the highest expression of JAK-STAT1-2 patients (n=5) had an lung infection. From community-acquired pneumonia its known that 70% of hospitalized CAP patients initially have sepsis or may develop sepsis during their hospital stay. It is likely these sepsis patients with an high JAK-STAT1-2 activity have an co infection of bacterial and viral infection.

### Distinction between mild and severe infection (figs. 5-8)

The pathway analysis showed that both the JAK-STAT1-2 INF 1 and II were increased in RSV infected patients compared to the control. JAK-STAT3 activity was progressively increased with the severity of the disease. Significance using Mann-Whitney-Wilcoxon test and legend are shown in the figure. We could distinguish between mild and severe infection. In pediatric patients infected with RSV (respiratory syncytial virus, which shows some clinical similarities to COVID-19) we could distinguish between mild and severe respiratory infections based on PBMC blood cell analysis (Figs. 5 and 6). In Dengue viral infection similarly JAK-STAT3 pathway activity was higher in the more serious hemorrhagic fever variant (Figs. 7 and 8). Both groups show a decrease in pathway activity of JAK-STAT1-2 INF I and INF II after day 4 of fever onset, which reflects the known immunosuppressive effect of the virus on the adaptive cellular immune response. There was a Higher JAK-STAT3 pathway activity in the G2 DHF group compared to the G2 DF group.

### Strength of the cellular immune response in an individual and in a population (figs. 9-11)

We could measure on patient PBMCs that the immune response to influenza virus infection was stronger than to RSV virus, in line with literature describing that in general in the population the immune response generated by the RSV virus is lower and less persistent (Figs. 9 and 10). The Influenza infected patients induces a higher JAK-STAT1-2 INF I and II pathway activity compared to RSV infected patients, which is in agreement with the authors. Furthermore, JAK/STAT3 and TGFbeta pathway activity slightly increased in influenza, not in RSV. https://www.ncbi.nlm.nih.gov/pmc/articles/PMC3347264/

In PBMCs of chronic hepatitis patients the cellular immune response was minimal or absent, in line with the lack of cellular immune response in this chronic persistent infection (Fig. 11). Hepatitis B infection was associated with reduced JAK-STAT3 activity in DCs.

Similarly, The Dengue virus is known to cause suppression of the cellular immune response, which is indeed measured in the clinical study on Dengue viral infection, where patients have been monitored over the course of the disease (Day1-6, and day 20 =recovery): in contrast to what we observed in influenza, RSV, rota, and yellow fever virus infections, here the JAK_STAT1/2 pathway peaks at the beginning of the disease (Day 1), and subsequently goes down instead of up (Figs. 7 and 8).
In general within a population with a certain type of viral infection a large range in the strength of the cellular immune response was observed, in line with common knowledge that the cellular immunity is determined by many factors that are variably present in the population, such as comorbidities, lifestyle factors, use of drugs, old age (all figures).

### Measuring the effectiveness of vaccination in humans (Figs. 12-14)

In clinical vaccination studies we could distinguish between effective and ineffective vaccines (Figs. 12-14). The significant pathways JAK-STAT1/2/3, TGFBeta and NFKB using T-test are included in the figure. The yellow fever vaccination induces JAK-STAT1-2 type I and II IFN pathway activity in all cell types, which is in agreement with the authors of the paper, also the JAK-STAT3 pathway activity was increased in the vaccinated individuals compared to the control. NFKB was only increased in the inflammatory monocytes (CD16-) and TGFBeta only in non-inflammatory monocytes (CD16+). Yellow fever vaccine in general appeared to be a highly effective vaccination, inducing a strong cellular immune response (Figs. 12 and 13). However, although reported to produce a humoral immune response, a clinically tested influenza vaccine appeared to not induce a cellular immune response (compare Fig. 14 with figs. 9 and 10). This is in line with the general observation that immunity generated by influenza vaccination is not as protective against influenza infection as having recovered from an influenza infection.

There is no change in JAK-STAT1/2 and JAK-STAT3 pathway activity upon vaccination. However, the authors measured an humoral protection measured (Fig. 14). This humoral activity was not measured using the pathway analysis which only measured the cellular immune response. A combined cellular and humoral immune response provides better protection against influenza than only an humoral immune response. https://www.ncbi.nlm.nih.gov/pmc/articles/PMC3140559, https://www.ncbi.nlm.nih.gov/pmc/articles/PMC6605831/

### Measuring the effectiveness of vaccination in experimental setting for vaccine development (Figs. 15-17)

Vaccine development with initial effectiveness/efficiency testing requires testing in in vitro cell model systems. For example by introduction of the vaccine in monocytes, macrophages or dendritic cells to investigate how they stimulate antigen processing and presentation within HLA on the cell membrane. Using pathway analysis of sample data from vaccine-infected cells, we show that the effectiveness of inducing antigen presentation can be quantitatively measured, in accordance with the reported behavior of the vaccine. The JAK-STAT signaling pathway was clearly the indicative signaling pathway, in line with its prominent immune role in antigen presentation.

Two non-efficient HIV vaccines did not elicit an increase in activity of the JAK-STAT1/2 pathway when introduced into dendritic cells, while the combination was successful (Figs. 15 and 16). Only a combination of combined HIVGFP(G)) and SIVVLP(G) infection resulted in an JAK-STAT1-2 response. Which is in agreement with author's in which MDDC infected with both HIVGFP(G) and SIVVLP(G) stimulated a high proportion of MHC class I and class II restricted T cell clones to produce IFNγ. Other pathways which were only significant different using the combination are the TGFBeta and NFKB pathway.

For vaccine development it is also important to compare effects of low pathogeneic (potentially to be used for vaccination purposes) and high pathogenic virus variants. In monocytes infected with low (PR8) and high pathogenic influenza viruses (FPV (H7N7) and H5N1), activity of the JAK-STAT1/2, JAK-STAT3 and TGFbeta pathways was distinctly lower when cells were infected by the virus, suggesting that partial suppression of the innate immune response may be part of the pathogenic mechanism used by the high pathogenic virus variants (FPV (H7N7), to evade the action of the immune response (Fig. 17).

### Cellular immune response can be measured on different immune cell types (Figs. 18-20)

The method can be used to measure the cellular immune response on a variety of relevant immune cells or mixtures of immune cells as are frequently derived from blood samples, such as PBMCs. Cellular immune response was measured in whole blood of rotavirus infected patients, and activity of the JAK-STAT1/2 pathway was indicative of a cellular immune response (Figs. 18 and 19). In both influenza patients and rotavirus patients the JAK-STAT1-2 INF 1 and INFII was higher during acute infection. JAK-STAT3 was also higher in the acute influenza infection but not the rotavirus.

CD8+ cells were used and informative in case of Dengue virus (fig. 20), as well. CD4+ T cells and monocytes (CD16- inflammatory and CD16+ non-inflammatory) were used and informative in Yellow fever (see Figs. 15 and 16). No high JAK-STAT1-2 pathway activity was measured, which is in agreement with the literature were was demonstrated that CD8 T cells from dengue patients become cytokine unresponsive due to TCR signaling insufficiencies. The measured JAK-STAT1/2 pathway activity was interferon type II dominant over type I.

### Tuberculosis vaccine (figs. 22 and 23)

Subjects were injected with M72/AS01E tuberculosis vaccine candidate at D0 and D30, whole blood samples were collected at D0, D30, D31, D37, D40, D44 and D47 and PBMC samples were collected at D0, D31, D44.

The tuberculosis vaccine consists of the adjuvant AS01E, containing MPL-A and immunostimulatory saponin QS-21, which induces an immune response characterized by the activation of interferon-gamma producing CD4+ T cells, and the production of antibodies.

The tuberculosis vaccination induces JAK-STAT1-2 type I and II IFN pathway activity after day 1 after vaccination in both blood sample types, which is in agreement with the authors of the paper , also the JAK-STAT3 and NFKB pathway activity was increased after day one of vaccination (see Figures 22 and 23).

### Malaria vacination and challenge - (fig. 24)

Healthy malaria-naive volunteers, randomized to two study arms participated in this study testing the efficacy of RTS,S and AdVac^{®} malaria vaccine candidates. Study arm 1 (hereafter referred to as ARR), comprised of volunteers who received the AdVac^{®} vaccine composed of Ad35 vector expressing full length CSP, as a primary immunization, followed by two doses of RTS,S/AS01E vaccine. The subjects in the second arm, received three doses of RTS,S/AS01 (RRR regimen). RTS,S/AS01E is a recombinant protein-based malaria vaccine.

The RTS,S vaccine was engineered using genes from the repeat and T-cell epitope in the pre-erythrocytic circumsporozoite protein (CSP) of the Plasmodium falciparum malaria parasite and a viral envelope protein of the hepatitis B virus (HBsAg), to which was added a chemical adjuvant (AS01E) to increase the immune system response.
Participants in both study arms were vaccinated at 28-day intervals, and subjected to controlled malaria infection 21 days following the final immunization.

Both the RRR and ARR vaccination groups showed induced JAK-STAT1-2 type I and II IFN pathway activity after one day of vaccination, also the JAK-STAT3 pathway activity was increased after vaccinations (see Figure 24). The ARR vaccine also resulted in significant increase in STAT1-2 after the two follow-up doses of RTS,S/AS01E.

The tuberculosis vaccine described above (Figures 22 and 23) is a bacterial vaccine with an adjuvant (AS01E; GSK). In general, no upregulation of the JAK-STAT1/2 cellular signaling pathway activity is observed from bacterial infections, as for example demonstrated by data obtained from sepsis patients. The upregulation of the JAK-STAT1/2 cellular signaling pathway activity obeserved in the dataset for the tuberculosis vaccine can be attributed to the adjuvant. The same adjuvant is used in the malaria vaccine described above (data relating to Figure 24).

Therefore, it is envisioned that the immune repsonse induced by a vaccine or vaccine efficacy can be determined based on the JAK-STAT1/2 cellular signaling pathway activity, if the vaccine comprises an adjuvant, in particular if the adjuvant is AS01, more in particular AS01E. It is envisioned that this response is independent of the vaccine type and thus the immune repsonse induced by a vaccine or vaccine efficacy can be determined based on the JAK-STAT1/2 cellular signaling pathway activity for viral vaccines, bacterial vaccines and live attenuated vaccines.

### Identification new JAK-STAT1/2 target genes.

Additional target genes were identified for the JAK-STAT1/2 cellular signaling pathway based on literature review and analysis on ground state truth datasets. Potential target genes were validated. The following genes were identified as JAK-STAT1/2 target genes which have been validated using the cellular signaling Pthway activity models described herein:
GBP1, TAP1, ISG15, APOL1, IFI6, IFIRM1, CXCL9, APOL2, IFIT2 and LY6E

### Validation of 23, 16 and 3 gene models (Figs. 25 - 31).

In order to validate the set of genes including the newly identified target genes for the JAK-STAT1/2 cellular signaling pathway, a selection of the datasets were analyzed again with the new 16 gene model, and put side by side with the old 23 gene model. It was found that the new 16 gene model performed at least equally. The data is indicated with 23g and 16g respectively.

To further support that also less genes can be used in the pathway models, random selection random selections of three genes were made as follows:
L1G3: USP18, APOL1, OAS1
L2G3: IRF1, TAP1, ISG15
L3G3: STAT1, IRF9, IFI6
L4G3: IRF7, GBP1, IFITM1

The data for datasets GSE22589 (Fig. 25), GSE29614 (Fig. 26), GSE34205 (Fig. 27), GSE35283 (Fig. 28), GSE43777 (Fig. 29), GSE50628 (Fig. 30) and GSE51997 (Fig. 31) was analyzed and compared. Similar trends were observed when using the randomly selected 3 gene models, although as expected the separation is less pronounced as compared to the 23 or 16 gene models.

### Live attenuated Yellow Fever vaccine (Fig. 32)

The immune response can be followed over time. Healthy individuals were adminstered Yellow Fever vaccine (YFV-17, live attenuated yellow fever virus strain without adjuvant). In PBMCs the increase in JAK-STAT1/2 actvity is seen to to increase at day 3 and peak at day 7, to return to baseline levels at day 21 after adminstration of the vaccine.

### COVID-19 (Figs. 33-35)

### RESULTS

***All results are based on pathway analysis of RNAseq data derived from whole blood samples.***

### 1. Measuring severity of COVID-19 (Fig. 33)

### Dataset GSE157103

Dataset GSE157103 contains data from samples of COVID-19 patients and non-COVID-19 patients. Whole blood was collected from patients and RNA was isolated using LeukoLOCK Total RNA Isolation System[1]. RNA seq was performed.

Samples were annotated with respect to diagnosis ( COVID-19 or non COVID-19) and severity (*moderate to severe respiratory issues*), ICU admission and mechanical ventilated status, for details see Overmyer *et al.*

For data analysis RNA sequencing-adapted signal transduction pathway analysis models for the AR, STAT1-2 and AP1 pathways were used, results shown in Figure 33.

For the AR pathway (Figure 33A), higher severity of disease was associated with higher AR pathway activity. This increase in AR pathway activity score was observed for both non-COVID patients who had other respiratory diseases and for COVID-19 patients, in which the mechanically ventilated patient group at the ICU had the highest AR pathway activity. The lowest AR pathway activity was found in the least severe group of non COVID patients, no ICU, and no mechanical ventilation.

For STAT1-2 pathway activity (Figure 33C), lower pathway activities were associated with more severe disease, that is, lowest in ventilated patients at the ICU, intermediate in non-ventilated patients at the ICU, and highest in ventilated and non-ventilated patients at the general ward (not needing admission to the ICU). The lowest JAK-STAT1-2 pathway activity was found in both COVID and non-COVID patients who were mechanically ventilated at the ICU. Low JAK-STAT1/2 pathway activity in a virally infected patient indicates failure of the adaptive immune response to cope with the viral infection (Bouwman et al, Frontiers) [13]. It is known that COVID-19 patients may have impaired JAK-STAT1-2 pathway activity, and that very severe cases have acute respiratory distress syndrome (ARDS), necessitating admission at the ICU [14, p. 19]. From our pathway analysis it is clear that the patients without an impaired JAK-STAT1-2 pathway, resulting in high JAK-STAT1-2 pathway activity during COVID-19, could be treated outside the ICU with or without ventilation.

MAPK-AP1 pathway activity (Figure 33B), showed no large differences between patients groups, but showed large spread in pathway activity scores within groups. This large spread for this signaling pathway could be due to the variety of comorbidities in patients with COVID-19, or different treatments that were administered and may have specifically affected activity of this pathway.

### Conclusion from analysis of dataset GSE157103:

COVID-19 patients admitted to the ICU have more severe disease than those that can remain in a general ward; also at the ICU the patients who need ventilation generally have more severe disease than the patients who do not receive artificial ventilation. The results show that the level of JAK-STAT1/2 pathway activity can distinguish between patients on a general ward and patients at the ICU, and between ICU patients on and off artificial ventilation. The JAK-STAT1/2 pathway is a crucial pathway of which activity is needed to mount an adaptive immune response to a viral infection. The lower the activity (pathway activity score) of this pathway, the higher the severity of the disease. Therefore, these results show that the JAK-STAT1/2 pathway assay can be used to measure severity of COVID-19 in whole blood samples.

Based on earlier analysis results in patients with other viral infections, it is expected that measurement results of the activity of the JAK-STAT3 pathway will indicate the severity of the inflammatory condition in the acutely ill COVID-19 patients, mediated predominantly by the innate immune response, and therefore is expected to be inversely related to the activity of the JAK-STAT1/2 pathway.

### Dataset GSE161777 (Fig. 34)

Dataset GSE161777 contains longitudinal data from 13 patients which were sampled at days 0, 2, 7, 10, 13 and/or at discharge. Whole blood samples were obtained, RNA isolated and RNA seq performed. One patient with mild disease was enrolled after recovery as a recovery control. 14 Healthy donors sampled at a single time point were included as controls. RNA was extracted from peripheral blood sampled at up to 5 time points per patient. At each sample point, a patient's disease trajectory, termed "pseudotime", was categorized according to clinical parameters. To describe the heterogenous disease trajectories over time, a modified WHO ordinal scale (WHO, 2020) was used, which also takes into account several inflammatory markers (serum c-reactive protein [CRP], serum IL-6, and ferritin)[15]. The score (see figure 34A) was used to classify patients along their disease course and is proposed for use to monitor the disease (figure 34B). Pathway scores for all patients can be found in Table 2.

Comparing the various disease severity categories as defined in pseudotime (1-7 in figure 34) with respect to pathway activity, it is clear that (1) in the critically ill patients JAK-STAT 1/2 pathway activity is low (below 50, in the range of healthy controls) compared to pathway activity in patients with complicated or moderately severe disease (pathway activity score above 60, many around 80), and (2) during convalescence the corona virus-induced increased JAK-STAT1/2 pathway activity (above 60) (indicating an appropriate adaptive immune response to the virus) returns to the normal healthy level (below 50).

### Conclusion from Dataset GSE161777

These results confirm that measurement of JAK-STAT1/2 pathway activity can be used to assess disease severity, where in the acute phase of the disease low JAK-STAT1/2 pathway activity means very severe disease with failure to mount a good adaptive immune response, while high JAK-STAT1/2 pathway activity indicates a good adaptive immune response. If the JAK-STAT 1/2 pathway activity was initially high and starts to decrease, this indicates that the patient is entering a reconvalescence phase.

### 2. Predicting prognosis in COVID-19 patients using JAK-STAT1/2 pathway activity Dataset GSE161777 (Figure 35).

**As** can be observed on the individual disease trajectories over time (pseudotime categories on the X-axis) (Figure 35), repeated JAK-STAT1/2 pathway activity measurement can be used to predict prognosis in a patient with COVID 19.

When measuring JAK-STAT 1/2 pathway activity *at least twice sequentially* in a patient with covid-19, addition of the pathway activity score to conventional clinical parameters of the patient (here defined as pseudotime parameters 1-7) is expected to improve prediction of prognosis of the patient. For example, if in an clinically acute disease phase JAK-STAT1/2 pathway activity decreases over a few days, this is likely to predict a bad prognosis; in contrast, if the JAK-STAT1/2 pathway activity shows an increase over that time period, this indicates a good adaptive immune response, with expected good prognosis. If two JAK-STAT1/2 measurements are performed while the patient is showing improvement according to conventional clinical parameters, an increase in pathway activity score indicates that the patient is still in the acute phase but mounts an adequate adaptive immune response with a good prognosis; while a decrease in pathway activity score indicates that that patient enters the reconvalescence phase with a good prognosis.

With respect to activity scores for AR and MAPK pathways, no difference was observed between critically ill and complicated/moderately ill patients, however pathway activities were increased compared to healthy controls, and decreased during reconvalescence to normal values (Figure 35). Activity of these pathways are not associated with activity of the adaptive immune system, but indicate activity of the inflammatory innate immune response. Measurements of activity cannot be used to assess severity during acute COVID-19 disease, but can be used for monitoring changes in disease severity, and based on that, predicting prognosis of the patient (see below).

### 1. Detailed description of the dataset analysis results, per pseudotime severity category

### a. Critically ill patients

To determine if the severity of disease (pseudo-time severity categories 1-7 in figure 35) could be linked to pathway scores we analyzed the RNA sequencing data. In Figure 35A and Table 2, the AP1, AR and STAT1-2 pathway activity scores of critically ill patients are shown, healthy individuals are plotted as HC.

All patients in this group showed low STAT1-2 pathway activity, further confirming that low JAK-STAT1/2 pathway activity is associated with severe disease
In non survivor patient 2, AR and AP1 pathway activity increased over time (pseudotime category 1 and 2).

Non-survivor Patient 3 had low pathway activity scores for all three pathways, probably associated with an impaired immune system due to chemotherapy treatment. Survivor-patient 12 showed decreasing pathway activity scores for the AP1 and AR pathways over time which was not seen for non-survivor patient 2 and 3. The decrease in AR and MAPK-AP1 pathway activity was associated with recovery. Lower activity of these pathways are likely to be associated with less severe inflammatory disease and activity of the innate immune system.

### b. Complicated patients

Complicated patients showed higher AP1, AR and STAT1-2 pathway activity compared to healthy controls (HC), and pathway activities decreased over the pseudotime during reconvalescence phase. The lowest pathway activity scores were observed in the recovery phase, see figure 35B, Table 2.

### c. Moderatetely ill patients

In the moderate ill patient group higher AR, MAPK-AP1 and JAK-STAT1/2 pathway activity scores compared to healthy controls HC) were only found with the pseudo score 4 and not in later pseudo scores (5-7) in which the patients are recovering and the pathway activities are normalized towards the healthy control levels, Figure 35C and A,

### d. Late convalescence/recovery patients

In the Late convalescence/recovery patient group (Figure 35D, Table 2), patient #6 showed decreasing pathway activity scores for the MAPK-AP1, AR and JAK-STAT1-2 pathways over the pseudotime.

For patient #11 several samples were taken during the same pseudotime phase 6. The MAPK-AP1 and AR pathway activity scores decreased over pseudotime, while JAK-STAT1-2 pathway activity scores showed a large variation during pseudotime 6 and entered the normal range during recovery (pseudotime 7).

### Changes in AR and MAPK pathway activity during disease progression and recovery (figure 36).

The incremental phase in AR and MAPK-AP1 pathway activity was observed in patient 4 and 10 who showed increased AP1 and AR pathway activity (and persistent high JAK-STAT1-2 pathway activity) in pseudotime nr 1 to 3. Such an increase was also seen in patient 1 who was re-admitted and in patient 2 (non-survivor). decreased pathway activities of patients 1, 4 and 10 was already seen at pseudotime 3 for patient 4 and pseudotime 5 for patients 1 and 10. See figure 36 and figure 34B for patient groups.

### Dataset GSE161731

Samples from subjects with COVID-19 were assigned to three groups based on time from symptom onset (early ≤10 days, middle 11-21 days, late >21 days). For comparison, we profiled banked blood samples from patients presenting to the emergency department with acute respiratory infection (ARI) due to seasonal coronavirus (n = 49), influenza (n = 17) or bacterial pneumonia (n = 23), and matched healthy controls (n = 19).

Pathway activities were determined for each sample, per group average pathway activities and mean values are indicasted in Table 3. This dataset further confirms that JAK-STAT1/2 pathway activity is initially high and then decreases in COVID-19 infections, likely due to the initial adaptive immun response which then subsides. The dataset further also confirms that JAK-STAT1/2 pathway activity is generally high in viral infections while not elevated in bacterial infections. Interestingly the JAK-STAT1/2 activity is much less elevated in COVID-19 patients compared to patients with seasonal coronavirus infections or influenza, suggesting a adaptive immune suppressive effect from SARS-CoV2. This could explain the generally more severe symptoms and further emphasize that those patients with lower JAK-STAT1/2 pathway activity (suggesting lower / no adaptie immune response) tend to havemuch more severe symptoms.

The dataset further shows a strong increase in AR pathway activity specfically in bacterial infected patients. Therefore the AR pathway activity can further be used to distinguish between bacterial and viral infected patients based on a blood sample.

### References

1. Wang D, Hu B, Hu C, Zhu F, Liu X, Zhang J, et al. Clinical Characteristics of 138 Hospitalized Patients With 2019 Novel Coronavirus-Infected Pneumonia in Wuhan, China. JAMA. 2020 Feb 7;
2. Zhou F, Yu T, Du R, Fan G, Liu Y, Liu Z, et al. Clinical course and risk factors for mortality of adult inpatients with COVID-19 in Wuhan, China: a retrospective cohort study. Lancet. 2020 28;395(10229):1054-62.
3. Verhaegh W, van Ooijen H, Inda MA, Hatzis P, Versteeg R, Smid M, et al. Selection of personalized patient therapy through the use of knowledge-based computational models that identify tumor-driving signal transduction pathways. Cancer Res. 2014 Jun 1;74(11):2936-45.
4. van Ooijen H, Hornsveld M, Dam-de Veen C, Velter R, Dou M, Verhaegh W, et al. Assessment of Functional Phosphatidylinositol 3-Kinase Pathway Activity in Cancer Tissue Using Forkhead Box-O Target Gene Expression in a Knowledge-Based Computational Model. Am J Pathol. 2018 Sep;188(9):1956-72.
5. Stolpe A van de, Holtzer L, van Ooijen H, de Inda MA, Verhaegh W. Enabling precision medicine by unravelling disease pathophysiology: quantifying signal transduction pathway activity across cell and tissue types. Sci Rep. 2019 Feb 7;9(1):1603.
6. Verhaegh W, Van de Stolpe A. Knowledge-based computational models. Oncotarget. 2014 Jul 30;5(14):5196-7.
7. van de Stolpe A. Quantitative Measurement of Functional Activity of the PI3K Signaling Pathway in Cancer. Cancers. 2019 Mar;11(3):293.
8. van Boxtel W, Verhaegh GW, van Engen-van Grunsven IA, van Strijp D, Kroeze LI, Ligtenberg MJ, et al. Prediction of clinical benefit from androgen deprivation therapy in salivary duct carcinoma patients. Int J Cancer. 2019 Nov 19;
9. Inda MA, Blok EJ, Kuppen PJK, Charehbili A, den Biezen-Timmermans EC, van Brussel A, et al. Estrogen Receptor Pathway Activity Score to Predict Clinical Response or Resistance to Neoadjuvant Endocrine Therapy in Primary Breast Cancer. Mol Cancer Ther. 2020 Feb;19(2):680-9.
10. Sieuwerts AM, Inda MA, Smid M, van Ooijen H, van de Stolpe A, Martens JWM, et al. ER and PI3K Pathway Activity in Primary ER Positive Breast Cancer Is Associated with Progression-Free Survival of Metastatic Patients under First-Line Tamoxifen. Cancers (Basel). 2020 Mar 27;12(4).
11. Stolpe AVD, Verhaegh W, Doorn A van, Noël G, Gu-Trantien C, Willard-Gallo K. Abstract 2371: Breast cancer induces tolerogenic state of healthy activated CD4+ lymphocytes, characterized by reduced PI3K, NFκB, JAK-STAT, Notch, and increased TGFβpathway activity. Cancer Res. 2019 Jul 1;79(13 Supplement):2371-2371.
12. K. A. Overmyer et al., "Large-Scale Multi-omic Analysis of COVID-19 Severity," cels, vol. 12, no. 1, pp. 23-40.e7, Jan. 2021, doi: 10.1016/j.cels.2020.10.003.
13. W. Bouwman, W. Verhaegh, L. Holtzer, and A. van de Stolpe, "Measurement of Cellular Immune Response to Viral Infection and Vaccination," Front Immunol, vol. 11, p. 575074, 2020, doi: 10.3389/fimmu.2020.575074.
14. T. Matsuyama, S. P. Kubli, S. K. Yoshinaga, K. Pfeffer, and T. W. Mak, "An aberrant STAT pathway is central to COVID-19," Cell Death & Differentiation, vol. 27, no. 12, Art. no. 12, Dec. 2020, doi: 10.1038/s41418-020-00633-7.
15. "Longitudinal Multi-omics Analyses Identify Responses of Megakaryocytes, Erythroid Cells, and Plasmablasts as Hallmarks of Severe COVID-19: Immunity." https://www.cell.com/immunity/fulltext/S1074-7613(20)30504-5?_returnURL=https%3A%2F%2Flinkinghub.elsevier.com%2Fretrieve%2Fpii%2FS107476 1320305045%3Fshowall%3Dtrue (accessed Mar. 29, 2021).
16. M. K. Angele, S. Pratschke, W. J. Hubbard, and I. H. Chaudry, "Gender differences in sepsis," Virulence, vol. 5, no. 1, pp. 12-19, Jan. 2014, doi: 10.4161/viru.26982.
17. M. R. Gubbels Bupp and T. N. Jorgensen, "Androgen-Induced Immunosuppression," Front Immunol, vol. 9, p. 794, 2018, doi: 10.3389/fimmu.2018.00794.

**Table 1**

| **Dataset** | **Sample type** | **Patient material of infected cells** | **Virus / bacteria type** | **Sample information** | **Reference** |
|---|---|---|---|---|---|
| GSE84331 | CD8 T Cell | Patient | Dengue hemorrha gic fever | PBMCs isolated from peripheral whole blood of dengue patients and healthy controls were stained with relevant antibodies at 4oC for 30 mins, washed thoroughly and suspended in PBS containg 2% FCS and immediately sorted on a BD FACS Aria III (Becton and Dickenson) with high forward scatter gates to account for the larger blasting effector lymphocytes. | https://ww w.ncbi.nlm. nih.gov/pu bmed/2770 7928 |
| | | | | CD3+CD8+CD45RA+CCR7+ naïve CD8 T cells and CD3+CD8+HLA-DR+CD38+ activated CD8 T cells were isolated up to a purity of 99% | |
| | CD8 T Cell | Patient | Dengue fever | | |
| GSE119322 | Dendriti c cells | Patient | Hepatitis B | Mononuclear cells were isolated from side-scatter and forward-scatter gating using fluorescence-activated cell sorting (FACS), the lineage-negative HLA-DR-positive fraction was extracted, and was further divided into CD123-positive plasmacytoid DCs (pDCs) and CD11c-positive myeloid DCs (mDCs). Cell sorting was performed, and the number of DCs was measured and surface markers were analyzed.RNA was extracted from sorted pDCs and subjected to gene expression analysis using Affymetrix Human 133U Plus 2.0 gene chip. | https://ww w.ncbi.nlm. nih.gov/pu bmed/2461 6721 |
| GSE52081 | Dendriti c cells | infected cells | Newcastl e disease virus | Human peripheral blood mononuclear cells were isolated from buffy coats by Ficoll density gradient centrifugation (Histopaque, Sigma Aldrich) at 1450 r.p.m. and CD14+ monocytes were immunomagnetically purified by using a MACS CD14 isolation kit (Miltenyi Biotech). Monocytes were then differentiated into naïve DCs by 5-6 day incubation at 37°C and 5% CO2 in DC growth media, which contains RPMI Medium 1640 (Invitrogen/Gibco) supplemented with 10% fetal calf serum (Hyclone), 2 mM of l--glutamine, 100 U/ml penicillin and 100 g/ml streptomycin (Pen/Strep) (Invitrogen), 500 U/ml hGM-CSF (Preprotech) and 1000 U/ml hIL-4 (Preprotech). Antiviral activated dendritic cells (AVDCs) were generated by employing a trans-well system. The trans-well system consists of an upper and a lower chamber separated by a 0.4 µm PET membrane (Millipore) that allows diffusion of cytokines and chemokines through the membrane but avoids the interaction of the cells in both chambers. To generate the AVDCs, naïve DCs were infected as described above. After the 40 minutes incubation, the cells were washed with PBS, and cultured in the trans-well system. Infected and non-infected DCs were allocated in the upper and lower chamber respectively. | https://ww w.ncbi.nlm. nih.gov/pu bmed/2461 6721 |
| GSE35283 | Monocy tes | infected cells | influenza low PR8 | Human monocytes were isolated from buffy coats of unrelated healthy blood donors. Cells were cultivated in Teflon bags in McCoy's modified medium (Biochrom AG, Berlin, Germany) supplemented with 1% glutamine, 1% penicillin-streptomycin and 15% fetal bovine serum overnight. Monocytes were infected with low (PR8) and high pathogenic influenza viruses (FPV (H7N7) and H5N1) | https://ww w.ncbi.nlm. nih.gov/pu bmed/2344 5660 |
| | Monocy tes | infected cells | influenza high FPV H7N7 | | |
| | Monocy tes | infected cells | influenza high H5N1 | | |
| GSE34205 | PBMCs | Patient | RSV | acute RSV or Influenza infection,children of median age 2.4 months (range 1.5-8.6) hospitalized with acute RSV and influenza virus infection were offered study enrollment after microbiologic confirmation of the diagnosis. Blood samples were collected from them within 42-72 hours of hospitalization. PBMCs were isolated within 6 h of sample collection by density gradient centrifugation using ficoll-hypaque and lysed in RLT reagent (Qiagen) with β-mercaptoethanol. Samples were run blind and in batches by the same laboratory team to ensure standardization of quality and handling. From 2-5 ug of total RNA, cDNA was generated as a template for single-round in vitro transcription with biotin-labeled nucleotides using the Affymetrix cDNA Synthesis and In Vitro Transcription kits (Affymetrix Inc.). | https://ww w.ncbi.nlm. nih.gov/pu bmed/2239 8282 |
| | PBMCs | Patient | Influenza | | |
| GSE43777 | PBMCs | Patient | Denque | DF (dengue fever) and DHF (dengue hemorrhagic fever) cases were used to study gene expression during the course of dengue acute illnessOnly if DENV infection was confirmed by RT-PCR, then serial blood samples were collected at 24, 48 and 72 hours following the initial sample, and one to two samples within 0-72 hours post-fever defervescence and one sample at ≥20 days (convalescent period) following the initial sample. Separation of plasma and PBMCs was performed by gradient centrifugation over Histopaque-Ficoll (Sigma, St. Louis, MO). | https://ww w.ncbi.nlm. nih.gov/pu bmed/2387 5036 |
| GSE50628 | PBMCs | Patient | Influenza H1N1 | The gene expression profiles in the peripheral whole blood with influenza A(H1N1)pdm09 or rotavirus gastroenteritis were examined. Whole blood samples were collected from patients in the acute phase of the disease and in the recovery phase. For patients with complex seizures, the blood samples were collected on the day of admission (acute phase: Flu, 1-3 days from disease onset; Rota, 2-4 days from disease onset) and on the day of discharge (recovery phase: Flu, 4-9 days from disease onset; Rota, 7-11 days from disease onset). After sample collection, the PAXgene tubes were incubated at room temperature for 2 h and then stored at -80 °C until RNA extraction. Total RNA was isolated using the PAXgene Blood RNA Kit (Qiagen). | https://ww w.ncbi.nlm. nih.gov/pu bmed/2446 4411 |
| | PBMCs | Patient | rota virus | | |
| GSE69606 | PBMCs | Patient | RSV | patients with acute RSV infections, divided into mild, moderate and severe disease. From moderate and severe diseased patients recovery samples were obtained as well. Peripheral blood mononuclear cells (PBMCs) were isolated by density gradient centrifugation (Lymphoprep, Axis Shield, Norway), counted and subsequently stored in Trizol reagent (Invitrogen, The Netherlands) at -80°C in the same laboratory by the same team for both cohorts. RNA from PBMC was extracted using Trizol (Invitrogen Life Technologies) according to the manufacturer's protocol. Total RNA was isolated using the RNeasy Minikit (Qiagen). | https://ww w.ncbi.nlm. nih.gov/pu bmed/2616 2090 |
| GSE6269 | PBMCs | Patient | Influenza | Peripheral blood samples from pediatric patients with acute infections of Influenza, S. pneumoniae or S. aureus. All blood samples were collected in acid-citrate-dextrose tubes (BD Vacutainer, Becton Dickinson, Franklin Lakes, NJ) at the CMC and immediately delivered at room temperature to the Baylor Institute for Immunology Research (Dallas, TX) for processing. Peripheral blood mononuclear cells (PBMCs) from 3 to 4 mL blood were isolated via Ficoll gradient and immediately lysed in RLT reagent (Qiagen, Valencia, CA) with β-mercaptoethanol (BME) and stored at -80°C. Total RNA was isolated using the RNeasy kit (Qiagen) according to the manufacturer's instructions, and RNA integrity was assessed by using an Agilent 2100 Bioanalyzer (Agilent, Palo Alto, CA). | https://ww w.ncbi.nlm. nih.gov/pu bmed/1710 5821 |
| | PBMCs | Patient | S. pneumoni ae | | |
| | PBMCs | Patient | S. aureus | | |
| GSE102459 | Whole blood and BPMCs | Patient | tuberculo sis | All eligible participants were stipulated to receive the candidate vaccine M72/AS01_{E} (referred to as M72/AS01 in the article; GSK, Rixensart, Belgium) by intramuscular injection at Days 0 and 30. PBMC were collected at Days 0, 31, and 44; and with WB-derived samples on Days 0, 30, 31, 37, 40, 44, and 47. BMCs were separated on Lymphoprep gradients, washed, counted by flow cytometry, frozen and further stored in liquid nitrogen until time of further evaluation. At least 10 ml of blood for WB gene expression analysis was collected in PAXgene tubes. | https://ww w.ncbi.nlm. nih.gov/pmc /articles/PM C5879450/ |
| GSE89292 | PBMCs | Patient | Malaria | Study arm 1 (ARR), comprised of volunteers who received the AdVac vaccine composed of Ad35 vector expressing full-length CSP, as a primary immunization, was followed by two doses of RTS,S/AS01 vaccine. The subjects in the second arm, , received three doses of RTS,S/AS01 (RRR regimen). Participants in both study arms were vaccinated at 28-d intervals, and subjected to CHMI 21 d following the final immunization. mRNA was isolated from frozen PBMCs | https://ww w.ncbi.nlm. nih.gov/pmc /articles/PM C5338562/ |
| GSE4607 | PBMCs | Patient | bacterial infections | Children < 10 years of age admitted to the pediatric intensive care unit and meeting the criteria for either SIRS or septic shock were eligible for the study. SIRS and septic shock were defined based on pediatric-specific criteria. We did not use separate categories of "sepsis" or "severe sepsis". Patients meeting criteria for "sepsis" or "severe sepsis" were placed in the categories of SIRS and septic shock, respectively, for study purposes. Total RNA was extracted from whole blood samples using the PaxGene Blood RNA System (PreAnalytiX, Qiagen/Becton Dickson) according the manufacturer's specifications. Quality control steps: RNA quality was assessed by using the Agilent bioanalyzer (Agilent Technologies, Palo Alto, CA) and only those samples with 28S/18S ratios between 1.3 and 2 were subsequently used | https://ww w.ncbi.nlm. nih.gov/pu bmed/1851 1707 |
| GSE13486 | PBMCs | Patient | YFV-17, live attenuate d yellow fever virus strain without adjuvant | vaccination in healthy individuals with Yellow Fever vaccine | https://pub med.ncbi.nl m.nih.gov/ 19029902/ |
| GSE157103 | Whole blood | COVID-19 patients and non-COVID-19 patients | COVID-19 | Large-scale Multi-omic Analysis of COVID-19 Severity | https://pub med.ncbi.nl m.nih.gov/ 33096026/ |
| GSE161777 | peripher al blood | 13 patients were sampled at days 0, 2, 7, 10, 13 and/or at discharge. | COVID-19 | Longitudinal multi-omics identifies responses of megakaryocytes, erythroid cells and plasmablasts as hallmarks of severe COVID-19 trajectories [sequencing] | https://pub med.ncbi.nl m.nih.gov/ 33296687/ |
| GSE161731 | Whole blood | Expressio n profiling by high throughp ut sequencin g | COVID-19, seasonal coronavir us, influenza, bacterial pneumoni a | Dysregulated transcriptional responses to SARS-CoV-2 in the periphery support novel diagnostic approaches | https://pub med.ncbi.nl m.nih.gov/ 33597532/ |

**Table 2**

| title | pseudotime | remission | AP1 | AR | STAT1 |
|---|---|---|---|---|---|
| Healthy control 01 | 0 | Healthy | 10.8 | 6.3 | 21.9 |
| Healthy control 02 | 0 | Healthy | 13.5 | 9.5 | 36.9 |
| Healthy control 03 | 0 | Healthy | 15.2 | 9.0 | 40.1 |
| Healthy control 04 | 0 | Healthy | 14.3 | 9.7 | 34.0 |
| Healthy control 05 | 0 | Healthy | 7.8 | 4.6 | 21.5 |
| Healthy control 06 | 0 | Healthy | 18.6 | 11.1 | 77.0 |
| Healthy control 07 | 0 | Healthy | 13.7 | 5.5 | 69.6 |
| Healthy control 08 | 0 | Healthy | 11.2 | 1.5 | 44.9 |
| Healthy control 09 | 0 | Healthy | 13.7 | 8.5 | 34.2 |
| Healthy control 10 | 0 | Healthy | 13.3 | 8.1 | 24.0 |
| Healthy control 11 | 0 | Healthy | 9.4 | 4.7 | 25.0 |
| Healthy control 11 | 0 | Healthy | 9.6 | 5.1 | 25.0 |
| Healthy control 12 | 0 | Healthy | 13.5 | 8.5 | 20.4 |
| Healthy control 12 | 0 | Healthy | 13.4 | 8.8 | 20.4 |
| Healthy control 13 | 0 | Healthy | 12.1 | 6.6 | 23.0 |
| Healthy control 13 | 0 | Healthy | 12.0 | 6.7 | 23.1 |
| Healthy control 14 | 0 | Healthy | 10.7 | 6.7 | 16.8 |
| Healthy control 14 | 0 | Healthy | 10.8 | 6.7 | 16.9 |
| patient1: COVID19 (Remission) t=4 | 4 | Remission | 24.5 | 9.8 | 83.2 |
| patient1: COVID19 (Remission) t=4 | 4 | Remission | 23.0 | 9.9 | 81.2 |
| patient1: COVID19 (Remission) t=4 | 4 | Remission | 30.5 | 17.4 | 70.9 |
| patient1: COVID19 (Remission) t=4 | 4 | Remission | 27.3 | 17.4 | 70.5 |
| patient1: COVID19 (Remission) t=5 | 5 | Remission | 15.8 | 11.3 | 34.5 |
| patient1: COVID19 (Remission) t=5 | 5 | Remission | 15.9 | 10.8 | 35.0 |
| patient1: COVID19 (Remission) t=6 | 6 | Remission | 12.7 | 6.0 | 27.2 |
| patient1: COVID19 (Remission) t=6 | 6 | Remission | 12.7 | 6.7 | 27.4 |
| patient1: COVID19 (Remission) t=6 | 6 | Remission | 12.1 | 6.1 | 27.2 |
| patient1: COVID19 (Remission) t=6 | 6 | Remission | 12.2 | 6.2 | 26.7 |
| patient10: COVID19 (Remission) t=1 | 1 | Remission | 24.1 | 16.6 | 66.1 |
| patient10: COVID19 (Remission) t=1 | 1 | Remission | 26.9 | 15.6 | 70.7 |
| patient10: COVID19 (Remission) t=3 | 3 | Remission | 24.7 | 19.9 | 80.8 |
| patient10: COVID19 (Remission) t=3 | 3 | Remission | 24.7 | 19.6 | 80.9 |
| patient10: COVID19 (Remission) t=3 | 3 | Remission | 15.4 | 9.1 | 66.4 |
| patient10: COVID19 (Remission) t=3 | 3 | Remission | 15.4 | 9.5 | 67.2 |
| patient10: COVID19 (Remission) t=4 | 4 | Remission | 16.6 | 15.1 | 62.7 |
| patient10: COVID19 (Remission) t=4 | 4 | Remission | 17.0 | 15.2 | 64.3 |
| patient10: COVID19 (Remission) t=5 | 5 | Remission | 10.4 | 2.6 | 56.6 |
| patient10: COVID19 (Remission) t=5 | 5 | Remission | 10.2 | 2.2 | 56.6 |
| patient11: COVID19 (Remission) t=6 | 6 | Remission | 17.4 | 14.9 | 27.1 |
| patient11: COVID19 (Remission) t=6 | 6 | Remission | 17.6 | 14.9 | 26.7 |
| patient11: COVID19 (Remission) t=6 | 6 | Remission | 17.6 | 16.1 | 34.2 |
| patient11: COVID19 (Remission) t=6 | 6 | Remission | 17.4 | 15.8 | 33.6 |
| patient11: COVID19 (Remission) t=6 | 6 | Remission | 17.6 | 16.2 | 44.2 |
| patient11: COVID19 (Remission) t=6 | 6 | Remission | 17.5 | 15.9 | 45.3 |
| patient11: COVID19 (Remission) t=6 | 6 | Remission | 18.4 | 15.1 | 53.9 |
| patient11: COVID19 (Remission) t=6 | 6 | Remission | 18.2 | 14.8 | 53.6 |
| patient12: COVID19 (Remission) t=2 | 2 | Remission | 17.1 | 7.2 | 41.7 |
| patient12: COVID19 (Remission) t=2 | 2 | Remission | 16.8 | 7.3 | 41.9 |
| patient12: COVID19 (Remission) t=3 | 3 | Remission | 2.0 | 0.0 | 13.5 |
| patient12: COVID19 (Remission) t=3 | 3 | Remission | 2.2 | 0.0 | 13.6 |
| patient12: COVID19 (Remission) t=5 | 5 | Remission | 3.8 | 0.3 | 17.9 |
| patient12: COVID19 (Remission) t=5 | 5 | Remission | 4.4 | 0.2 | 17.8 |
| patient12: COVID19 (Remission) t=5 | 5 | Remission | 0.6 | 0.1 | 8.7 |
| patient12: COVID19 (Remission) t=5 | 5 | Remission | 0.7 | 0.1 | 8.4 |
| patient12: COVID19 (Remission) t=5 | 5 | Remission | 1.5 | 0.2 | 14.5 |
| patient12: COVID19 (Remission) t=5 | 5 | Remission | 1.8 | 0.3 | 14.3 |
| patient13: COVID19 (Remission) t=4 | 4 | Remission | 25.5 | 14.8 | 70.2 |
| patient13: COVID19 (Remission) t=4 | 4 | Remission | 24.4 | 14.4 | 67.1 |
| patient13: COVID19 (Remission) t=5 | 5 | Remission | 11.6 | 5.5 | 32.5 |
| patient13: COVID19 (Remission) t=5 | 5 | Remission | 11.0 | 5.7 | 31.9 |
| patient14: COVID19 (Remission) t=4 | 4 | Remission | 21.8 | 7.6 | 74.1 |
| patient14: COVID19 (Remission) t=4 | 4 | Remission | 21.7 | 7.6 | 75.3 |
| patient14: COVID19 (Remission) t=5 | 5 | Remission | 22.3 | 14.3 | 50.1 |
| patient14: COVID19 (Remission) t=5 | 5 | Remission | 20.7 | 14.0 | 51.0 |
| patient2: COVID19 (No Remission) t=1 | 1 | No Remission | 18.1 | 9.2 | 29.3 |
| patient2: COVID19 (No Remission) t=1 | 1 | No Remission | 18.3 | 9.2 | 29.3 |
| patient2: COVID19 (No Remission) t=2 | 2 | No Remission | 21.7 | 13.5 | 29.7 |
| patient2: COVID19 (No Remission) t=2 | 2 | No Remission | 21.5 | 13.5 | 29.6 |
| patient3: COVID19 (No Remission) t=2 | 2 | No Remission | 13.5 | 6.1 | 24.3 |
| patient3: COVID19 (No Remission) t=2 | 2 | No Remission | 13.5 | 5.8 | 24.2 |
| patient4: COVID19 (Remission) t=1 | 1 | Remission | 29.1 | 14.3 | 80.3 |
| patient4: COVID19 (Remission) t=1 | 1 | Remission | 28.1 | 14.1 | 80.1 |
| patient4: COVID19 (Remission) t=3 | 3 | Remission | 27.7 | 18.6 | 82.5 |
| patient4: COVID19 (Remission) t=3 | 3 | Remission | 28.4 | 18.1 | 80.0 |
| patient4: COVID19 (Remission) t=5 | 5 | Remission | 15.3 | 8.8 | 30.7 |
| patient4: COVID19 (Remission) t=5 | 5 | Remission | 15.0 | 8.4 | 31.5 |
| patient5: COVID19 (Remission) t=1 | 1 | Remission | 19.8 | 12.5 | 68.3 |
| patient5: COVID19 (Remission) t=1 | 1 | Remission | 19.8 | 12.5 | 68.1 |
| patient5: COVID19 (Remission) t=3 | 3 | Remission | 16.6 | 8.1 | 48.4 |
| patient5: COVID19 (Remission) t=3 | 3 | Remission | 16.8 | 7.8 | 48.6 |
| patient5: COVID19 (Remission) t=6 | 6 | Remission | 5.7 | 2.1 | 9.6 |
| patient5: COVID19 (Remission) t=6 | 6 | Remission | 6.3 | 1.5 | 9.4 |
| patient5: COVID19 (Remission) t=6 | 6 | Remission | 3.7 | 2.7 | 6.5 |
| patient5: COVID19 (Remission) t=6 | 6 | Remission | 3.6 | 2.5 | 6.3 |
| patient6: COVID19 (Remission) t=5 | 5 | Remission | 24.5 | 10.9 | 69.0 |
| patient6: COVID19 (Remission) t=5 | 5 | Remission | 25.1 | 10.8 | 68.5 |
| patient6: COVID19 (Remission) t=5 | 5 | Remission | 25.6 | 12.8 | 55.6 |
| patient6: COVID19 (Remission) t=5 | 5 | Remission | 23.9 | 12.6 | 55.2 |
| patient6: COVID19 (Remission) t=6 | 6 | Remission | 11.0 | 6.2 | 15.6 |
| patient6: COVID19 (Remission) t=6 | 6 | Remission | 11.0 | 5.7 | 15.7 |
| patient6: COVID19 (Remission) t=7 | 7 | Remission | 9.5 | 4.0 | 20.6 |
| patient6: COVID19 (Remission) t=7 | 7 | Remission | 9.5 | 3.1 | 20.4 |
| patient7: COVID19 (Remission) t=7 | 7 | Remission | 14.0 | 11.8 | 39.1 |
| patient7: COVID19 (Remission) t=7 | 7 | Remission | 13.8 | 11.7 | 40.4 |
| patient8: COVID19 (Remission) t=3 | 3 | Remission | 22.7 | 12.6 | 71.6 |
| patient8: COVID19 (Remission) t=3 | 3 | Remission | 21.8 | 12.7 | 76.9 |
| patient9: COVID19 (Remission) t=4 | 4 | Remission | 15.7 | 9.2 | 30.1 |
| patient9: COVID19 (Remission) t=4 | 4 | Remission | 15.8 | 9.1 | 31.2 |
| patient9: COVID19 (Remission) t=5 | 5 | Remission | 10.5 | 7.7 | 16.3 |
| patient9: COVID19 (Remission) t=5 | 5 | Remission | 10.5 | 7.9 | 16.5 |
| patient9: COVID19 (Remission) t=6 | 6 | Remission | 5.1 | 4.9 | 8.2 |
| patient9: COVID19 (Remission) t=6 | 6 | Remission | 5.2 | 4.9 | 8.1 |

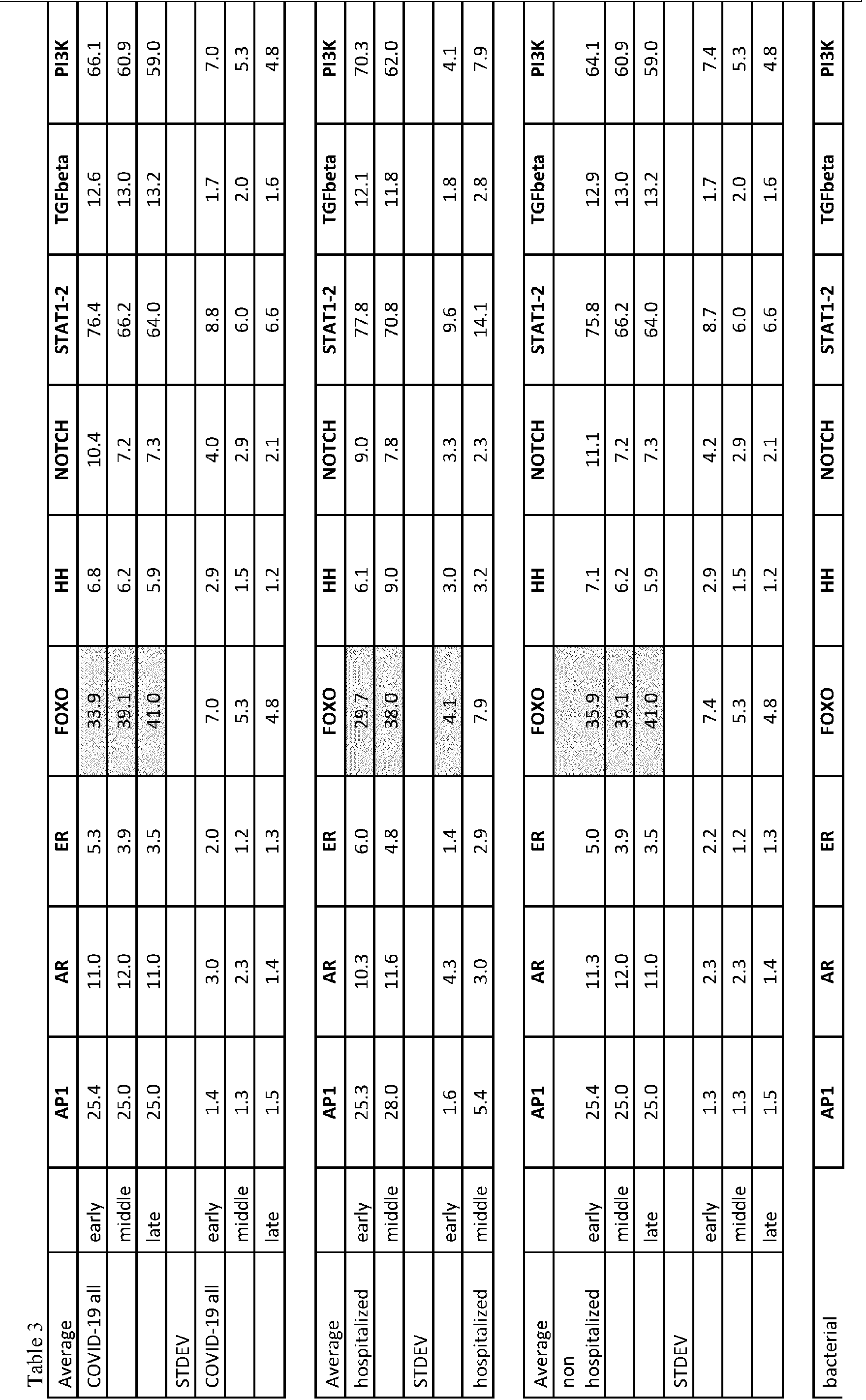

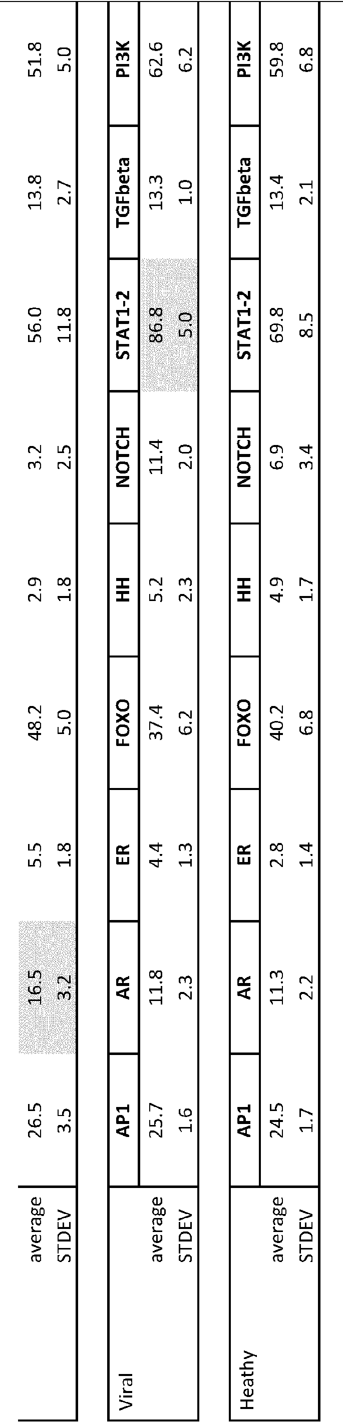

## Claims

1. A computer implemented method for distinguishing between a bacterial and a viral infection in a blood sample obtained from a subject with an infection, based on the determined expression levels of three or more target genes of the JAK-STAT1/2 cellular signaling pathway, the method comprising:
- receiving the determined expression levels of the three or more target genes of the JAK-STAT1/2 cellular signaling pathway;
- determining the JAK-STAT1/2 cellular signaling pathway activity, wherein the determining the JAK-STAT1/2 cellular signaling pathway activity comprises assigning a numeric value to the JAK-STAT1/2 cellular signaling pathway activity level by evaluating a calibrated mathematical pathway model relating the expression levels of the three or more JAK-STAT1/2 target genes to an activity level of the JAK-STAT1/2 cellular signaling pathway,
wherein the method further comprises determining an activity level of a signaling pathway associated transcription factor (TF) element, the signaling pathway associated TF element controlling transcription of the three or more target genes, the determining being based on evaluating a calibrated mathematical pathway model relating expression levels of the target genes to the activity level of the JAK-STAT1/2 cellular signaling pathway, and
inferring the activity of the JAK-STAT1/2 cellular signaling pathway in the blood sample based on the determined activity level of the signaling pathway associated TF element,
- comparing the JAK-STAT1/2 cellular signaling pathway activity determined in the blood sample obtained from the subject with the JAK-STAT1/2 cellular signaling pathway activity determined in a reference blood sample,
wherein the reference blood sample is obtained from a healthy subject or a subject recovered from an infection,
and wherein the infection in the subject from which the blood sample is obtained is determined to be viral when the JAK-STAT1/2 cellular signaling pathway activity is higher compared to the JAK-STAT1/2 cellular signaling pathway activity in the reference blood sample, or
wherein the infection in the subject from which the blood sample is obtained is determined to be bacterial when the JAK-STAT1/2 cellular signaling pathway activity is not higher compared to the JAK-STAT1/2 cellular signaling pathway activity in the reference blood sample,
wherein the calibrated mathematical pathway model is a centroid or a linear model, or a Bayesian network model based on conditional probabilities, and
wherein the three or more target genes of the JAK-STAT1/2 cellular signaling pathway are selected from the group consisting of:
BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFD1L, USP18, and ZNRF3.

2. A computer implemented method for determining the cellular immune response to a viral infection or a vaccine in a blood sample obtained from a subject with a viral infection or a subject who received a vaccine, based on the determined expression levels of three or more target genes of the JAK-STAT1/2 cellular signaling pathway, the method comprising:
- receiving the determined expression levels of the three or more target genes of the JAK-STAT1/2 cellular signaling pathway;
- determining the JAK-STAT1/2 cellular signaling pathway activity, wherein the determining the JAK-STAT1/2 cellular signaling pathway activity comprises assigning a numeric value to the JAK-STAT1/2 cellular signaling pathway activity level by evaluating a calibrated mathematical pathway model relating the expression levels of the three or more JAK-STAT1/2 target genes to an activity level of the JAK-STAT1/2 cellular signaling pathway;
- comparing the JAK-STAT1/2 cellular signaling pathway activity determined in the blood sample obtained from the subject with a viral infection or a subject who received a vaccine with the JAK-STAT1/2 cellular signaling pathway activity determined in a reference blood sample obtained from a healthy subject,
wherein the activity of the JAK-STAT1/2 cellular signaling pathway is compared with the cellular signaling pathway activities determined in the reference blood samples in order to determine whether the immune response to the viral infection is weak or strong, wherein the method further comprises:
- receiving the determined expression levels of the three or more target genes of the JAK-STAT3 cellular signaling pathway;
- determining the JAK-STAT3 cellular signaling pathway activity, wherein the determining the JAK-STAT3 cellular signaling pathway activity comprises assigning a numeric value to the JAK-STAT3 cellular signaling pathway activity level by evaluating a calibrated mathematical pathway model relating expression levels of the target genes to the activity level of the JAK-STAT3 cellular signaling pathway;
wherein the method further comprises determining an activity level of a signaling pathway associated transcription factor (TF) element, the signaling pathway associated TF element controlling transcription of the three or more target genes, the determining being based on evaluating a calibrated mathematical pathway model relating expression levels of the target genes to the activity level of the JAK-STAT1/2 cellular signaling pathway, and
inferring the activity of the JAK-STAT1/2 cellular signaling pathway in the blood sample based on the determined activity level of the signaling pathway associated TF element,
- comparing the JAK-STAT3 cellular signaling pathway activity determined in the blood sample obtained from the subject with a viral infection or a subject who received a vaccine with the JAK-STAT3 cellular signaling pathway activity determined in a reference blood sample obtained from a healthy subject,
wherein the calibrated mathematical pathway model is a centroid or a linear model, or a Bayesian network model based on conditional probabilities, and
wherein the three or more target genes of the JAK-STAT1/2 cellular signaling pathway are selected from the group consisting of:
BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFD1L, USP18, and ZNRF3, and
wherein the three or more target genes of the JAK-STAT3 cellular signaling pathway are selected from the group consisting of:
AKT1, BCL2, BCL2L1, BIRC5, CCND1, CD274, CDKNIA, CRP, FGF2, FOS, FSCN1, FSCN2, FSCN3, HIFIA, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST1, VIM and ZEB1.

3. The computer implemented method according to claim 2, wherein the immune response to a viral infection is considered weak when the numeric value assigned to the JAK-STAT1/2 cellular signaling pathway activity in the blood sample obtained from the subject with a viral infection or a subject who received a vaccine is one standard deviation higher than the numerical value assigned to the JAK-STAT1/2 cellular signaling pathway activity in the reference blood sample of the healthy subject and the immune response to a viral infection is considered strong when the numeric value assigned to the JAK-STAT1/2 cellular signaling pathway activity in the blood sample obtained from the subject with a viral infection or a subject who received a vaccine is at least two, preferably three or more, standard deviations higher than the numerical value assigned to the JAK-STAT1/2 cellular signaling pathway activity in the reference blood sample of the healthy subject.

4. The computer implemented method according to claim 2, wherein comparing the JAK-STAT1/2 and optionally the JAK-STAT3 cellular signaling pathway activities determined in the blood sample obtained from the subject with a viral infection or the subject who received a vaccine further comprises comparing with the JAK-STAT1/2 and optionally the JAK-STAT3 cellular signaling pathway activities determined in a reference blood sample obtained from a reference patient with a weak immune response and the JAK-STAT1/2 and optionally the JAK-STAT3 cellular signaling pathway activities determined in a reference blood sample obtained from a reference patient with a strong immune response, and wherein the strength of the immune response in the subject with a viral infection or the subject who received a vaccine is based on the comparison between the JAK-STAT1/2 cellular signaling pathway activities from the subject with a viral infection or the subject who received a vaccine with the JAK-STAT1/2 cellular signaling pathway activities determined in the reference blood samples obtained from the reference patient with a weak immune response and the reference blood samples obtained from the reference patient with a strong immune response.

5. The computer implemented method according to any one of claims 2 to 4, wherein the JAK-STAT1/2 cellular signaling pathway activity corresponds to the strength of the immune response, wherein a higher JAK-STAT1/2 cellular signaling pathway activity signifies a stronger immune response, optionally wherein the blood sample is from a subject with a viral infection and wherein a higher JAK-STAT3 cellular signaling pathway activity is indicative of a more severe infection or wherein the blood sample is from a subject who receives a vaccine and wherein a stronger immune response and optionally a higher JAK-STAT3 cellular signaling pathway activity is indicative of a stronger cellular immunity.

6. The computer implemented method according to any one of the preceding claims, wherein the determined activity levels of the JAK-STAT1/2 and optionally the JAK-STAT3 cellular signaling pathways are further used to
- monitor a patient with an infection, or
- determine the strength of the cellular immunity induced by a viral infection or vaccination in an individual, or
- predict the response to an immune modulatory therapy or drug, or
- monitor the response to a drug or therapy, or
- predict the toxicity of an immunomodulatory therapy or drug, or
- estimate the strength of the cellular immunity that will result in a community during an viral infection epidemic/pandemic, or
- determine the strength of the immunity induced by viral infection or vaccination in an individual with a specific immune compromising condition, such as a specific comorbidity, therapy, lifestyle, or
- diagnose patients with an viral infection during an epidemic or pandemic , or
- develop an drug or therapy to treat the infectious disease, or
- predict the immune activating or immune suppressive state caused by the viral infection.

7. The computer implemented method according to any one of the preceding claims wherein the blood sample is whole blood sample, a peripheral blood mononuclear cell sample, or isolated blood cells such as dendritic cells, CD4+ T cells, CD8+ T cells, CD16-monocytes, CD16+ monocytes, Neutrophils, NK cells and B cells.

8. The computer implemented method according to any one of the preceding claims wherein the activity of the JAK-STAT1/2 cellular signaling pathway and optionally the JAK-STAT3 cellular signaling pathway in the blood sample is inferable by a method comprising: receiving expression levels of three or more target genes of the JAK-STAT1/2 cellular signaling pathway and optionally the JAK-STAT3 cellular signaling pathway,
determining an activity level of a signaling pathway associated transcription factor (TF) element, the signaling pathway associated TF element controlling transcription of the three or more target genes, the determining being based on evaluating a calibrated mathematical pathway model relating expression levels of the target genes to the activity level of the JAK-STAT1/2 cellular signaling pathway and optionally the JAK-STAT3 cellular signaling pathway, and
inferring the activity of the JAK-STAT1/2 cellular signaling pathway and optionally the JAK-STAT3 cellular signaling pathway in the blood sample based on the determined activity level of the signaling pathway associated TF element,
wherein the calibrated mathematical pathway model is preferably a centroid or a linear model, or a Bayesian network model based on conditional probabilities.

9. A non-transitory storage medium storing instructions that are executable by a digital processing device to perform the method of any of claims 1 to 8.

10. A computer program comprising program code means for causing digital processing device to perform the method of any of claims 1 to 8, when the computer program is run on a digital processing device.

11. A kit of parts, comprising components for determining the expression levels of three or more target genes of the JAK-STAT1/2 cellular signaling pathway and three or more target genes of the JAK-STAT3 cellular signaling pathway,
wherein the three or more target genes of the JAK-STAT1/2 cellular signaling pathway are selected from the group consisting of:
BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFD1L, USP18, ZNRF3, GBP1, TAP1, ISG15, APOL1, IFI6, IFIRM1, CXCL9, APOL2, IFIT2 and LY6E, preferably, from the group consisting of: IRF1, IRF7, IRF8, IRF9, OAS1, PDCD1, ST13, STAT1 and USP1 or from the group consisting of GBP1, IRF9, STAT1, TAP1, ISG15, APOL1, IRF1, IRF7, IFI6, IFIRM1, USP18, CXCL9, OAS1, APOL2, IFIT2 and LY6E, and
wherein the three or more target genes of the JAK-STAT3 cellular signaling pathway are selected from the group consisting of: AKT1, BCL2, BCL2L1, BIRC5, CCND1, CD274, CDKNIA, CRP, FGF2, FOS, FSCN1, FSCN2, FSCN3, HIFIA, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST1, VIM and ZEB1, and
wherein the components for determining the expression level are primers and probes, and optionally further comprising the non-transitory storage medium of claim 9 or the computer program of claim 10.

12. Use of the kit of claim 11 in a method for distinguishing between a bacterial and a viral infection in a blood sample obtained from a subject with an infection, based on the determined expression levels of three or more target genes of the JAK-STAT1/2 cellular signaling pathway, the method comprising:
- determining the expression levels of the three or more target genes of the JAK-STAT1/2 cellular signaling pathway;
- determining the JAK-STAT1/2 cellular signaling pathway activity, wherein the determining the JAK-STAT1/2 cellular signaling pathway activity comprises assigning a numeric value to the JAK-STAT1/2 cellular signaling pathway activity level by evaluating a calibrated mathematical pathway model relating the expression levels of the three or more JAK-STAT1/2 target genes to an activity level of the JAK-STAT1/2 cellular signaling pathway,
wherein the method further comprises determining an activity level of a signaling pathway associated transcription factor (TF) element, the signaling pathway associated TF element controlling transcription of the three or more target genes, the determining being based on evaluating a calibrated mathematical pathway model relating expression levels of the target genes to the activity level of the JAK-STAT1/2 cellular signaling pathway, and
inferring the activity of the JAK-STAT1/2 cellular signaling pathway in the blood sample based on the determined activity level of the signaling pathway associated TF element,
- comparing the JAK-STAT1/2 cellular signaling pathway activity determined in the blood sample obtained from the subject with the JAK-STAT1/2 cellular signaling pathway activity determined in a reference blood sample,
wherein the reference blood sample is obtained from a healthy subject or a subject recovered from an infection,
and wherein the infection in the subject from which the blood sample is obtained is determined to be viral when the JAK-STAT1/2 cellular signaling pathway activity is higher compared to the JAK-STAT1/2 cellular signaling pathway activity in the reference blood sample, or
wherein the infection in the subject from which the blood sample is obtained is determined to be bacterial when the JAK-STAT1/2 cellular signaling pathway activity is not higher compared to the JAK-STAT1/2 cellular signaling pathway activity in the reference blood sample,
wherein the calibrated mathematical pathway model is a centroid or a linear model, or a Bayesian network model based on conditional probabilities, and
wherein the three or more target genes of the JAK-STAT1/2 cellular signaling pathway are selected from the group consisting of:
BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFD1L, USP18, and ZNRF3, preferably wherein the method is for the *in vitro* or *ex vivo* diagnosis of a viral infection in a subject.

13. A computer implemented method for classifying a subject with a viral infection as critical based on a blood sample obtained from a subject with a confirmed viral infection, based on the determined expression levels of three or more target genes of the JAK-STAT1/2 cellular signaling pathway, the method comprising:
- receiving the determined expression levels of the three or more target genes of the JAK-STAT1/2 cellular signaling pathway;
- determining the JAK-STAT1/2 cellular signaling pathway activity, wherein the determining the JAK-STAT1/2 cellular signaling pathway activity comprises assigning a numeric value to the JAK-STAT1/2 cellular signaling pathway activity level by evaluating a calibrated mathematical pathway model relating the expression levels of the three or more JAK-STAT1/2 target genes to an activity level of the JAK-STAT1/2 cellular signaling pathway; and
wherein the method further comprises determining an activity level of a signaling pathway associated transcription factor (TF) element, the signaling pathway associated TF element controlling transcription of the three or more target genes, the determining being based on evaluating a calibrated mathematical pathway model relating expression levels of the target genes to the activity level of the JAK-STAT1/2 cellular signaling pathway, and
inferring the activity of the JAK-STAT1/2 cellular signaling pathway in the blood sample based on the determined activity level of the signaling pathway associated TF element,
comparing the JAK-STAT1/2 cellular signaling pathway activity determined in the blood sample obtained from the subject with the JAK-STAT1/2 cellular signaling pathway activity determined in a reference blood sample,
wherein the reference blood sample is obtained from a healthy subject,
and wherein the subject with the viral infection is considered critical if the determined JAK-STAT1/2 pathway activity is equal or lower than the JAK-STAT1/2 pathway activity determined in the reference blood sample of the healthy subject, or wherein the subject with the viral infection is considered not critical if the determined JAK-STAT1/2 pathway activity is higher than the JAK-STAT1/2 pathway activity determined in the reference blood sample of the healthy subject,
wherein said blood sample from the subject with the confirmed viral infection has been obtained during the acute phase of said infection,
wherein the calibrated mathematical pathway model is a centroid or a linear model, or a Bayesian network model based on conditional probabilities, and
wherein the three or more target genes of the JAK-STAT1/2 cellular signaling pathway are selected from the group consisting of:
BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFD1L, USP18, and ZNRF3.

## Patentansprüche

1. Computer-implementiertes Verfahren zum Unterscheiden zwischen einer bakteriellen Infektion und einer Virusinfektion in einer aus einem Subjekt mit einer Infektion entnommenen Blutprobe, basierend auf den ermittelten Expressionsniveaus von drei oder mehr Zielgenen des zellulären JAK-STAT1/2-Signalgebungswegs, wobei das Verfahren umfasst:
- Empfangen der ermittelten Expressionsniveaus der drei oder mehr Zielgene des zellulären JAK-STAT1/2-Signalgebungswegs;
- Ermitteln der Aktivität des zellulären JAK-STAT1/2-Signalgebungswegs, wobei das Ermitteln der Aktivität des zellulären JAK-STAT1/2-Signalgebungswegs das Zuweisen eines numerischen Werts an das Aktivitätsniveau des zellulären JAK-STAT1/2-Signalgebungswegs durch Evaluierung eines kalibrierten mathematischen Pfad-Modells, das die Expressionsniveaus der drei oder mehr JAK-STAT1/2-Zielgene mit einem Aktivitätsniveau des zellulären JAK-STAT1/2-Signalgebungswegs in Zusammenhang bringt, umfasst,
wobei das Verfahren ferner das Ermitteln eines Aktivitätsniveaus eines Signalgebungsweg-assoziierten Transkriptionsfaktor (TF)-Elements umfasst, wobei das Signalgebungsweg-assoziierte TF-Element die Transkription der drei oder mehr Zielgene steuert, wobei das Ermitteln auf der Evaluierung eines kalibrierten mathematischen Pfad-Modells, das Expressionsniveaus der Zielgene mit dem Aktivitätsniveau des zellulären JAK-STAT1/2-Signalgebungswegs in Zusammenhang bringt, basiert, und
Ableiten der Aktivität des zellulären JAK-STAT1/2-Signalgebungswegs in der Blutprobe basierend auf dem ermittelten Aktivitätsniveau des Signalgebungsweg-assoziierten TF-Elements,
- Vergleichen der Aktivität des zellulären JAK-STAT1/2-Signalgebungswegs, ermittelt in der aus dem Subjekt entnommenen Blutprobe, mit der Aktivität des zellulären JAK-STAT1/2-Signalgebungswegs, die in einer Referenzblutprobe ermittelt wurde,
wobei die Referenzblutprobe aus einem gesunden Subjekt oder einem Subjekt, das sich von einer Infektion erholt hat, entnommen wird,
und wobei die Infektion in dem Subjekt, aus dem die Blutprobe entnommen wird, als viral bestimmt wird, wenn die Aktivität des zellulären JAK-STAT1/2-Signalgebungswegs höher ist im Vergleich zu der Aktivität des zellulären JAK-STAT1/2-Signalgebungswegs in der Referenzblutprobe, oder
wobei die Infektion in dem Subjekt, aus dem die Blutprobe entnommen wird, als bakteriell bestimmt wird, wenn die Aktivität des zellulären JAK-STAT1/2-Signalgebungswegs nicht höher ist im Vergleich zu der Aktivität des zellulären JAK-STAT1/2-Signalgebungswegs in der Referenzblutprobe,
wobei das kalibrierte mathematische Pfad-Modell ein Zentroid-Modell oder ein lineares Modell, oder ein Bayes'sches Netzwerkmodell, basierend auf bedingten Wahrscheinlichkeiten, ist, und
wobei die drei oder mehr Zielgene des zellulären JAK-STAT1/2-Signalgebungswegs ausgewählt sind aus der Gruppe, bestehend aus:
BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFD1L, USP18 und ZNRF3.

2. Computer-implementiertes Verfahren zum Bestimmen der zellulären Immunantwort auf eine Virusinfektion oder einen Impfstoff in einer Blutprobe, entnommen aus einem Subjekt mit einer Virusinfektion oder einem Subjekt, das einen Impfstoff erhalten hat, basierend auf den ermittelten Expressionsniveaus von drei oder mehr Zielgenen des zellulären JAK-STAT1/2-Signalgebungswegs, wobei das Verfahren umfasst:
- Empfangen der ermittelten Expressionsniveaus der drei oder mehr Zielgene des zellulären JAK-STAT1/2-Signalgebungswegs;
- Ermitteln der Aktivität des zellulären JAK-STAT1/2-Signalgebungswegs, wobei das Ermitteln der Aktivität des zellulären JAK-STAT1/2-Signalgebungswegs das Zuweisen eines numerischen Werts an das Aktivitätsniveau des zellulären JAK-STAT1/2-Signalgebungswegs durch Evaluierung eines kalibrierten mathematischen Pfad-Modells, das die Expressionsniveaus der drei oder mehr JAK-STAT1/2-Zielgene mit einem Aktivitätsniveau des zellulären JAK-STAT1/2-Signalgebungswegs in Zusammenhang bringt, umfasst;
- Vergleichen der Aktivität des zellulären JAK-STAT1/2-Signalgebungswegs, ermittelt in der Blutprobe, entnommen aus dem Subjekt mit einer Virusinfektion oder einem Subjekt, das einen Impfstoff erhalten hat, mit der Aktivität des zellulären JAK-STAT1/2-Signalgebungswegs, ermittelt in einer Referenzblutprobe, entnommen aus einem gesunden Subjekt,
wobei die Aktivität des zellulären JAK-STAT1/2-Signalgebungswegs mit den Aktivitäten des zellulären Signalgebungswegs, ermittelt in den Referenzblutproben, verglichen wird, um zu bestimmen, ob die Immunantwort auf die Virusinfektion schwach oder stark ist, wobei das Verfahren ferner umfasst:
- Empfangen der ermittelten Expressionsniveaus der drei oder mehr Zielgene des zellulären JAK-STAT3-Signalgebungswegs;
- Ermitteln der Aktivität des zellulären JAK-STAT3-Signalgebungswegs, wobei das Ermitteln der Aktivität des zellulären JAK-STAT3-Signalgebungswegs das Zuweisen eines numerischen Werts an das Aktivitätsniveau des zellulären JAK-STAT3-Signalgebungswegs durch Evaluierung eines kalibrierten mathematischen Pfad-Modells, das Expressionsniveaus der Zielgene mit dem Aktivitätsniveau des zellulären JAK-STAT3-Signalgebungswegs in Zusammenhang bringt, umfasst;
wobei das Verfahren ferner das Ermitteln eines Aktivitätsniveaus eines Signalgebungsweg-assoziierten Transkriptionsfaktor (TF)-Elements umfasst, wobei das Signalgebungsweg-assoziierte TF-Element die Transkription der drei oder mehr Zielgene steuert, wobei das Ermitteln auf der Evaluierung eines kalibrierten mathematischen Pfad-Modells, das Expressionsniveaus der Zielgene mit dem Aktivitätsniveau des zellulären JAK-STAT1/2-Signalgebungswegs in Zusammenhang bringt, basiert, und
Ableiten der Aktivität des zellulären JAK-STAT1/2-Signalgebungswegs in der Blutprobe basierend auf dem ermittelten Aktivitätsniveau des Signalgebungsweg-assoziierten TF-Elements,
- Vergleichen der Aktivität des zellulären JAK-STAT3-Signalgebungswegs, ermittelt in der Blutprobe, entnommen aus dem Subjekt mit einer Virusinfektion oder einem Subjekt, das einen Impfstoff erhalten hat, mit der Aktivität des zellulären JAK-STAT3-Signalgebungswegs, ermittelt in einer Referenzblutprobe, die aus einem gesunden Subjekt entnommen wird,
wobei das kalibrierte mathematische Pfad-Modell ein Zentroid-Modell oder ein lineares Modell, oder ein Bayes'sches Netzwerkmodell, basierend auf bedingten Wahrscheinlichkeiten, ist, und
wobei die drei oder mehr Zielgene des zellulären JAK-STAT1/2-Signalgebungswegs ausgewählt sind aus der Gruppe, bestehend aus:
BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFD1L, USP18 und ZNRF3, und
wobei die drei oder mehr Zielgene des zellulären JAK-STAT3-Signalgebungswegs ausgewählt sind aus der Gruppe, bestehend aus:
AKT1, BCL2, BCL2L1, BIRC5, CCND1, CD274, CDKNIA, CRP, FGF2, FOS, FSCN1, FSCN2, FSCN3, HIFIA, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST1, VIM und ZEB1.

3. Computer-implementiertes Verfahren nach Anspruch 2, wobei die Immunantwort auf eine Virusinfektion als schwach betrachtet wird, wenn der numerische Wert, zugewiesen an die Aktivität des zellulären JAK-STAT1/2-Signalgebungswegs in der Blutprobe, entnommen aus dem Subjekt mit einer Virusinfektion oder einem Subjekt, das einen Impfstoff erhalten hat, um eine Standardabweichung höher ist als der numerische Wert, zugewiesen an die Aktivität des zellulären JAK-STAT1/2-Signalgebungswegs in der Referenzblutprobe des gesunden Subjekts, und die Immunantwort auf eine Virusinfektion als stark betrachtet wird, wenn der numerische Wert, zugewiesen an die Aktivität des zellulären JAK-STAT1/2-Signalgebungswegs in der Blutprobe, entnommen aus dem Subjekt mit einer Virusinfektion oder einem Subjekt, das einen Impfstoff erhalten hat, um mindestens zwei, vorzugsweise drei oder mehr Standardabweichungen höher ist als der numerische Wert, zugewiesen an die Aktivität des zellulären JAK-STAT1/2-Signalgebungswegs in der Referenzblutprobe des gesunden Subjekts.

4. Computer-implementiertes Verfahren nach Anspruch 2, wobei das Vergleichen der Aktivitäten des zellulären JAK-STAT1/2- und optional JAK-STAT3-Signalgebungswegs, ermittelt in der Blutprobe, entnommen aus dem Subjekt mit einer Virusinfektion oder dem Subjekt, das einen Impfstoff erhalten hat, ferner das Vergleichen mit den Aktivitäten des zellulären JAK-STAT1/2- und optional JAK-STAT3-Signalgebungswegs, ermittelt in einer Referenzblutprobe, entnommen aus einem Referenzpatienten mit einer schwachen Immunantwort, und den Aktivitäten des zellulären JAK-STAT1/2- und optional JAK-STAT3-Signalgebungswegs, ermittelt in einer Referenzblutprobe, entnommen aus einem Referenzpatienten mit einer starken Immunantwort, umfasst, und wobei die Stärke der Immunantwort in dem Subjekt mit einer Virusinfektion oder dem Subjekt, das einen Impfstoff erhalten hat, auf dem Vergleich zwischen den Aktivitäten des zellulären JAK-STAT1/2-Signalgebungswegs aus dem Subjekt mit einer Virusinfektion oder dem Subjekt, das einen Impfstoff erhalten hat, mit den Aktivitäten des zellulären JAK-STAT1/2-Signalgebungswegs, ermittelt in den Referenzblutproben, entnommen aus dem Referenzpatienten mit einer schwachen Immunantwort, und den Referenzblutproben, entnommen aus dem Referenzpatienten mit einer starken Immunantwort, basiert.

5. Computer-implementiertes Verfahren nach einem der Ansprüche 2 bis 4, wobei die Aktivität des zellulären JAK-STAT1/2-Signalgebungswegs der Stärke der Immunantwort entspricht, wobei eine höhere Aktivität des zellulären JAK-STAT1/2-Signalgebungswegs eine stärkere Immunantwort anzeigt, optional wobei die Blutprobe aus einem Subjekt mit einer Virusinfektion stammt und wobei eine höhere Aktivität des zellulären JAK-STAT3-Signalgebungswegs auf eine schwerere Infektion hinweist, oder wobei die Blutprobe aus einem Subjekt, das einen Impfstoff erhält, stammt und wobei eine stärkere Immunantwort und optional eine höhere Aktivität des zellulären JAK-STAT3-Signalgebungswegs auf eine stärkere zelluläre Immunität hinweist.

6. Computer-implementiertes Verfahren nach einem der vorhergehenden Ansprüche, wobei die ermittelten Aktivitätsniveaus der zellulären JAK-STAT1/2- und optional JAK-STAT3-Signalgebungswege ferner verwendet werden zum
- Überwachen eines Patienten mit einer Infektion, oder
- Bestimmen der Stärke der zellulären Immunität, die durch eine Virusinfektion oder Impfung in einem Individuum hervorgerufen wird, oder
- Vorhersagen des Ansprechens auf eine/ein immunmodulierende Therapie oder immunmodulierendes Arzneimittel, oder
- Überwachen des Ansprechens auf ein Arzneimittel oder eine Therapie, oder
- Vorhersagen der Toxizität einer/eines immunmodulierenden Therapie oder Arzneimittels, oder
- Einschätzen der Stärke der zellulären Immunität, die während einer Virusinfektionsepidemie/-pandemie in einer Gesellschaft entstehen wird, oder
- Bestimmen der Stärke der Immunität, die durch Virusinfektion oder Impfung bei einem Individuum mit einer speziellen immunschwächenden Erkrankung, wie einer speziellen Komorbidität, Therapie oder Lebensweise, hervorgerufen wird, oder
- Diagnostizieren von Patienten mit einer Virusinfektion während einer Epidemie oder Pandemie, oder
- Entwickeln eines Arzneimittels oder einer Therapie zum Behandeln der Infektionskrankheit, oder
- Vorhersagen des immunaktivierenden oder immunsuppressiven Status, der durch die Virusinfektion verursacht wird.

7. Computer-implementiertes Verfahren nach einem der vorhergehenden Ansprüche, wobei es sich bei der Blutprobe um eine Vollblutprobe, eine Probe von mononukleären Zellen des peripheren Blutes, oder isolierte Blutzellen, wie dendritische Zellen, CD4+ T-Zellen, CD8+ T-Zellen, CD16-- Monozyten, CD16+ Monozyten, Neutrophile, NK-Zellen und B-Zellen, handelt.

8. Computer-implementiertes Verfahren nach einem der vorhergehenden Ansprüche, wobei die Aktivität des zellulären JAK-STAT1/2-Signalgebungswegs und optional des zellulären JAK-STAT3-Signalgebungswegs in der Blutprobe durch ein Verfahren abgeleitet werden kann, umfassend: Empfangen der Expressionsniveaus von drei oder mehr Zielgenen des zellulären JAK-STAT1/2-Signalgebungswegs und optional des zellulären JAK-STAT3-Signalgebungswegs,
Ermitteln eines Aktivitätsniveaus eines Signalgebungsweg-assoziierten Transkriptionsfaktor (TF)-Elements, wobei das Signalgebungsweg-assoziierte TF-Element die Transkription der drei oder mehr Zielgene steuert, wobei das Ermitteln auf der Evaluierung eines kalibrierten mathematischen Pfad-Modells, das Expressionsniveaus der Zielgene mit dem Aktivitätsniveau des zellulären JAK-STAT1/2-Signalgebungswegs und optional des zellulären JAK-STAT3-Signalgebungswegs in Zusammenhang bringt, basiert, und
Ableiten der Aktivität des zellulären JAK-STAT1/2-Signalgebungswegs und optional des zellulären JAK-STAT3-Signalgebungswegs in der Blutprobe basierend auf dem ermittelten Aktivitätsniveau des Signalgebungsweg-assoziierten TF-Elements, wobei das kalibrierte mathematische Pfad-Modell vorzugsweise ein Zentroid-Modell oder ein lineares Modell, oder ein Bayes'sches Netzwerkmodell, basierend auf bedingten Wahrscheinlichkeiten, ist.

9. Nichtflüchtiges Speichermedium, das Befehle speichert, welche durch eine digitale Verarbeitungsvorrichtung ausführbar sind, um das Verfahren nach beliebigen der Ansprüche 1 bis 8 durchzuführen.

10. Computerprogramm, umfassend Programmcode-Vorkehrungen, die eine digitale Verarbeitungsvorrichtung dazu veranlassen, das Verfahren nach beliebigen der Ansprüche 1 bis 8 durchzuführen, wenn des Computerprogramm auf einer digitalen Verarbeitungsvorrichtung laufen gelassen wird.

11. Kit von Teilen, umfassend Komponenten zum Ermitteln der Expressionsniveaus von drei oder mehr Zielgenen des zellulären JAK-STAT1/2-Signalgebungswegs und drei oder mehr Zielgenen des zellulären JAK-STAT3-Signalgebungswegs,
wobei die drei oder mehr Zielgene des zellulären JAK-STAT1/2-Signalgebungswegs ausgewählt sind aus der Gruppe, bestehend aus:
BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFD1L, USP18, ZNRF3, GBP1, TAP1, ISG15, APOL1, IFI6, IFIRM1, CXCL9, APOL2, IFIT2 und LY6E, vorzugsweise aus der Gruppe, bestehend aus: IRF1, IRF7, IRF8, IRF9, OAS1, PDCD1, ST13, STAT1 und USP1, oder aus der Gruppe, bestehend aus GBP1, IRF9, STAT1, TAP1, ISG15, APOL1, IRF1, IRF7, IFI6, IFIRM1, USP18, CXCL9, OAS1, APOL2, IFIT2 und LY6E, und
wobei die drei oder mehr Zielgene des zellulären JAK-STAT3-Signalgebungswegs ausgewählt sind aus der Gruppe, bestehend aus: AKT1, BCL2, BCL2L1, BIRC5, CCND1, CD274, CDKNIA, CRP, FGF2, FOS, FSCN1, FSCN2, FSCN3, HIFIA, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST1, VIM und ZEB1, und
wobei die Komponenten zum Ermitteln des Expressionsniveaus Primer und Sonden sind, und optional ferner umfassend das nichtflüchtige Speichermedium nach Anspruch 9 oder das Computerprogramm nach Anspruch 10.

12. Verwendung des Kits nach Anspruch 11 in einem Verfahren zum Unterscheiden zwischen einer bakteriellen Infektion und einer Virusinfektion in einer aus einem Subjekt mit einer Infektion entnommenen Blutprobe, basierend auf den ermittelten Expressionsniveaus von drei oder mehr Zielgenen des zellulären JAK-STAT1/2-Signalgebungswegs, wobei das Verfahren umfasst:
- Ermitteln der Expressionsniveaus der drei oder mehr Zielgene des zellulären JAK-STAT1/2-Signalgebungswegs;
- Ermitteln der Aktivität des zellulären JAK-STAT1/2-Signalgebungswegs, wobei das Ermitteln der Aktivität des zellulären JAK-STAT1/2-Signalgebungswegs das Zuweisen eines numerischen Werts an das Aktivitätsniveau des zellulären JAK-STAT1/2-Signalgebungswegs durch Evaluierung eines kalibrierten mathematischen Pfad-Modells, das die Expressionsniveaus der drei oder mehr JAK-STAT1/2-Zielgene mit einem Aktivitätsniveau des zellulären JAK-STAT1/2-Signalgebungswegs in Zusammenhang bringt, umfasst,
wobei das Verfahren ferner das Ermitteln eines Aktivitätsniveaus eines Signalgebungsweg-assoziierten Transkriptionsfaktor (TF)-Elements umfasst, wobei das Signalgebungsweg-assoziierte TF-Element die Transkription der drei oder mehr Zielgene steuert, wobei das Ermitteln auf der Evaluierung eines kalibrierten mathematischen Pfad-Modells, das Expressionsniveaus der Zielgene mit dem Aktivitätsniveau des zellulären JAK-STAT1/2-Signalgebungswegs in Zusammenhang bringt, basiert, und
Ableiten der Aktivität des zellulären JAK-STAT1/2-Signalgebungswegs in der Blutprobe basierend auf dem ermittelten Aktivitätsniveau des Signalgebungsweg-assoziierten TF-Elements,
- Vergleichen der Aktivität des zellulären JAK-STAT1/2-Signalgebungswegs, ermittelt in der aus dem Subjekt entnommenen Blutprobe, mit der Aktivität des zellulären JAK-STAT1/2-Signalgebungswegs, die in einer Referenzblutprobe ermittelt wurde,
wobei die Referenzblutprobe aus einem gesunden Subjekt oder einem Subjekt, das sich von einer Infektion erholt hat, entnommen wird,
und wobei die Infektion in dem Subjekt, aus dem die Blutprobe entnommen wird, als viral bestimmt wird, wenn die Aktivität des zellulären JAK-STAT1/2-Signalgebungswegs höher ist im Vergleich zu der Aktivität des zellulären JAK-STAT1/2-Signalgebungswegs in der Referenzblutprobe, oder
wobei die Infektion in dem Subjekt, aus dem die Blutprobe entnommen wird, als bakteriell bestimmt wird, wenn die Aktivität des zellulären JAK-STAT1/2-Signalgebungswegs nicht höher ist im Vergleich zu der Aktivität des zellulären JAK-STAT1/2-Signalgebungswegs in der Referenzblutprobe,
wobei das kalibrierte mathematische Pfad-Modell ein Zentroid-Modell oder ein lineares Modell, oder ein Bayes'sches Netzwerkmodell, basierend auf bedingten Wahrscheinlichkeiten, ist, und
wobei die drei oder mehr Zielgene des zellulären JAK-STAT1/2-Signalgebungswegs ausgewählt sind aus der Gruppe, bestehend aus:
BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFD1L, USP18 und ZNRF3, vorzugsweise wobei das Verfahren vorgesehen ist für die *In-vitro-* oder Ex-vivo-Diagnose einer Virusinfektion in einem Subjekt.

13. Computer-implementiertes Verfahren zum Klassifizieren eines Subjekts mit einer Virusinfektion als kritischem Fall, basierend auf einer aus einem Subjekt mit einer bestätigten Virusinfektion entnommenen Blutprobe, basierend auf den ermittelten Expressionsniveaus von drei oder mehr Zielgenen des zellulären JAK-STAT1/2-Signalgebungswegs, wobei das Verfahren umfasst:
- Empfangen der ermittelten Expressionsniveaus der drei oder mehr Zielgene des zellulären JAK-STAT1/2-Signalgebungswegs;
- Ermitteln der Aktivität des zellulären JAK-STAT1/2-Signalgebungswegs, wobei das Ermitteln der Aktivität des zellulären JAK-STAT1/2-Signalgebungswegs das Zuweisen eines numerischen Werts an das Aktivitätsniveau des zellulären JAK-STAT1/2-Signalgebungswegs durch Evaluierung eines kalibrierten mathematischen Pfad-Modells, das die Expressionsniveaus der drei oder mehr JAK-STAT1/2-Zielgene mit einem Aktivitätsniveau des zellulären JAK-STAT1/2-Signalgebungswegs in Zusammenhang bringt, umfasst; und
wobei das Verfahren ferner das Ermitteln eines Aktivitätsniveaus eines Signalgebungsweg-assoziierten Transkriptionsfaktor (TF)-Elements umfasst, wobei das Signalgebungsweg-assoziierte TF-Element die Transkription der drei oder mehr Zielgene steuert, wobei das Ermitteln auf der Evaluierung eines kalibrierten mathematischen Pfad-Modells, das Expressionsniveaus der Zielgene mit dem Aktivitätsniveau des zellulären JAK-STAT1/2-Signalgebungswegs in Zusammenhang bringt, basiert, und
Ableiten der Aktivität des zellulären JAK-STAT1/2-Signalgebungswegs in der Blutprobe basierend auf dem ermittelten Aktivitätsniveau des Signalgebungsweg-assoziierten TF-Elements,
Vergleichen der Aktivität des zellulären JAK-STAT1/2-Signalgebungswegs, ermittelt in der aus dem Subjekt entnommenen Blutprobe, mit der Aktivität des zellulären JAK-STAT1/2-Signalgebungswegs, ermittelt in einer Referenzblutprobe,
wobei die Referenzblutprobe aus einem gesunden Subjekt entnommen wird,
und wobei das Subjekt mit der Virusinfektion als als kritischer Fall betrachtet wird, wenn die ermittelte Aktivität des JAK-STAT1/2-Wegs gleich oder niedriger als die in der Referenzblutprobe des gesunden Subjekts ermittelte Aktivität des JAK-STAT1/2-Wegs ist, oder wobei das Subjekt mit der Virusinfektion als nicht-kritischer Fall betrachtet wird, wenn die ermittelte Aktivität des JAK-STAT1/2-Wegs höher als die in der Referenzblutprobe des gesunden Subjekts ermittelte Aktivität des JAK-STAT1/2-Wegs ist,
wobei die Blutprobe aus dem Subjekt mit der bestätigten Virusinfektion während der akuten Phase der Infektion entnommen worden ist,
wobei das kalibrierte mathematische Pfad-Modell ein Zentroid-Modell oder ein lineares Modell, oder ein Bayes'sches Netzwerkmodell, basierend auf bedingten Wahrscheinlichkeiten, ist, und
wobei die drei oder mehr Zielgene des zellulären JAK-STAT1/2-Signalgebungswegs ausgewählt sind aus der Gruppe, bestehend aus:
BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFD1L, USP18 und ZNRF3.

## Revendications

1. Procédé mis en œuvre par ordinateur pour distinguer une infection bactérienne d'une infection virale dans un échantillon de sang obtenu à partir d'un sujet souffrant d'une infection, sur la base des niveaux d'expression déterminés de trois gènes cibles ou plus de la voie de signalisation cellulaire JAK-STAT1/2, le procédé comprenant :
- la réception des niveaux d'expression déterminés des trois gènes cibles ou plus de la voie de signalisation cellulaire JAK-STAT1/2 ;
- la détermination de l'activité de la voie de signalisation cellulaire JAK-STAT1/2, où la détermination de l'activité de la voie de signalisation cellulaire JAK-STAT1/2 comprend l'attribution d'une valeur numérique au niveau d'activité de la voie de signalisation cellulaire JAK-STAT1/2 en évaluant un modèle de voie mathématique étalonné reliant les niveaux d'expression des trois gènes cibles JAK-STAT1/2 ou plus à un niveau d'activité de la voie de signalisation cellulaire JAK-STAT1/2,
dans lequel le procédé comprend en outre la détermination d'un niveau d'activité d'un élément facteur de transcription (TF) associé à la voie de signalisation, l'élément TF associé à la voie de signalisation régulant la transcription des trois gènes cibles ou plus, la détermination étant basée sur l'évaluation d'un modèle de voie mathématique étalonné reliant les niveaux d'expression des gènes cibles au niveau d'activité de la voie de signalisation cellulaire JAK-STAT1/2, et
la déduction de l'activité de la voie de signalisation cellulaire JAK-STAT1/2 dans l'échantillon de sang sur la base du niveau d'activité déterminé de l'élément TF associé à la voie de signalisation,
- la comparaison de l'activité de la voie de signalisation cellulaire JAK-STAT1/2 déterminée dans l'échantillon de sang obtenu à partir du sujet avec l'activité de la voie de signalisation cellulaire JAK-STAT1/2 déterminée dans un échantillon de sang de référence,
dans lequel l'échantillon de sang de référence est obtenu à partir d'un sujet sain ou d'un sujet guéri d'une infection,
et dans lequel l'infection chez le sujet à partir duquel l'échantillon de sang est obtenu est déterminée comme étant virale lorsque l'activité de la voie de signalisation cellulaire JAK-STAT1/2 est plus élevée par rapport à l'activité de la voie de signalisation cellulaire JAK-STAT1/2 dans l'échantillon de sang de référence, ou
dans lequel l'infection chez le sujet à partir duquel l'échantillon de sang est obtenu est déterminée comme étant bactérienne lorsque l'activité de la voie de signalisation cellulaire JAK-STAT1/2 n'est pas plus élevée par rapport à l'activité de la voie de signalisation cellulaire JAK-STAT1/2 dans l'échantillon de sang de référence,
dans lequel le modèle de voie mathématique étalonné est un centroïde ou un modèle linéaire, ou un modèle de réseau bayésien basé sur des probabilités conditionnelles, et
dans lequel les trois gènes cibles ou plus de la voie de signalisation cellulaire JAK-STAT1/2 sont choisis dans le groupe constitué par :
BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFD1L, USP18 et ZNRF3.

2. Procédé mis en œuvre par ordinateur pour déterminer la réponse immunitaire cellulaire à une infection virale ou à un vaccin dans un échantillon de sang obtenu à partir d'un sujet souffrant d'une infection virale ou d'un sujet ayant reçu un vaccin, sur la base des niveaux d'expression déterminés de trois gènes cibles ou plus de la voie de signalisation cellulaire JAK-STAT1/2, le procédé comprenant :
- la réception des niveaux d'expression déterminés des trois gènes cibles ou plus de la voie de signalisation cellulaire JAK-STAT1/2 ;
- la détermination de l'activité de la voie de signalisation cellulaire JAK-STAT1/2, où la détermination de l'activité de la voie de signalisation cellulaire JAK-STAT1/2 comprend l'attribution d'une valeur numérique au niveau d'activité de la voie de signalisation cellulaire JAK-STAT1/2 en évaluant un modèle de voie mathématique étalonné reliant les niveaux d'expression des trois gènes cibles JAK-STAT1/2 ou plus à un niveau d'activité de la voie de signalisation cellulaire JAK-STAT1/2 ;
- la comparaison de l'activité de la voie de signalisation cellulaire JAK-STAT1/2 déterminée dans l'échantillon de sang obtenu à partir du sujet souffrant d'une infection virale ou d'un sujet ayant reçu un vaccin avec l'activité de la voie de signalisation cellulaire JAK-STAT1/2 déterminée dans un échantillon de sang de référence obtenu à partir d'un sujet sain,
dans lequel l'activité de la voie de signalisation cellulaire JAK-STAT1/2 est comparée aux activités de la voie de signalisation cellulaire déterminées dans les échantillons de sang de référence afin de déterminer si la réponse immunitaire à l'infection virale est faible ou forte, où le procédé comprend en outre :
- la réception des niveaux d'expression déterminés des trois gènes cibles ou plus de la voie de signalisation cellulaire JAK-STAT3 ;
- la détermination de l'activité de la voie de signalisation cellulaire JAK-STAT3, où la détermination de l'activité de la voie de signalisation cellulaire JAK-STAT3 comprend l'attribution d'une valeur numérique au niveau d'activité de la voie de signalisation cellulaire JAK-STAT3 en évaluant un modèle de voie mathématique étalonné reliant des niveaux d'expression des gènes cibles au niveau d'activité de la voie de signalisation cellulaire JAK-STAT3 ;
dans lequel le procédé comprend en outre la détermination d'un niveau d'activité d'un élément facteur de transcription (TF) associé à la voie de signalisation, l'élément TF associé à la voie de signalisation régulant la transcription des trois gènes cibles ou plus, la détermination étant basée sur l'évaluation d'un modèle de voie mathématique étalonné reliant les niveaux d'expression des gènes cibles au niveau d'activité de la voie de signalisation cellulaire JAK-STAT1/2, et
la déduction de l'activité de la voie de signalisation cellulaire JAK-STAT1/2 dans l'échantillon de sang sur la base du niveau d'activité déterminé de l'élément TF associé à la voie de signalisation,
- la comparaison de l'activité de la voie de signalisation cellulaire JAK-STAT3 déterminée dans l'échantillon de sang obtenu à partir du sujet souffrant d'une infection virale ou d'un sujet ayant reçu un vaccin avec l'activité de la voie de signalisation cellulaire JAK-STAT3 déterminée dans un échantillon de sang de référence obtenu à partir d'un sujet sain,
dans lequel le modèle de voie mathématique étalonné est un centroïde ou un modèle linéaire, ou un modèle de réseau bayésien basé sur des probabilités conditionnelles, et
dans lequel les trois gènes cibles ou plus de la voie de signalisation cellulaire JAK-STAT1/2 sont choisis dans le groupe constitué par :
BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFD1L, USP18 et ZNRF3, et
dans lequel les trois gènes cibles ou plus de la voie de signalisation cellulaire JAK-STAT3 sont choisis dans le groupe constitué par :
AKT1, BCL2, BCL2L1, BIRC5, CCND1, CD274, CDKNIA, CRP, FGF2, FOS, FSCN1, FSCN2, FSCN3, HIFIA, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST1, VIM et ZEB1.

3. Procédé mis en œuvre par ordinateur selon la revendication 2, dans lequel la réponse immunitaire à une infection virale est considérée comme faible lorsque la valeur numérique attribuée à l'activité de la voie de signalisation cellulaire JAK-STAT1/2 dans l'échantillon de sang obtenu à partir du sujet souffrant d'une infection virale ou d'un sujet ayant reçu un vaccin est supérieure d'un écart type à la valeur numérique attribuée à l'activité de la voie de signalisation cellulaire JAK-STAT1/2 dans l'échantillon de sang de référence du sujet sain et la réponse immunitaire à une infection virale est considérée comme forte lorsque la valeur numérique attribuée à l'activité de la voie de signalisation cellulaire JAK-STAT1/2 dans l'échantillon de sang obtenu à partir du sujet souffrant d'une infection virale ou d'un sujet ayant reçu un vaccin est supérieure d'au moins deux, de préférence trois écarts types ou plus, à la valeur numérique attribuée à l'activité de la voie de signalisation cellulaire JAK-STAT1/2 dans l'échantillon de sang de référence du sujet sain.

4. Procédé mis en œuvre par ordinateur selon la revendication 2, dans lequel la comparaison des activités des voies de signalisation cellulaire JAK-STAT1/2 et facultativement JAK-STAT3 déterminées dans l'échantillon de sang obtenu à partir du sujet souffrant d'une infection virale ou du sujet ayant reçu un vaccin comprend en outre la comparaison avec les activités des voies de signalisation cellulaire JAK-STAT1/2 et facultativement JAK-STAT3 déterminées dans un échantillon de sang de référence obtenu à partir d'un patient de référence ayant une faible réponse immunitaire et les activités des voies de signalisation cellulaire JAK-STAT1/2 et facultativement JAK-STAT3 déterminées dans un échantillon de sang de référence obtenu à partir d'un patient de référence ayant une forte réponse immunitaire, et dans lequel la force de la réponse immunitaire chez le sujet souffrant d'une infection virale ou le sujet ayant reçu un vaccin est basée sur la comparaison entre les activités de la voie de signalisation cellulaire JAK-STAT1/2 du sujet souffrant d'une infection virale ou du sujet ayant reçu un vaccin et les activités de la voie de signalisation cellulaire JAK-STAT1/2 déterminées dans les échantillons de sang de référence obtenus à partir du patient de référence ayant une faible réponse immunitaire et les échantillons de sang de référence obtenus à partir du patient de référence ayant une forte réponse immunitaire.

5. Procédé mis en œuvre par ordinateur selon l'une quelconque des revendications 2 à 4, dans lequel l'activité de la voie de signalisation cellulaire JAK-STAT1/2 correspond à la force de la réponse immunitaire, dans lequel une activité de la voie de signalisation cellulaire JAK-STAT1/2 plus élevée signifie une réponse immunitaire plus forte, facultativement dans lequel l'échantillon de sang provient d'un sujet souffrant d'une infection virale et dans lequel une activité de la voie de signalisation cellulaire JAK-STAT3 plus élevée est indicative d'une infection plus grave ou dans lequel l'échantillon de sang provient d'un sujet qui reçoit un vaccin et dans lequel une réponse immunitaire plus forte et facultativement une activité de la voie de signalisation cellulaire JAK-STAT3 plus élevée est indicative d'une immunité cellulaire plus forte.

6. Procédé mis en œuvre par ordinateur selon l'une quelconque des revendications précédentes, dans lequel les niveaux d'activité déterminés des voies de signalisation cellulaire JAK-STAT1/2 et facultativement JAK-STAT3 sont en outre utilisés pour
- surveiller un patient souffrant d'une infection, ou
- déterminer la force de l'immunité cellulaire induite par une infection virale ou une vaccination chez un individu, ou
- prédire la réponse à une thérapie immunomodulatrice ou à un médicament immunomodulateur, ou
- surveiller la réponse à un médicament ou à une thérapie, ou
- prédire la toxicité d'une thérapie immunomodulatrice ou d'un médicament immunomodulateur, ou
- estimer la force de l'immunité cellulaire qui résultera dans une communauté pendant une épidémie/pandémie d'infection virale, ou
- déterminer la force de l'immunité induite par une infection virale ou une vaccination chez un individu ayant une affection immunodéprimante spécifique, telle qu'une comorbidité, une thérapie, un mode de vie spécifique, ou
- diagnostiquer des patients souffrant d'une infection virale pendant une épidémie ou une pandémie, ou
- développer un médicament ou une thérapie pour traiter la maladie infectieuse, ou
- prédire l'état d'activation immunitaire ou d'immunosuppression provoqué par l'infection virale.

7. Procédé mis en œuvre par ordinateur selon l'une quelconque des revendications précédentes, dans lequel l'échantillon de sang est un échantillon de sang total, un échantillon de cellules mononucléaires du sang périphérique ou des cellules sanguines isolées telles que des cellules dendritiques, des cellules T CD4+, des cellules T CD8+, des monocytes CD16-, des monocytes CD16+, des neutrophiles, des cellules NK et des cellules B.

8. Procédé mis en œuvre par ordinateur selon l'une quelconque des revendications précédentes, dans lequel l'activité de la voie de signalisation cellulaire JAK-STAT1/2 et facultativement de la voie de signalisation cellulaire JAK-STAT3 dans l'échantillon de sang peut être déduite par un procédé comprenant : la réception des niveaux d'expression de trois gènes cibles ou plus de la voie de signalisation cellulaire JAK-STAT1/2 et facultativement de la voie de signalisation cellulaire JAK-STAT3,
la détermination d'un niveau d'activité d'un élément facteur de transcription (TF) associé à la voie de signalisation, l'élément TF associé à la voie de signalisation régulant la transcription des trois gènes cibles ou plus, la détermination étant basée sur l'évaluation d'un modèle de voie mathématique étalonné reliant les niveaux d'expression des gènes cibles au niveau d'activité de la voie de signalisation cellulaire JAK-STAT1/2 et facultativement de la voie de signalisation cellulaire JAK-STAT3, et
la déduction de l'activité de la voie de signalisation cellulaire JAK-STAT1/2 et facultativement de la voie de signalisation cellulaire JAK-STAT3 dans l'échantillon de sang sur la base du niveau d'activité déterminé de l'élément TF associé à la voie de signalisation,
dans lequel le modèle de voie mathématique étalonné est de préférence un centroïde ou un modèle linéaire, ou un modèle de réseau bayésien basé sur des probabilités conditionnelles.

9. Support de stockage non transitoire stockant des instructions qui sont exécutables par un dispositif de traitement numérique pour réaliser le procédé selon l'une des revendications 1 à 8.

10. Programme informatique comprenant un moyen de code de programme pour amener un dispositif de traitement numérique à réaliser le procédé selon l'une des revendications 1 à 8, lorsque le programme informatique est exécuté sur un dispositif de traitement numérique.

11. Kit de pièces, comprenant des composants pour déterminer les niveaux d'expression de trois gènes cibles ou plus de la voie de signalisation cellulaire JAK-STAT1/2 et de trois gènes cibles ou plus de la voie de signalisation cellulaire JAK-STAT3,
dans lequel les trois gènes cibles ou plus de la voie de signalisation cellulaire JAK-STAT1/2 sont choisis dans le groupe constitué par :
BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFD1L, USP18, ZNRF3, GBP1, TAP1, ISG15, APOL1, IFI6, IFIRM1, CXCL9, APOL2, IFIT2 et LY6E, de préférence, dans le groupe constitué par : IRF1, IRF7, IRF8, IRF9, OAS1, PDCD1, ST13, STAT1 et USP1 ou dans le groupe constitué par GBP1, IRF9, STAT1, TAP1, ISG15, APOL1, IRF1, IRF7, IFI6, IFIRM1, USP18, CXCL9, OAS1, APOL2, IFIT2 et LY6E, et
dans lequel les trois gènes cibles ou plus de la voie de signalisation cellulaire JAK-STAT3 sont choisis dans le groupe constitué par : AKT1, BCL2, BCL2L1, BIRC5, CCND1, CD274, CDKNIA, CRP, FGF2, FOS, FSCN1, FSCN2, FSCN3, HIFIA, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST1, VIM et ZEB1, et
dans lequel les composants pour déterminer le niveau d'expression sont des amorces et des sondes, et comprenant facultativement en outre le support de stockage non transitoire selon la revendication 9 ou le programme informatique selon la revendication 10.

12. Utilisation du kit selon la revendication 11 dans un procédé pour distinguer une infection bactérienne d'une infection virale dans un échantillon de sang obtenu à partir d'un sujet souffrant d'une infection, sur la base des niveaux d'expression déterminés de trois gènes cibles ou plus de la voie de signalisation cellulaire JAK-STAT1/2, le procédé comprenant :
- la détermination des niveaux d'expression des trois gènes cibles ou plus de la voie de signalisation cellulaire JAK-STAT1/2 ;
- la détermination de l'activité de la voie de signalisation cellulaire JAK-STAT1/2, où la détermination de l'activité de la voie de signalisation cellulaire JAK-STAT1/2 comprend l'attribution d'une valeur numérique au niveau d'activité de la voie de signalisation cellulaire JAK-STAT1/2 en évaluant un modèle de voie mathématique étalonné reliant les niveaux d'expression des trois gènes cibles JAK-STAT1/2 ou plus à un niveau d'activité de la voie de signalisation cellulaire JAK-STAT1/2,
dans laquelle le procédé comprend en outre la détermination d'un niveau d'activité d'un élément facteur de transcription (TF) associé à la voie de signalisation, l'élément TF associé à la voie de signalisation régulant la transcription des trois gènes cibles ou plus, la détermination étant basée sur l'évaluation d'un modèle de voie mathématique étalonné reliant les niveaux d'expression des gènes cibles au niveau d'activité de la voie de signalisation cellulaire JAK-STAT1/2, et
la déduction de l'activité de la voie de signalisation cellulaire JAK-STAT1/2 dans l'échantillon de sang sur la base du niveau d'activité déterminé de l'élément TF associé à la voie de signalisation,
- la comparaison de l'activité de la voie de signalisation cellulaire JAK-STAT1/2 déterminée dans l'échantillon de sang obtenu à partir du sujet avec l'activité de la voie de signalisation cellulaire JAK-STAT1/2 déterminée dans un échantillon de sang de référence,
dans laquelle l'échantillon de sang de référence est obtenu à partir d'un sujet sain ou d'un sujet guéri d'une infection,
et dans laquelle l'infection chez le sujet à partir duquel l'échantillon de sang est obtenu est déterminée comme étant virale lorsque l'activité de la voie de signalisation cellulaire JAK-STAT1/2 est plus élevée par rapport à l'activité de la voie de signalisation cellulaire JAK-STAT1/2 dans l'échantillon de sang de référence, ou
dans laquelle l'infection chez le sujet à partir duquel l'échantillon de sang est obtenu est déterminée comme étant bactérienne lorsque l'activité de la voie de signalisation cellulaire JAK-STAT1/2 n'est pas plus élevée par rapport à l'activité de la voie de signalisation cellulaire JAK-STAT1/2 dans l'échantillon de sang de référence,
dans laquelle le modèle de voie mathématique étalonné est un centroïde ou un modèle linéaire, ou un modèle de réseau bayésien basé sur des probabilités conditionnelles, et
dans laquelle les trois gènes cibles ou plus de la voie de signalisation cellulaire JAK-STAT1/2 sont choisis dans le groupe constitué par :
BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFD1L, USP18 et ZNRF3, de préférence dans laquelle le procédé est pour le diagnostic *in vitro* ou *ex vivo* d'une infection virale chez un sujet.

13. Procédé mis en œuvre par ordinateur pour classer un sujet souffrant d'une infection virale comme critique sur la base d'un échantillon de sang obtenu à partir d'un sujet souffrant d'une infection virale confirmée, sur la base des niveaux d'expression déterminés de trois gènes cibles ou plus de la voie de signalisation cellulaire JAK-STAT1/2, le procédé comprenant :
- la réception des niveaux d'expression déterminés des trois gènes cibles ou plus de la voie de signalisation cellulaire JAK-STAT1/2 ;
- la détermination de l'activité de la voie de signalisation cellulaire JAK-STAT1/2, où la détermination de l'activité de la voie de signalisation cellulaire JAK-STAT1/2 comprend l'attribution d'une valeur numérique au niveau d'activité de la voie de signalisation cellulaire JAK-STAT1/2 en évaluant un modèle de voie mathématique étalonné reliant les niveaux d'expression des trois gènes cibles JAK-STAT1/2 ou plus à un niveau d'activité de la voie de signalisation cellulaire JAK-STAT1/2 ; et
dans lequel le procédé comprend en outre la détermination d'un niveau d'activité d'un élément facteur de transcription (TF) associé à la voie de signalisation, l'élément TF associé à la voie de signalisation régulant la transcription des trois gènes cibles ou plus, la détermination étant basée sur l'évaluation d'un modèle de voie mathématique étalonné reliant les niveaux d'expression des gènes cibles au niveau d'activité de la voie de signalisation cellulaire JAK-STAT1/2, et
la déduction de l'activité de la voie de signalisation cellulaire JAK-STAT1/2 dans l'échantillon de sang sur la base du niveau d'activité déterminé de l'élément TF associé à la voie de signalisation,
la comparaison de l'activité de la voie de signalisation cellulaire JAK-STAT1/2 déterminée dans l'échantillon de sang obtenu à partir du sujet avec l'activité de la voie de signalisation cellulaire JAK-STAT1/2 déterminée dans un échantillon de sang de référence,
dans lequel l'échantillon de sang de référence est obtenu à partir d'un sujet sain,
et dans lequel le sujet souffrant de l'infection virale est considéré comme critique si l'activité de la voie JAK-STAT1/2 déterminée est inférieure ou égale à l'activité de la voie JAK-STAT1/2 déterminée dans l'échantillon de sang de référence du sujet sain, ou dans lequel le sujet souffrant de l'infection virale est considéré comme non critique si l'activité de la voie JAK-STAT1/2 déterminée est supérieure à l'activité de la voie JAK-STAT1/2 déterminée dans l'échantillon de sang de référence du sujet sain,
dans lequel ledit échantillon de sang du sujet souffrant de l'infection virale confirmée a été obtenu pendant la phase aiguë de ladite infection,
dans lequel le modèle de voie mathématique étalonné est un centroïde ou un modèle linéaire, ou un modèle de réseau bayésien basé sur des probabilités conditionnelles, et
dans lequel les trois gènes cibles ou plus de la voie de signalisation cellulaire JAK-STAT1/2 sont choisis dans le groupe constitué par :
BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFD1L, USP18 et ZNRF3.
